# EUROPEAN PATENT APPLICATION

(11) **EP 3 866 216 A1**
(43) Date of publication of application: **18.08.2021**
(21) Application number: 21156414.1
(22) Date of filing: 10.02.2021
(51) Int. Cl.: H01L 51/00, H01L 51/50

(54) **ORGANIC LIGHT-EMITTING DEVICE AND APPARATUS INCLUDING THE SAME**

(30) Priority: 13.02.2020 KR 20200017916
(71) Applicant: Samsung Display Co., Ltd., Yongin-si, Gyeonggi-Do 17113 (KR)
(72) Inventor: KIM, Seulong, 17113 Yongin-si (KR); KANG, Pilgu, 17113 Yongin-si (KR); KWON, Jiyoung, 17113 Yongin-si (KR); KIM, Taehyung, 17113 Yongin-si (KR); KIM, Hyoyeon, 17113 Yongin-si (KR); PARK, Huiju, 17113 Yongin-si (KR); LEE, Dongkyu, 17113 Yongin-si (KR); LEE, Seho, 17113 Yongin-si (KR); LEE, Yongdae, 17113 Yongin-si (KR); JIN, Changgwi, 17113 Yongin-si (KR); CHOI, Jungho, 17113 Yongin-si (KR)
(74) Representative: Shearman, James Ward

(57) **Abstract**

Provided are an organic light-emitting device and an apparatus including the same. The organic light-emitting device includes a first electrode; a second electrode facing the first electrode; and an organic layer between the first electrode and the second electrode. The organic layer includes m emission units; and m-1 charge generation units between two neighboring emission units among the m emission units, wherein m is an integer of 2 or more, and at least one of the m-1 charge generation units includes a heterocyclic compound represented by Formula 1 below:

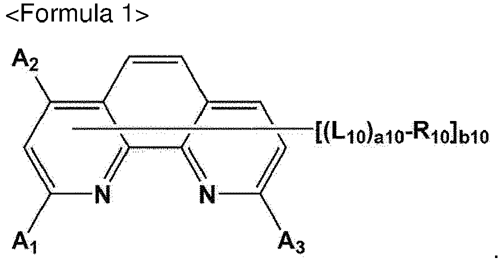

Substituents in Formula 1 may be understood as described in connection with the detailed description.

## Description

### CROSS-REFERENCE TO RELATED APPLICATION(S)

### BACKGROUND

### 1. Field

Embodiments relate to an organic light-emitting device and an apparatus including the same.

### 2. Description of the Related Art

Organic light-emitting devices are self-emission devices that produce full-color images, and also have wide viewing angles, high contrast ratios, short response times, and excellent characteristics in terms of brightness, driving voltage, and response speed, compared to devices in the art.

An organic light-emitting device may include a first electrode disposed on a substrate, a hole transport region, an emission layer, an electron transport region, and a second electrode, which are sequentially disposed on the first electrode. Holes provided from the first electrode may move toward the emission layer through the hole transport region, and electrons provided from the second electrode may move toward the emission layer through the electron transport region. Carriers, such as holes and electrons, recombine in the emission layer to produce excitons. These excitons transit from an excited state to a ground state to thereby generate light.

### SUMMARY

Embodiments include an organic light-emitting device and an apparatus including the same.

Additional aspects will be set forth in part in the description which follows and, in part, will be apparent from the description, or may be learned by practice of the embodiments of the disclosure.

According to embodiments, an organic light-emitting device includes a first electrode, a second electrode facing the first electrode, and an organic layer disposed between the first electrode and the second electrode,
wherein the organic layer includes emission units (*e.g.* m emission units, wherein m is an integer of 2 or more) and charge generation units (*e.g.* m-1 charge generation units) disposed between two neighboring ones of the emission units, wherein
at least one of the charge generation units includes a (*e.g.* at least one) heterocyclic compound represented by Formula 1 below.

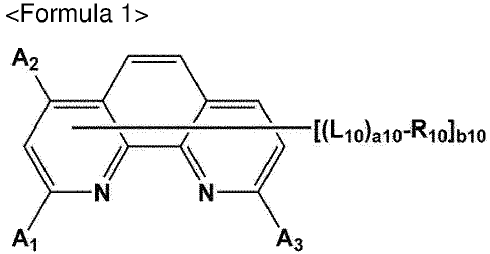

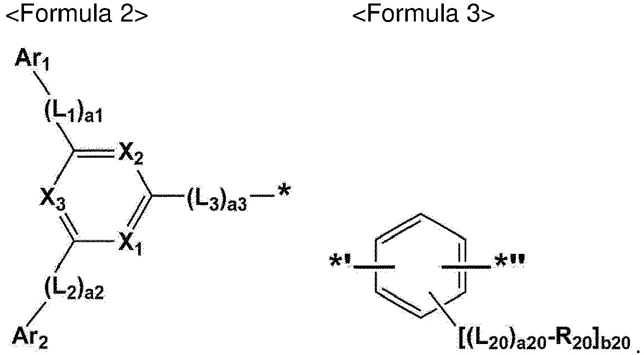

In Formulae 1 to 3,
A₁ to A₃ are each independently selected from a group represented by Formula 2, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₁)(Q₂)(Q₃), -N(Q₁)(Q₂), -B(Q₁)(Q₂), -P(Q₁)(Q₂), -C(=O)(Q₁), -S(=O)₂(Q₁), and - P(=O)(Q₁)(Q₂),
at least one selected from A₁ to A₃ is a group represented by Formula 2,
X₁ is N or C(R₃₁), X₂ is N or C(R₃₂), and X₃ is N or C(R₃₃),
at least one selected from X₁ to X₃ is N,
L₁, L₂, L₁₀, and L₂₀ are each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
a1, a2, a10, and a20 are each independently an integer from 0 to 5,
L₃ is a group represented by Formula 3,
a3 is an integer from 0 to 5,
Ar₁ and Ar₂ are each independently selected from deuterium, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₁)(Q₂), -B(Q₁)(Q₂), - C(=O)(O₁), -S(=O)₂(Q₁), and -P(=O)(O₁)(O₂),
R₁₀, R₂₀, and R₃₁ to R₃₃ are each independently selected from hydrogen, deuterium, -F, -CI, -Br, -I, a hydroxyl group, a cyano group, a nitro group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₁)(Q₂), -B(Q₁)(Q₂), - P(Q₁)(Q₂), -C(=O)(Q₁), -S(=O)₂(Q₁), and -P(=O)(O₁)(O₂),
two or more substituents selected from the group consisting of R₁₀, R₂₀, and R₃₁ to R₃₃ are optionally linked to each other to form a substituted or unsubstituted C₅-C₆₀ carbocyclic group or a substituted or unsubstituted C₁-C₆₀ heterocyclic group,
b10 is an integer from 1 to 5,
b20 is an integer from 1 to 4,
*, *', and *" each indicate a binding site to a neighboring atom, and
at least one substituent of the substituted C₅-C₆₀ carbocyclic group, the substituted C₁-C₆₀ heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is selected from
deuterium, -F, -CI, -Br, -I, a hydroxyl group, a cyano group, a nitro group, an amidino group, a hydrazine group, a hydrazone group, a C₁-C₆₀ alkyl group, a C₂C₆₀ alkenyl group, a C₂-C₆₀ alkynyl group, and a C₁-C₆₀ alkoxy group,
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -CI, - Br, -I, a hydroxyl group, a cyano group, a nitro group, an amidino group, a hydrazine group, a hydrazone group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₁₁)(Q₁₂)(Q₁₃), -N(Q₁₁)(Q₁₂), -B(Q₁₁)(Q₁₂), -P(Q₁₁)(Q₁₂), - C(=O)(Q₁₁), -S(=O)₂(Q₁₁), and -P(=O)(Q₁₁)(Q₁₂),
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, a biphenyl group, and a terphenyl group,
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, a biphenyl group, and a terphenyl group, each substituted with at least one selected from deuterium, -F, -CI, -Br, -I, a hydroxyl group, a cyano group, a nitro group, an amidino group, a hydrazine group, a hydrazone group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, a biphenyl group, a terphenyl group, -Si(Q₂₁)(Q₂₂)(Q₂₃), -N(Q₂₁)(Q₂₂), -B(Q₂₁)(Q₂₂), - P(Q₂₁)(Q₂₂), -C(=O)(Q₂₁), -S(=O)₂(Q₂₁), and -P(=O)(Q₂₁)(Q₂₂), and
-Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), - S(=O)₂(Q₃₁), and -P(=O)(Q₃₁)(Q₃₂),
wherein Q₁ to Q₃, Q₁₁ to Q₁₃, Q₂₁ to Q₂₃, and Q₃₁ to Q₃₃ are each independently selected from hydrogen, deuterium, -F, -CI, -Br, -I, a hydroxyl group, a cyano group, a nitro group, an amidino group, a hydrazine group, a hydrazone group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with a C₁-C₆₀ alkyl group, a C₁-C₆₀ heteroaryl group, a monovalent non-aromatic condensed polycyclic group, a monovalent non-aromatic condensed heteropolycyclic group, a biphenyl group, and a terphenyl group.

In an embodiment, at least one of the charge generation units may include an n-type charge generation layer and a p-type charge generation layer, and the n-type charge generation layer may include the heterocyclic compound.

In an embodiment, the n-type charge generation layer may include an electron transport compound.

In an embodiment, the n-type charge generation layer may include an alkali metal or a lanthanide metal.

In an embodiment, each of the emission units may include an emission layer, and the emission layer may include a host and a dopant.

In an embodiment, at least one of the emission units may emit blue light having a maximum emission wavelength in a range of about 410 nm to about 490 nm.

In an embodiment, at least one of the emission units may emit green light having a maximum emission wavelength in a range of about 490 nm to about 580 nm.

In an embodiment, each of the emission units may include a hole transport region and an electron transport region. The hole transport region may include at least one selected from the group consisting of a hole injection layer, a hole transport layer, a buffer layer, an emission auxiliary layer, and an electron blocking layer. The electron transport region may include at least one selected from the group consisting of a hole blocking layer, an electron transport layer, and an electron injection layer.

In an embodiment, the electron transport compound may be a metal-free compound including at least one π electron-deficient nitrogen-containing ring.

According to embodiments, an apparatus includes a substrate, the organic light-emitting device disposed on the substrate, and a color conversion layer disposed on at least one traveling direction of light emitted from the organic light-emitting device. The color conversion layer includes quantum dots.

At least some of the above and other features of the invention are set out in the claims.

### BRIEF DESCRIPTION OF THE DRAWINGS

The above and other aspects, features, and advantages of embodiments of the disclosure will be more apparent from the following description taken in conjunction with the accompanying drawings, in which:

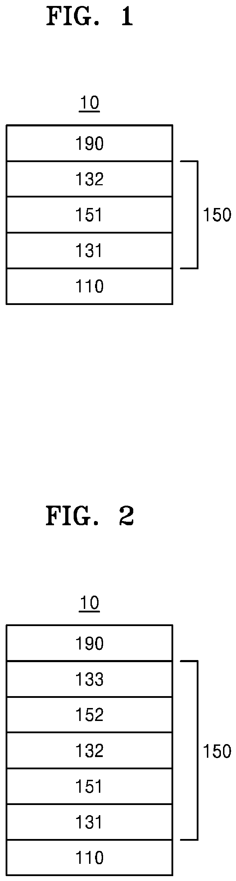

FIGS. 1 to 3 are each a schematic cross-sectional view of an organic light-emitting device according to an embodiment.

### DETAILED DESCRIPTION OF THE EMBODIMENTS

Reference will now be made in detail to embodiments, which are illustrated in the accompanying drawings, wherein like reference numerals refer to like elements throughout. In this regard, the embodiments may have different forms and should not be construed as being limited to the descriptions set forth herein. Accordingly, the embodiments are merely described below, by referring to the figures, to explain aspects of the description.

As used herein, the term "and/or" includes any and all combinations of one or more of the associated listed items. For example, "A and/or B" may be understood to mean "A, B, or A and B." The terms "and" and "or" may be used in the conjunctive or disjunctive sense and may be understood to be equivalent to "and/or". Throughout the disclosure, the expression "at least one of A, B, or C" may indicate only A, only B, only C, both A and B, both A and C, both B and C, all of A, B, and C, or variations thereof.

The term "at least one of" is intended to include the meaning of "at least one selected from the group consisting of" for the purpose of its meaning and interpretation. For example, "at least one of A and B" may be understood to mean "A, B, or A and B." When preceding a list of elements, the term, "at least one of," modifies the entire list of elements and does not modify the individual elements of the list.

Hereinafter, embodiments of the disclosure will be described in detail with reference to the accompanying drawings. The same or corresponding components will be denoted by the same reference numerals, and thus redundant description thereof will be omitted.

As used herein, the singular forms "a," "an" and "the" are intended to include the plural forms as well, unless the context clearly indicates otherwise.

It will be further understood that the terms "comprises", "includes", and/or "contains" as used herein specify the presence of stated features or components, but do not preclude the presence or addition of one or more other features or components.

It will be understood that when a layer, region, or component is referred to as being "on" or "onto" another layer, region, or component, it may be directly or indirectly formed on the other layer, region, or component. For example, intervening layers, regions, or components may be present.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 20%, 10%, or 5% of the stated value.

Sizes of elements in the drawings may be exaggerated for convenience of explanation. In other words, since sizes and thicknesses of components in the drawings are arbitrarily illustrated for convenience of explanation, the following embodiments of the disclosure are not limited thereto.

### [Description of FIGS. 1 to 3]

FIGS. 1 to 3 each illustrate a schematic cross-sectional view of an organic light-emitting device 10 according to an embodiment. The organic light-emitting device 10 includes: a first electrode 110; a second electrode 190 facing the first electrode 110; and an organic layer 150 between the first electrode 110 and the second electrode 190, wherein the organic layer 150 includes: m emission units; and

m-1 charge generation units between two neighboring emission units among the m emission units.

The term "organic layer" as used herein refers to a single layer and/or all layers between the first electrode and the second electrode of the organic light-emitting device. A material included in "the organic layer" is not limited to an organic material.

Hereinafter, the structure of the organic light-emitting device 10 according to an embodiment and a method of manufacturing the organic light-emitting device 10 will be described in connection with FIG. 1.

In an embodiment, m may be an integer of 2 or more.

In embodiments, m may be an integer from 2 to 5. For example, m may be 2, 3, or 4.

FIG. 1 illustrates the organic light-emitting device 10 in an embodiment where m is 2, where the organic light-emitting device 10 includes two emission units and one charge generation unit. Referring to FIG. 1, the organic light-emitting device 10 includes a first electrode 110, a first emission unit 131 on the first electrode 110, a first charge generation unit 151 on the first emission unit 131, a second emission unit 132 on the first charge generation unit 151, and a second electrode 190 on the second emission unit 132.

FIG. 2 illustrates the organic light-emitting device 10 in an embodiment where m is 3, where the organic light-emitting device 10 includes three emission units and two charge generation units. Referring to FIG. 2, the organic light-emitting device 10 includes a first electrode 110, a first emission unit 131 on the first electrode 110, a first charge generation unit 151 on the first emission unit 131, a second emission unit 132 on the first charge generation unit 151, a second charge generation unit 152 on the second emission unit 132, a third emission unit 133 on the second charge generation unit 152, and a second electrode 190 on the third emission unit 133.

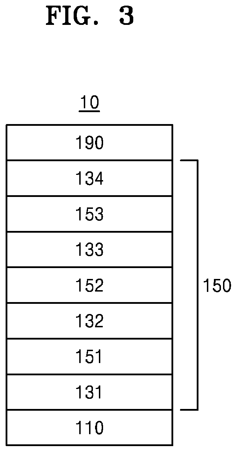

FIG. 3 illustrates the organic light-emitting device 10 in an embodiment where m is 4, where the organic light-emitting device 10 includes four emission units and three charge generation units. Referring to FIG. 3, the organic light-emitting device 10 includes a first electrode 110, a first emission unit 131 on the first electrode 110, a first charge generation unit 151 on the first emission unit 131, a second emission unit 132 on the first charge generation unit 151, a second charge generation unit 152 on the second emission unit 132, a third emission unit 133 on the second charge generation unit 152, a third charge generation unit 153 on the third emission unit 133, a fourth emission unit 134 on the third charge generation unit 153, and the second electrode 190 on the fourth emission unit 134.

At least one of the m-1 charge generation units includes a heterocyclic compound represented by Formula 1 below.

At least one of the m-1 charge generation units may include an n-type charge generation layer, and the n-type charge generation layer may include the heterocyclic compound.

The heterocyclic compound may be understood by referring to the related description to be presented later.

In an embodiment, the n-type charge generation layer may further include an electron transport compound.

The electron transport compound may be understood by referring to the description of an electron transport compound in an electron transport region to be presented later.

For example, the term "electron transport compound" as used herein may be a metal-free compound including at least one π electron-deficient nitrogen-containing ring.

In an embodiment, the electron transport compound may be different from the heterocyclic compound.

When the n-type charge generation layer includes the heterocyclic compound and the electron transport compound, which enhance electron transport properties by reducing the intermolecular control, free volume caused by crystallization that may occur partially at the interface of the n-type charge generation layer may be produced, leading to generation of an electron trap site. In terms of the configuration of the charge generation layer, when the electron transport compound and the heterocyclic compound are combined and arranged in the n-type charge generation layer, crystallization occurring at the interface between an electron transport region and a charge generation unit may be controlled, so that the morphology of the interface may be improved to be uniform. Accordingly, the charge flow in the organic light-emitting device may be improved, thereby reducing driving voltage.

In an embodiment, the n-type charge generation layer may further include an alkali metal or a lanthanide metal.

For example, the n-type charge generation layer may further include lithium (Li) or ytterbium (Yb).

In an embodiment, in the n-type charge generation layer, a weight ratio of the heterocyclic compound to the alkali metal or the lanthanide metal may be in a range of about 99.9:0.1 to about 90:10.

When the n-type charge generation layer is doped with the alkali metal or the lanthanide metal, the metal may form a complex with a nitrogen (N) atom of the heterocyclic compound included in the n-type charge generation layer, resulting in a lower LUMO level than that of the heterocyclic compound. In this regard, an appropriate energy level may result between adjacent layers, such as an electron transport layer and a p-type charge generation layer, and an energy barrier between these two layers may be reduced. Accordingly, charges generated from the p-type charge generation layer may be easily transferred to the electron transport layer.

In an embodiment, the charge generation unit may further include the p-type charge generation layer.

In an embodiment, the p-type charge generation layer may be used by doping with an organic material including a strong electron-withdrawing group to promote charge generation. A charge generation material may be understood by referring to the description of a charge generation material included in a hole transport region to be presented later.

In an embodiment, each of the m emission units may include an emission layer, and the emission layer may include a host and a dopant. The host and the dopant included in the emission layer may be understood by referring to the description of a host and a dopant included in an emission layer to be presented later.

According to an embodiment, at least one of the m emission units may emit blue light having a maximum emission wavelength of about 410 nm to about 490 nm.

For example, the organic light-emitting device may be a tandem blue organic light-emitting device in which each of the m emission units emits blue light having a maximum emission wavelength of about 410 nm to about 490 nm.

In an embodiment, at least one of the m emission units may emit green light having a maximum emission wavelength of about 490 nm to about 580 nm.

In an embodiment, assuming that the organic light-emitting device includes three emission units that are regarded as a first emission unit, a second emission unit, and a third emission unit from the one closest to the first electrode, the first emission unit may emit blue light, the second emission unit may emit green light, and the third emission unit may emit blue light. In embodiments, the first emission unit may emit blue light, the second emission unit may emit blue light, and the third emission unit may emit green light.

In embodiments, assuming that the organic light-emitting device includes four emission units that are regarded as a first emission unit, a second emission unit, a third emission unit, and a fourth emission unit from the one closest to the first electrode, the first emission unit may emit blue light, the second emission unit may emit blue light, the third emission unit may emit green light, and the fourth emission unit may emit blue light. In embodiments, the first emission unit may emit blue light, the second emission unit may emit blue light, the third emission unit may emit blue light, and the fourth emission unit may emit green light.

In an embodiment, each of the m emission units may further include a hole transport region and an electron transport region,
the hole transport region may include at least one selected from a hole injection layer, a hole transport layer, a buffer layer, an emission auxiliary layer, and an electron blocking layer, and
the electron transport region may include at least one selected from a hole blocking layer, an electron transport layer, and an electron injection layer.

Regarding the stacking order of the emission units, a hole transport region, an emission layer, and an electron transport region may be stacked in the stated order from the first electrode.

In an embodiment, the electron transport region may include an electron transport layer, and the electron transport layer may directly contact the n-type charge generation layer of the charge generation unit.

In an embodiment, each of the n-type charge generation layer and the electron transport layer may further include an electron transport compound. In embodiments, an electron transport compound included in the n-type charge generation layer and an electron transport compound included in the electron transport layer may be identical to or different from each other.

The heterocyclic compound is represented by Formula 1 below:

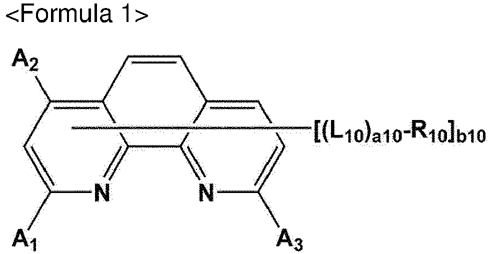

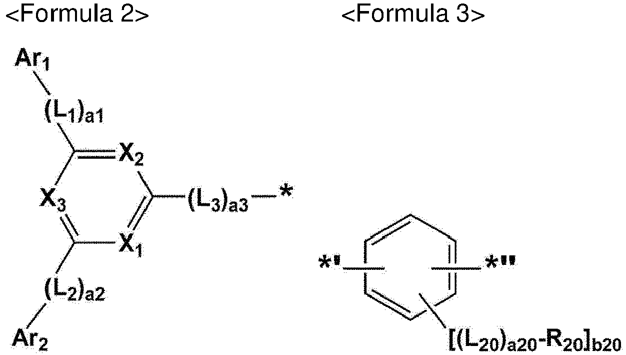

In Formula 1, A₁ to A₃ are each independently selected from a group represented by Formula 2, a substituted or unsubstituted C₁-C₆₀ (*e.g*. C₁-C₂₀) alkyl group, a substituted or unsubstituted C₂-C₆₀ (*e.g*. C₂-C₂₀) alkenyl group, a substituted or unsubstituted C₂-C₆₀ (*e.g*. C₂-C₂₀) alkynyl group, a substituted or unsubstituted C₁-C₆₀ (*e.g*. C₁-C₂₀) alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g*. C₆-C₃₀) aryl group, a substituted or unsubstituted C₆-C₆₀ (*e.g*. C₆-C₃₀) aryloxy group, a substituted or unsubstituted C₆-C₆₀ (*e.g*. C₆-C₃₀) arylthio group, a substituted or unsubstituted C₁-C₆₀ (*e.g*. C₁-C₂₀) heteroaryl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₁)(Q₂), -B(Q₁)(Q₂), -P(Q₁)(Q₂), -C(=O)(Q₁), -S(=O)₂(Q₁), and -P(=O)(O₁)(O₂), and
at least one selected from A₁ to A₃ is a group represented by Formula 2.

In an embodiment, one (*e.g*. at least one) selected from A₁ to A₃ may be a group represented by Formula 2, and
the remaining substituents may each independently be selected from a substituted or unsubstituted C₁-C₆₀ (*e.g*. C₁-C₂₀) alkyl group, a substituted or unsubstituted C₆-C₆₀ (*e.g.* C₆-C₃₀) aryl group, a substituted or unsubstituted C₁-C₆₀ (*e.g.* C₁-C₂₀) heteroaryl group, a substituted or unsubstituted monovalent non-aromatic condensed polycyclic group, and a substituted or unsubstituted monovalent non-aromatic condensed heteropolycyclic group.

In an embodiment, one (*e.g*. at least one) selected from A₁ to A₃ may be a group represented by Formula 2, and
the remaining substituents may each independently be selected from:
a methyl group, an ethyl group, a propyl group, an isobutyl group, a sec-butyl group, a ter-butyl group, a pentyl group, an isoamyl group, and a hexyl group;
a methyl group, an ethyl group, a propyl group, an isobutyl group, a sec-butyl group, a ter-butyl group, a pentyl group, an isoamyl group, and a hexyl group, each substituted with at least one selected from deuterium, -F, -CI, -Br, -I, a hydroxyl group, a cyano group, a nitro group, an amino group, a C₁-C₆₀ (*e.g*. C₁-C₂₀) alkyl group, a C₆-C₆₀ (*e.g.* C₆-C₃₀) aryl group, and a C₁-C₆₀ (*e.g.* C₂-C₂₀) heteroaryl group;
a phenyl group, a naphthyl group, a pyridyl group, a pyrimidyl group, a triazinyl group, a quinoline group, an isoquinoline group, a biphenyl group, and a terphenyl group; and
a phenyl group, a naphthyl group, a pyridyl group, a pyrimidyl group, a triazinyl group, a quinoline group, an isoquinoline group, a biphenyl group, and a terphenyl group, each substituted with at least one selected from deuterium, -F, -CI, -Br, -I, a hydroxyl group, a cyano group, a nitro group, an amino group, a C₁-C₆₀ (*e.g*. C₁-C₂₀) alkyl group, a C₆-C₆₀ (*e.g.* C₆-C₃₀) aryl group, and a C₁-C₆₀ (*e.g.* C₂-C₂₀) heteroaryl group.

In Formula 2, X₁ is N or C(R₃₁), X₂ is N or C(R₃₂), X₃ is N or C(R₃₃), and at least one of X₁ to X₃ is N.

In embodiments, at least one of X₁ to X₃ may be N.

In embodiments, two substituents of X₁ to X₃ may be N.

In an embodiment, X₁ to X₃ may each be N.

In Formulae 1 to 3, L₁, L₂, L₁₀, and L₂₀ are each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (*e.g*. C₆-C₃₂) arylene group, a substituted or unsubstituted C₁-C₆₀ (*e.g*. C₁-C₂₀) heteroarylene group, a substituted or unsubstituted divalent non-aromatic condensed polycyclic group, and a substituted or unsubstituted divalent non-aromatic condensed heteropolycyclic group.

In an embodiment, in Formulae 1 to 3, L₁, L₂, L₁₀, and L₂₀ may each independently be selected from:
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a spiro-fluorene-benzofluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a dibenzosilolylene group, a benzocarbazolylene group, a dibenzocarbazolylene group, a thiadiazolylene group, an imidazopyridinylene group, and an imidazopyrimidinylene group; and
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a spiro-fluorene-benzofluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a dibenzosilolylene group, a benzocarbazolylene group, a dibenzocarbazolylene group, a thiadiazolylene group, an imidazopyridinylene group, and an imidazopyrimidinylene group, each substituted with at least one selected from deuterium, -F, -CI, -Br, -I, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolylene group, a benzocarbazolyl group, a dibenzocarbazolyl group, a thiadiazolyl group, an imidazopyridinyl group, an imidazopyrimidinyl group, -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
wherein Q₃₁ to Q₃₃ may each independently be selected from a C₁-C₁₀ alkyl group, a C₁-C₁₀ alkoxy group, a phenyl group, a phenyl group substituted with a C₁-C₁₀ alkyl group, a biphenyl group, a terphenyl group, a naphthyl group, a pyridinyl group, a pyrimidyl group, a quinolinyl group, and an isoquinolinyl group.

In an embodiment, L₁, L₂, L₁₀, and L₂₀ may each independently be a group represented by one of Formulae 3-1 to 3-99 below:

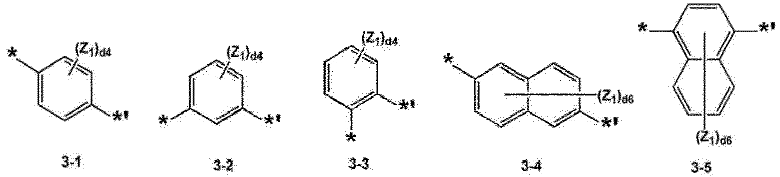

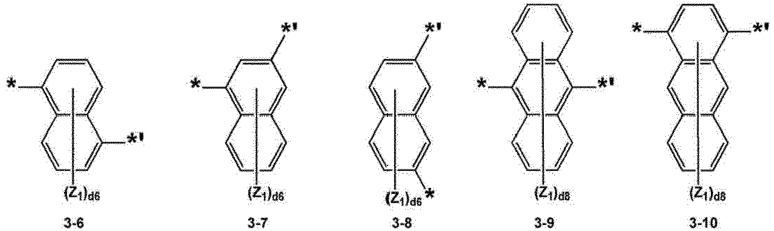

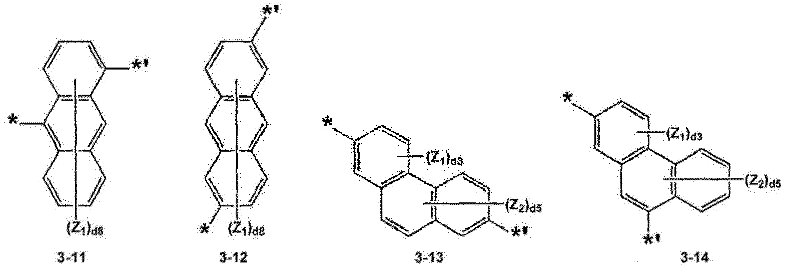

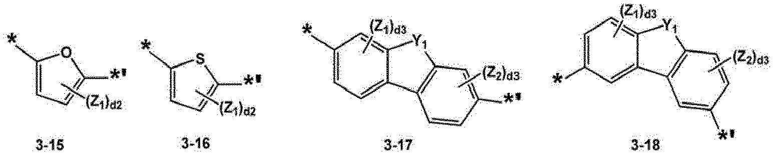

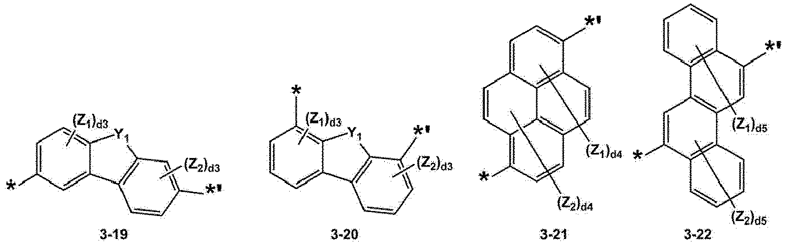

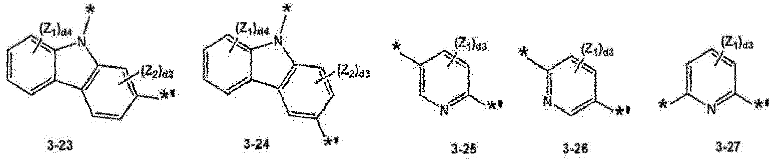

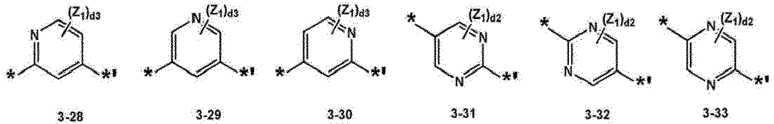

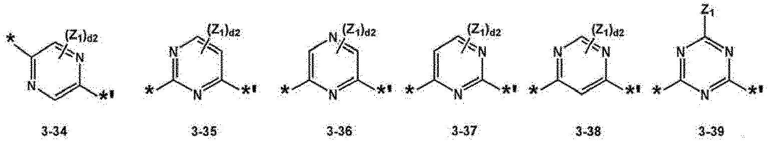

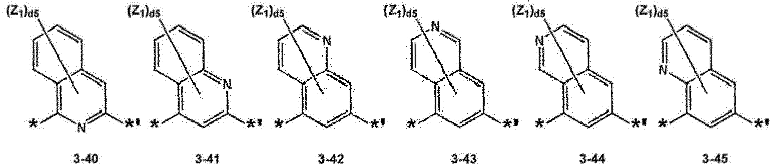

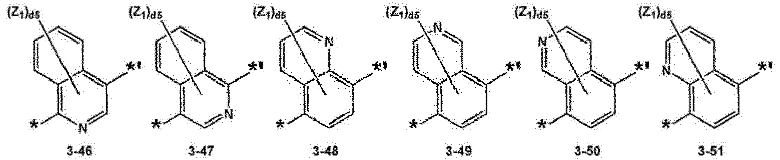

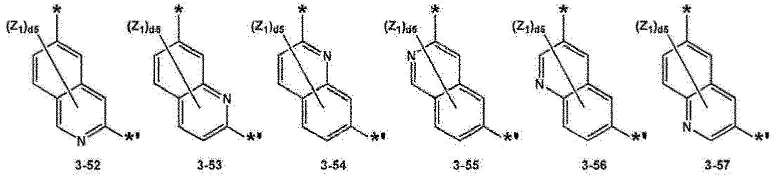

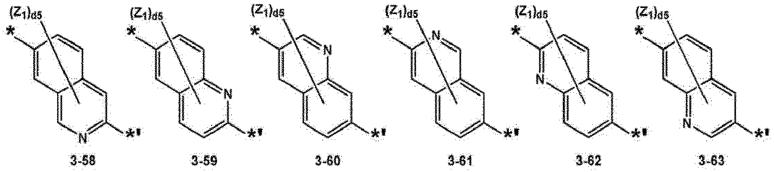

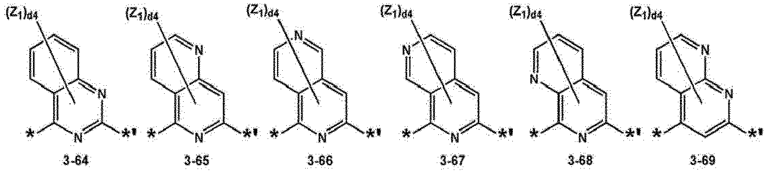

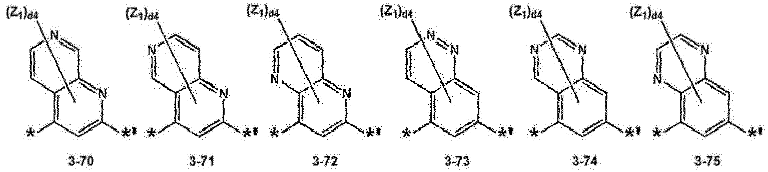

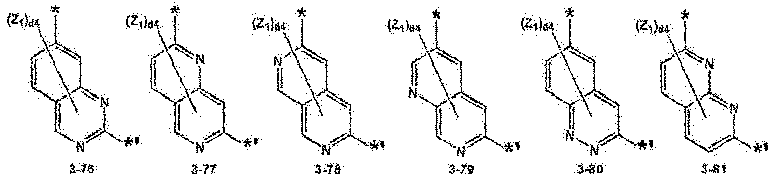

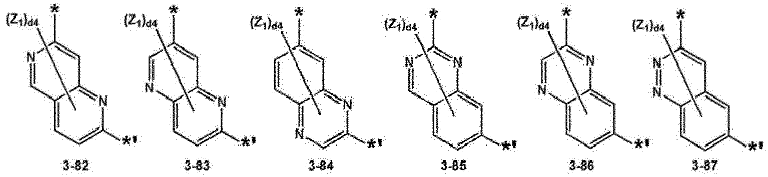

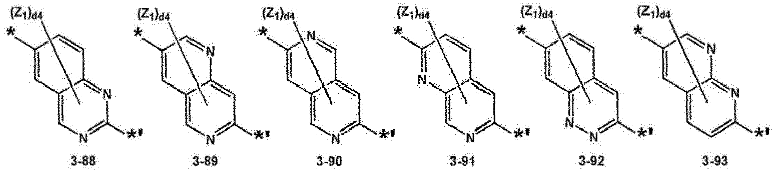

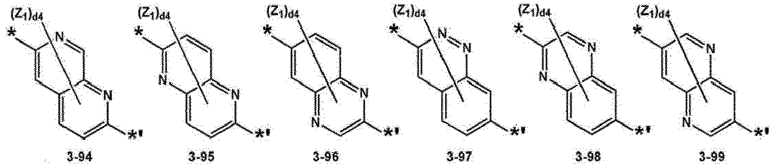

In Formulae 3-1 to 3-99,
Y₁ is O, S, C(Z₃)(Z₄), N(Z₅), or Si(Z₆)(Z₇),
wherein Z₁ to Z₇ are each independently selected from hydrogen, deuterium, -F, -CI, -Br, -I, a hydroxyl group, a cyano group, a nitro group, an amidino group, a hydrazine group, a hydrazone group, a C₁-C₂₀ (*e.g.* C₁-C₁₀) alkyl group, a C₁-C₂₀ (*e.g.* C₁-C₁₀) alkoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a carbazolyl group, a benzocarbazolyl group, a dibenzocarbazolyl group, a thiadiazolyl group, an imidazopyridinyl group, an imidazopyrimidinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, -Si(Q₃₁)(Q₃₂)(Q₃₃), -N(Q₃₁)(Q₃₂), -B(Q₃₁)(Q₃₂), -C(=O)(Q₃₁), - S(=O)₂(Q₃₁), and -P(=O)(Q₃₁)(Q₃₂),
wherein Q₃₁ to Q₃₃ are each independently selected from:
a C₁-C₁₀ alkyl group, a C₁-C₁₀ alkoxy group, a phenyl group, a biphenyl group, a terphenyl group, a naphthyl group, a pyridinyl group, a pyrimidinyl group, a pyrazinyl group, a quinolinyl group, an isoquinolinyl group, a quinoxalinyl group, and a quinazolinyl group; and
a phenyl group, a biphenyl group, a terphenyl group, a naphthyl group, a pyridinyl group, a pyrimidinyl group, a pyrazinyl group, a quinolinyl group, an isoquinolinyl group, a quinoxalinyl group, and a quinazolinyl group, each substituted with at least one selected from a C₁-C₁₀ alkyl group, a C₁-C₁₀ alkoxy group, and a phenyl group,
d2 is an integer from 0 to 2,
d3 is an integer from 0 to 3,
d4 is an integer from 0 to 4,
d5 is an integer from 0 to 5,
d6 is an integer from 0 to 6,
d8 is an integer from 0 to 8, and
* and *' each indicate a binding site to a neighboring atom.

In Formulae 1 to 3, a1, a2, a10, and a20 are each independently an integer from 0 to 5.

In Formula 2, L₃ is a group represented by Formula 3.

In an embodiment, L₃ may be a group represented by one of Formulae 4-1 to 4-3 below: 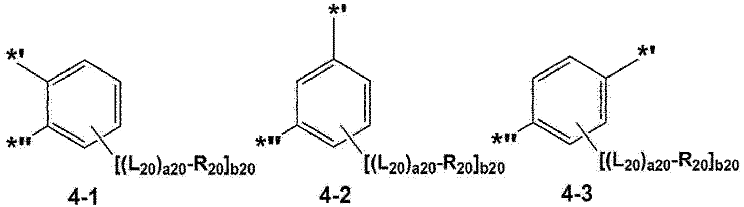 wherein, in Formulae 4-1 to 4-3,
L₂₀, a20, R₂₀, and b20 are the same as described in connection with Formula 3, and
*' and *" each indicate a binding site to a neighboring atom.

In Formula 2, a3 is an integer from 0 to 5.

In Formula 2, Ar₁ and Ar₂ are each independently selected from deuterium, a substituted or unsubstituted C₁-C₆₀ (*e.g*. C₁-C₂₀) alkyl group, a substituted or unsubstituted C₂-C₆₀ (*e.g*. C₂-C₂₀) alkenyl group, a substituted or unsubstituted C₂-C₆₀ (*e.g.* C₂-C₂₀) alkynyl group, a substituted or unsubstituted C₁-C₆₀ (*e.g.* C₁-C₂₀) alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g*. C₆-C₃₀) aryl group, a substituted or unsubstituted C₆-C₆₀ (*e.g*. C₆-C₃₀) aryloxy group, a substituted or unsubstituted C₆-C₆₀ (*e.g.* C₆-C₃₀) arylthio group, a substituted or unsubstituted C₁-C₆₀ (*e.g.* C₁-C₂₀) heteroaryl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₁)(Q₂), -B(Q₁)(Q₂), -C(=O)(Q₁), - S(=O)₂(Q₁), and -P(=O)(Q₁)(Q₂).

In an embodiment, Ar₁ and Ar₂ may each independently be selected from deuterium, a substituted or unsubstituted C₆-C₆₀ (*e.g*. C₆-C₃₀) aryl group, a substituted or unsubstituted C₆-C₆₀ (*e.g*. C₆-C₃₀) aryloxy group, a substituted or unsubstituted C₆-C₆₀ (*e.g.* C₆-C₃₀) arylthio group, and a substituted or unsubstituted C₁-C₆₀ (*e.g.* C₁-C₂₀) heteroaryl group.

In an embodiment, Ar₁ and Ar₂ may each independently be selected from: deuterium; and
a group represented by one of Formulae 5-1 to 5-26 and 6-1 to 6-55 below:

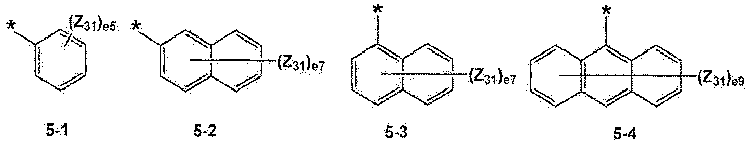

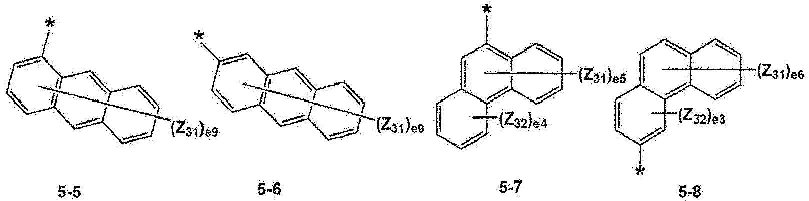

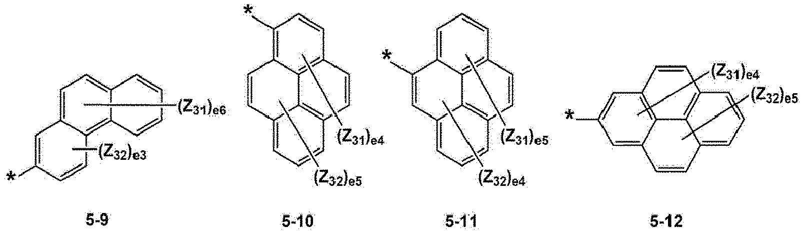

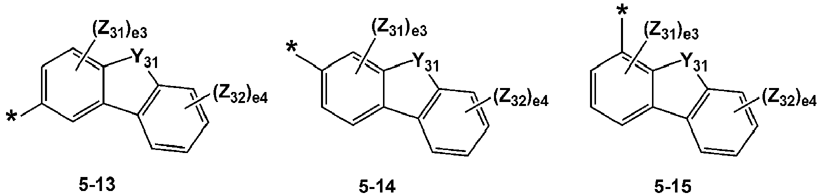

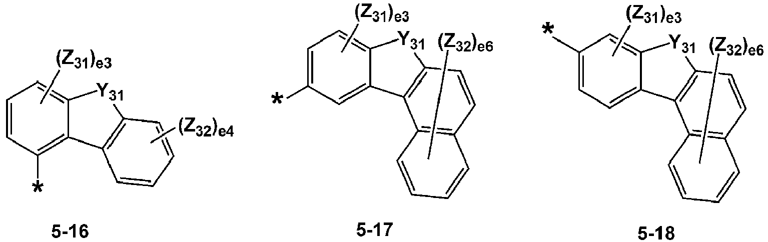

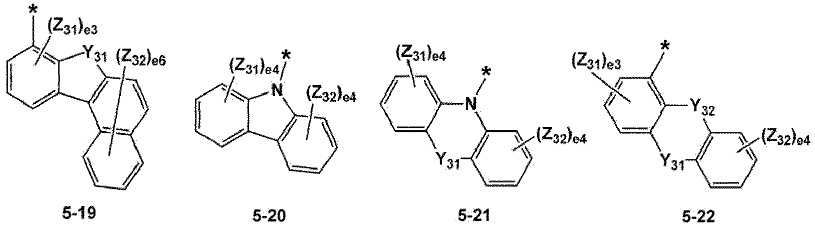

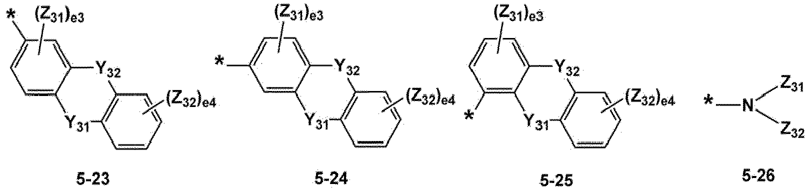

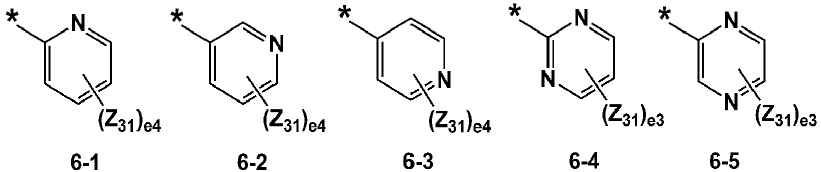

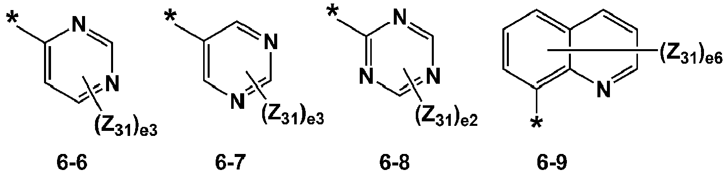

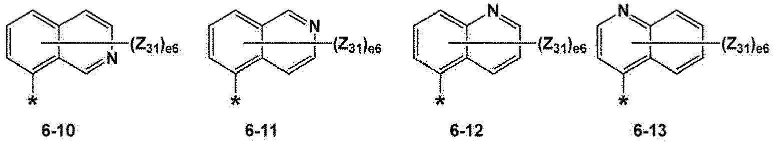

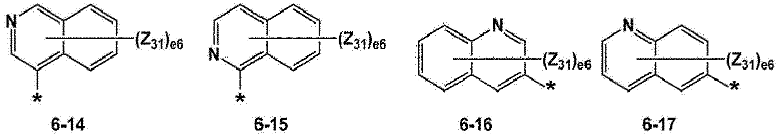

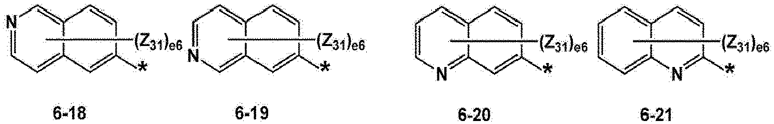

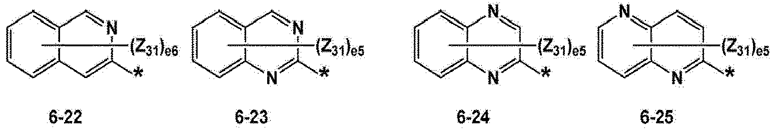

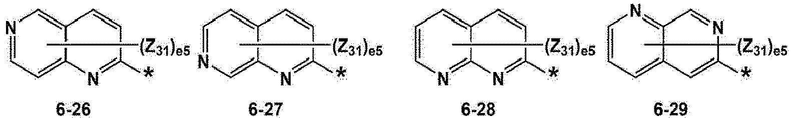

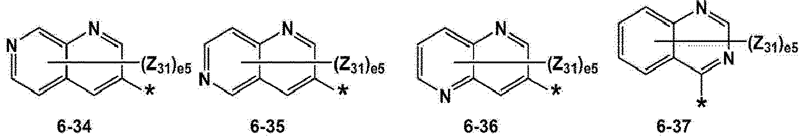

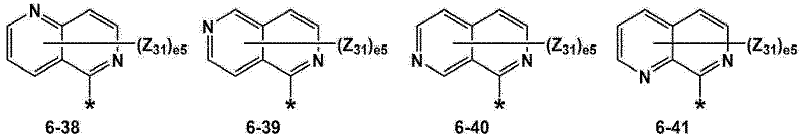

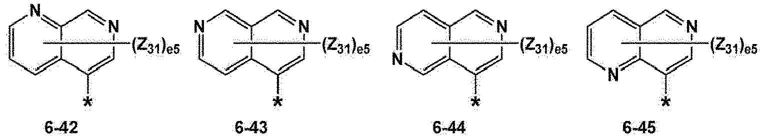

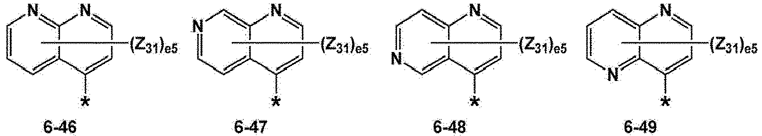

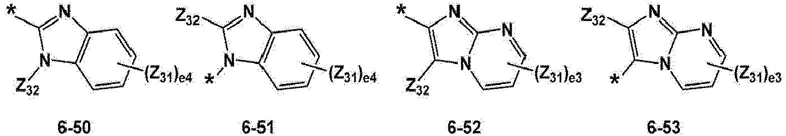

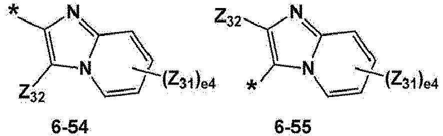

In Formulae 5-1 to 5-26 and 6-1 to 6-55,
Y31 and Y32 are each independently O, S, C(Z₃₃)(Z₃₄), N(Z₃₃), or Si(Z₃₃)(Z₃₄),
Z₃₁ to Z₃₄ are each independently selected from hydrogen, deuterium, -F, -CI, -Br, -I, a hydroxyl group, a cyano group, a nitro group, an amidino group, a hydrazino group, a hydrazono group, a C₁-C₂₀ (*e.g.* C₁-C₁₀) alkyl group, a C₂-C₂₀ (*e.g.* C₂-C₁₀) alkenyl group, a C₂-C₂₀ (*e.g.* C₂-C₁₀) alkynyl group, a C₁-C₂₀ (*e.g.* C₁-C₁₀) alkoxy group, a phenyl group, a biphenyl group, a terphenyl group, a naphthyl group, a fluorenyl group, a spiro-bifluorenyl group, a phenanthrenyl group, an anthracenyl group, a triphenylenyl group, a pyridinyl group, a pyrimidinyl group, a carbazolyl group, and a triazinyl group,
e2 is 1 or 2,
e3 is an integer from 1 to 3,
e4 is an integer from 1 to 4,
e5 is an integer from 1 to 5,
e6 is an integer from 1 to 6,
e7 is an integer from 1 to 7,
e9 is an integer from 1 to 9, and
* indicates a binding site to a neighboring atom.

In an embodiment, Ar₁ and Ar₂ may each independently be selected from:
a phenyl group, a naphthyl group, a pyridyl group, a pyrimidyl group, a triazinyl group, a quinoline group, an isoquinoline group, a biphenyl group, and a terphenyl group; and
a phenyl group, a naphthyl group, a pyridyl group, a pyrimidyl group, a triazinyl group, a quinoline group, an isoquinoline group, a biphenyl group, and a terphenyl group, each substituted with at least one selected from deuterium, -F, -CI, -Br, -I, a hydroxyl group, a cyano group, a nitro group, an amino group, a C₁-C₆₀ (*e.g*. C₁-C₂₀) alkyl group, a C₆-C₆₀ (*e.g.* C₆-C₃₀) aryl group, and a C₁-C₆₀ (*e.g.* C₂-C₂₀) heteroaryl group.

In Formulae 1 to 3, R₁₀, R₂₀, and R₃₁ to R₃₃ are each independently selected from hydrogen, deuterium, -F, -CI, -Br, -I, a hydroxyl group, a cyano group, a nitro group, a substituted or unsubstituted C₁-C₆₀ (*e.g.* C₁-C₂₀) alkyl group, a substituted or unsubstituted C₂-C₆₀ (*e.g*. C₂-C₂₀) alkenyl group, a substituted or unsubstituted C₂-C₆₀ (*e.g.* C₂-C₂₀) alkynyl group, a substituted or unsubstituted C₁-C₆₀ (*e.g.* C₁-C₂₀) alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g*. C₆-C₃₀) aryl group, a substituted or unsubstituted C₆-C₆₀ (*e.g*. C₆-C₃₀) aryloxy group, a substituted or unsubstituted C₆-C₆₀ (*e.g.* C₆-C₃₀) arylthio group, a substituted or unsubstituted C₁-C₆₀ (*e.g.* C₁-C₂₀) heteroaryl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₁)(Q₂), -B(Q₁)(Q₂), -P(Q₁)(Q₂), - C(=O)(Q₁), -S(=O)₂(Q₁), and -P(=O)(Q₁)(Q₂).

In an embodiment, R₁₀, R₂₀, and R₃₁ to R₃₃ may each independently be selected from:
hydrogen, deuterium, -F, -CI, -Br, -I, a cyano group, a C₁-C₂₀ (*e.g*. C₁-C₁₀) alkyl group, and a C₁-C₂₀ (*e.g*. C₁-C₁₀) alkoxy group;
a C₁-C₂₀ (*e.g.* C₁-C₁₀) alkyl group and a C₁-C₂₀ (*e.g.* C₁-C₁₀) alkoxy group, each substituted with at least one selected from deuterium, -F, -CI, -Br, -I, a cyano group, a phenyl group, and a biphenyl group;
a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a benzoisoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, a benzoisothiazolyl group, a benzoxazolyl group, a benzoisoxazolyl group, a triazolyl group, a tetrazolyl group, a thiadiazolyl group, an oxadiazolyl group, a triazinyl group, a carbazolyl group, a dibenzofuranyl group, a dibenzothiophenyl group, a dibenzosilolyl group, a benzocarbazolyl group, a naphthobenzofuranyl group, a naphthobenzothiophenyl group, a naphthobenzosilolyl group, a dibenzocarbazolyl group, a dinaphthofuranyl group, a dinaphthothiophenyl group, a dinaphtho silolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indenopyrrolyl group, an indolopyrrolyl group, an indeno carbazolyl group, and an indolocarbazolyl group;
a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, an isoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, a benzoisothiazolyl group, a benzoxazolyl group, a benzoisoxazolyl group, a triazolyl group, a tetrazolyl group, a thiadiazolyl group, an oxadiazolyl group, a triazinyl group, a carbazolyl group, a dibenzofuranyl group, a dibenzothiophenyl group, a dibenzosilolyl group, a benzocarbazolyl group, a naphthobenzofuranyl group, a naphthobenzothiophenyl group, a naphthobenzosilolyl group, a dibenzocarbazolyl group, a dinaphthofuranyl group, a dinaphthothiophenyl group, a dinaphtho silolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indenopyrrolyl group, an indolopyrrolyl group, an indeno carbazolyl group, and an indolocarbazolyl group, each substituted with at least one selected from deuterium, -F, -CI, -Br, -I, a cyano group, a C₁-C₂₀ (*e.g*. C₁-C₁₀) alkyl group, a C₁-C₂₀ (*e.g*. C₁-C₁₀) alkoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, an isoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, a benzoisothiazolyl group, a benzoxazolyl group, a benzoisoxazolyl group, a triazolyl group, a tetrazolyl group, a thiadiazolyl group, an oxadiazolyl group, a triazinyl group, a carbazolyl group, a dibenzofuranyl group, a dibenzothiophenyl group, a dibenzosilolyl group, a benzocarbazolyl group, a naphthobenzofuranyl group, a naphthobenzothiophenyl group, a naphthobenzosilolyl group, a dibenzocarbazolyl group, a dinaphthofuranyl group, a dinaphthothiophenyl group, a dinaphtho silolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indenopyrrolyl group, an indolopyrrolyl group, an indeno carbazolyl group, an indolocarbazolyl group, -Si(Q₃₁)(Q₃₂)(Q₃₃), -N(Q₃₁)(Q₃₂), - B(Q₃₁)(Q₃₂), -C(=O)(Q₃₁), -S(=O)(Q₃₁), -S(=O)₂(Q₃₁), -P(=O)(Q₃₁)(Q₃₂), and - P(=S)(Q₃₁)(Q₃₂); and
-Si(Q₁)(Q₂)(Q₃), -B(Q₁)(Q₂), -N(Q₁)(Q₂), -P(Q₁)(Q₂), -C(=O)(Q₁), -S(=O)(Q₁), - S(=O)₂(Q₁), -P(=O)(Q₁)(Q₂), and -P(=S)(Q₁)(Q₂),
wherein Q₁ to Q₃ and Q₃₁ to Q₃₃ may each independently be selected from:
   a C₁-C₁₀ alkyl group, a C₁-C₁₀ alkoxy group, a phenyl group, a biphenyl group, a terphenyl group, a naphthyl group, a pyridinyl group, a pyrimidinyl group, a pyrazinyl group, a quinolinyl group, an isoquinolinyl group, a quinoxalinyl group, and a quinazolinyl group; and
   a phenyl group, a biphenyl group, a terphenyl group, a naphthyl group, a pyridinyl group, a pyrimidinyl group, a pyrazinyl group, a quinolinyl group, an isoquinolinyl group, a quinoxalinyl group, and a quinazolinyl group, each substituted with at least one selected from a C₁-C₁₀ alkyl group, a C₁-C₁₀ alkoxy group, and a phenyl group.

In an embodiment, R₁₀ and R₂₀ may each independently be selected from:
a methyl group, an ethyl group, a propyl group, an isobutyl group, a sec-butyl group, a ter-butyl group, a pentyl group, an isoamyl group, and a hexyl group;
a methyl group, an ethyl group, a propyl group, an isobutyl group, a sec-butyl group, a ter-butyl group, a pentyl group, an isoamyl group, and a hexyl group, each substituted with at least one selected from deuterium, -F, -CI, -Br, -I, a hydroxyl group, a cyano group, a nitro group, an amino group, a C₁-C₆₀ (*e.g*. C₁-C₂₀) alkyl group, a C₆-C₆₀ (*e.g.* C₆-C₃₀) aryl group, and a C₁-C₆₀ (*e.g.* C₂-C₂₀) heteroaryl group;
a phenyl group, a naphthyl group, a pyridyl group, a pyrimidyl group, a triazinyl group, a quinoline group, an isoquinoline group, a biphenyl group, and a terphenyl group; and
a phenyl group, a naphthyl group, a pyridyl group, a pyrimidyl group, a triazinyl group, a quinoline group, an isoquinoline group, a biphenyl group, and a terphenyl group, each substituted with at least one selected from deuterium, -F, -CI, -Br, -I, a hydroxyl group, a cyano group, a nitro group, an amino group, a C₁-C₆₀ (*e.g*. C₁-C₂₀) alkyl group, a C₆-C₆₀ (*e.g.* C₆-C₃₀) aryl group, and a C₁-C₆₀ (*e.g.* C₂-C₂₀) heteroaryl group.

In Formulae 1 to 3, any two or more substituents among R₁₀, R₂₀, and R₃₁ to R₃₃ are optionally linked to each other to form a substituted or unsubstituted C₅-C₆₀ (*e.g.* C₅-C₃₀) carbocyclic group or a substituted or unsubstituted C₁-C₆₀ (*e.g.* C₁-C₂₀) heterocyclic group.

In an embodiment, any two or more substituents among R₁₀, R₂₀, and R₃₁ to R₃₃ may optionally be linked to each other to form a benzene, a naphthalene, a fluorene, a spiro-fluorene, an indene, a pyrrole, a thiophene, a furan, an imidazole, a pyrazole, a thiazole, an isothiazole, an oxazole, an isoxazole, a pyridine, a pyrazine, a pyrimidine, a pyridazine, a quinoline, an isoquinoline, a benzoquinoline, a quinoxaline, a quinazoline, a carbazole, a benzimidazole, a benzofuran, a benzothiophene, an isobenzothiophene, a benzoxazole, an isobenzoxazole, a triazole, an oxadiazole, a triazine, a dibenzofuran or a dibenzothiophene; or
a benzene, a naphthalene, a fluorene, a spiro-fluorene, an indene, a pyrrole, a thiophene, a furan, an imidazole, a pyrazole, a thiazole, an isothiazole, an oxazole, an isoxazole, a pyridine, a pyrazine, a pyrimidine, a pyridazine, a quinoline, an isoquinoline, a benzoquinoline, a quinoxaline, a quinazoline, a carbazole, a benzimidazole, a benzofuran, a benzothiophene, an isobenzothiophene, a benzoxazole, an isobenzoxazole, a triazole, an oxadiazole, a triazine, a dibenzofuran, or a dibenzothiophene, each substituted with at least one selected from deuterium, -F, -CI, -Br, -I, a cyano group, and a C₁-C₆₀ (*e.g*. C₁-C₂₀) alkyl group.

In Formulae 1 to 3, b10 is an integer from 1 to 5.

In Formulae 1 to 3, b20 is an integer from 1 to 4.

In an embodiment, a10 may be 0, and R₁₀ may be hydrogen. For example, - [(L₁₀)ₐ₁₀-R₁₀]_{b10} may represent hydrogen.

In an embodiment, a20 may be 0, and R₂₀ may be hydrogen. For example, - [(L₂₀)ₐ₂₀-R₂₀]_{b20} may represent hydrogen.

In an embodiment, a10 and a20 may each be 0, and R₁₀ and R₂₀ may each be hydrogen. For example, -[(L₁₀)ₐ₁₀-R₁₀]_{b10} and -[(L₂₀)ₐ₂₀-R₂₀]_{b20} may each represent hydrogen.

In Formulae 1 to 3, *, *', and *" each indicate a binding site to a neighboring atom.

At least one substituent of the substituted C₅-C₆₀ (*e.g*. C₅-C₃₀) carbocyclic group, the substituted C₁-C₆₀ (*e.g*. C₁-C₂₀) heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ (*e.g.* C₆-C₃₀) arylene group, the substituted C₁-C₆₀ (*e.g.* C₁-C₂₀) heteroarylene group, the substituted divalent non-aromatic condensed polycyclic group, the substituted divalent non-aromatic condensed heteropolycyclic group, the substituted C₁-C₆₀ (*e.g.* C₁-C₂₀) alkyl group, the substituted C₂-C₆₀ (*e.g.* C₂-C₂₀) alkenyl group, the substituted C₂-C₆₀ (*e.g.* C₂-C₂₀) alkynyl group, the substituted C₁-C₆₀ (*e.g.* C₁-C₂₀) alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ (*e.g*. C₆-C₃₀) aryl group, the substituted C₆-C₆₀ (*e.g.* C₆-C₃₀) aryloxy group, the substituted C₆-C₆₀ (*e.g.* C₆-C₃₀) arylthio group, the substituted C₁-C₆₀ (*e.g*. C₁-C₂₀) heteroaryl group, the substituted monovalent non-aromatic condensed polycyclic group, and the substituted monovalent non-aromatic condensed heteropolycyclic group is selected from:
deuterium, -F, -CI, -Br, -I, a hydroxyl group, a cyano group, a nitro group, an amidino group, a hydrazino group, a hydrazono group, a C₁-C₆₀ (*e.g*. C₁-C₂₀) alkyl group, a C₂-C₆₀ (*e.g.* C₂-C₂₀) alkenyl group, a C₂-C₆₀ (*e.g.* C₂-C₂₀) alkynyl group, and a C₁-C₆₀ (*e.g*. C₁-C₂₀) alkoxy group,
a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, and a C₁-C₆₀ (*e.g.* C₁-C₂₀) alkoxy group, each substituted with at least one selected from deuterium, -F, -CI, -Br, -I, a hydroxyl group, a cyano group, a nitro group, an amidino group, a hydrazine group, a hydrazone group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, a monovalent non-aromatic condensed heteropolycyclic group, -Si(Q₁₁)(Q₁₂)(Q₁₃), - N(Q₁₁)(Q₁₂), -B(Q₁₁)(Q₁₂), -P(Q₁₁)(Q₁₂), -C(=O)(Q₁₁), -S(=O)₂(Q₁₁), and - P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g*. C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g*. C₁-C₂₀) heteroaryl group, a monovalent non-aromatic condensed polycyclic group, a monovalent non-aromatic condensed heteropolycyclic group, a biphenyl group, and a terphenyl group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g*. C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g*. C₁-C₂₀) heteroaryl group, a monovalent non-aromatic condensed polycyclic group, a monovalent non-aromatic condensed heteropolycyclic group, a biphenyl group, and a terphenyl group, each substituted with at least one selected from deuterium, -F, -CI, -Br, -I, a hydroxyl group, a cyano group, a nitro group, an amidino group, a hydrazine group, a hydrazone group, a C₁-C₆₀ (*e.g*. C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g*. C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g*. C₁-C₂₀) heteroaryl group, a monovalent non-aromatic condensed polycyclic group, a monovalent non-aromatic condensed heteropolycyclic group, a biphenyl group, a terphenyl group, -Si(Q₂₁)(Q₂₂)(Q₂₃), -N(Q₂₁)(Q₂₂), -B(Q₂₁)(Q₂₂), -P(Q₂₁)(Q₂₂), - C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), - S(=O)₂(Q₃₁), and -P(=O)(Q₃₁)(Q₃₂), and
wherein Q₁ to Q₃, Q₁₁ to Q₁₃, Q₂₁ to Q₂₃, and Q₃₁ to Q₃₃ are each independently selected from hydrogen, deuterium, -F, -CI, -Br, -I, a hydroxyl group, a cyano group, a nitro group, an amidino group, a hydrazine group, a hydrazone group, a C₁-C₆₀ (*e.g*. C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g*. C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryl group substituted with a C1-C60 (*e.g.* C₁-C₂₀) alkyl group, a C₁-C₆₀ (*e.g.* C₁-C₂₀) heteroaryl group, a monovalent non-aromatic condensed polycyclic group, a monovalent non-aromatic condensed heteropolycyclic group, a biphenyl group, and a terphenyl group.

In an embodiment, the heterocyclic compound may be represented by one of Formulae 11-1 to 11-3 below:

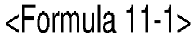

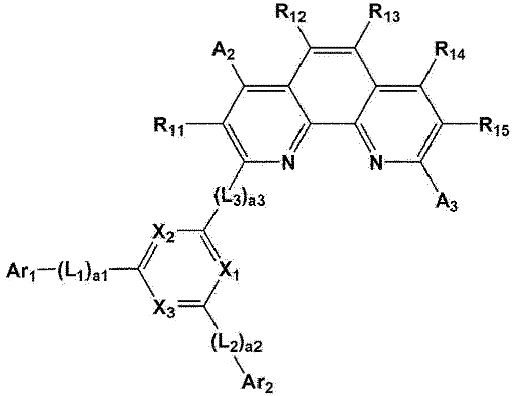

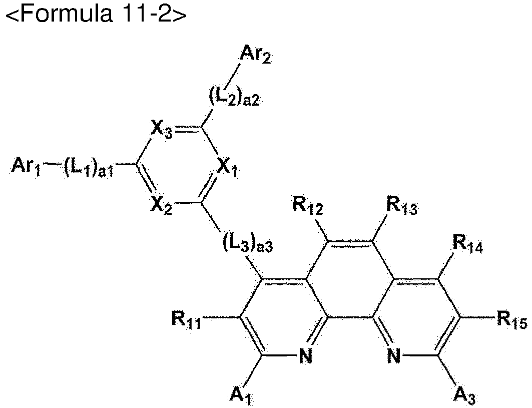

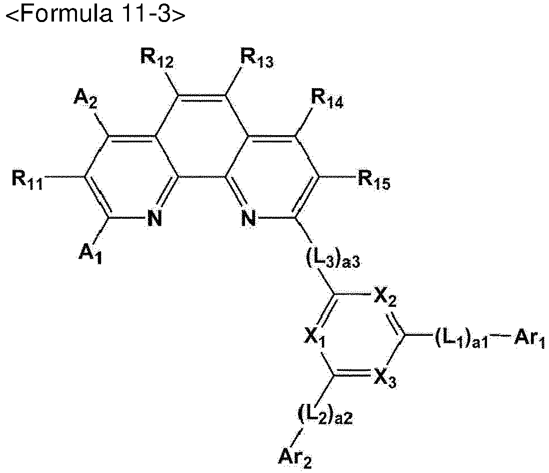

In Formulae 11-1 to 11-3,
A₁ to A₃ are the same as described in connection with Formula 1,
X₁ to X₃, L₁ to L₃, a1 to a3, Ar₁, and Ar₂ are the same as described in connection with Formula 2, and
R₁₁ to R₁₅ are the same as described in connection with R₁₀.

In an embodiment, the heterocyclic compound may be represented by one of Formulae 12-1 to 12-12 below:

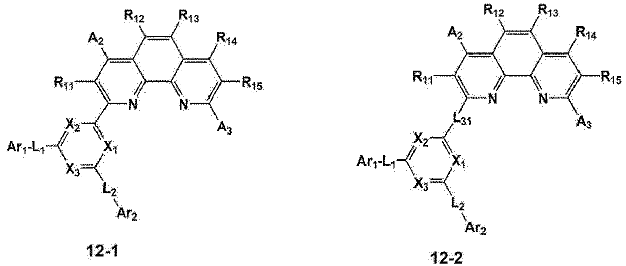

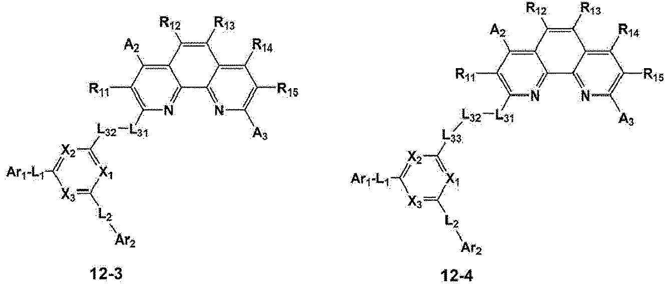

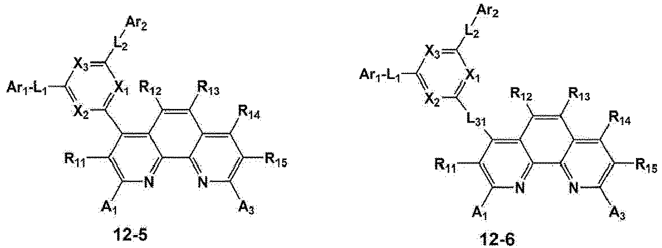

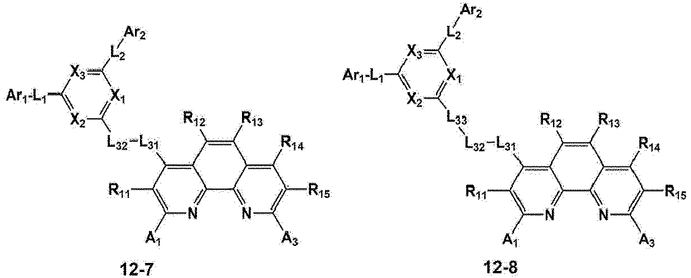

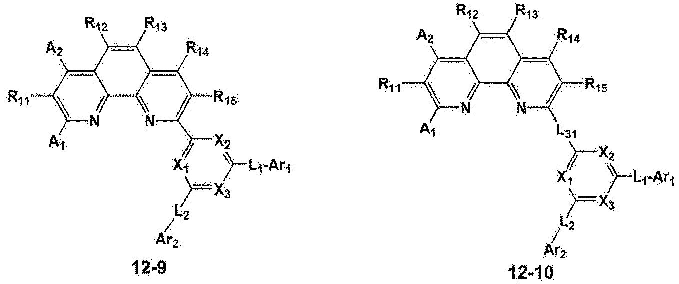

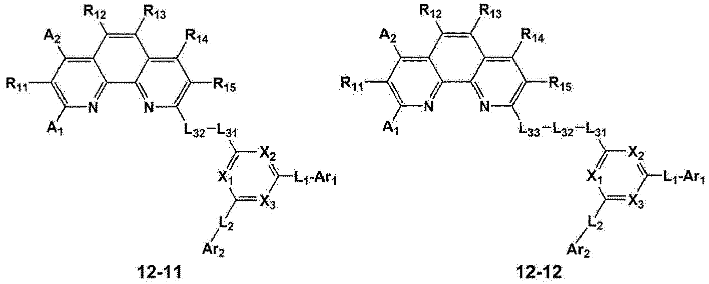

In Formulae 12-1 to 12-12,
A₁ to A₃ are the same as described in connection with Formula 1,
X₁ to X₃, L₁, L₂, Ar₁, and Ar₂ are the same as described in connection with Formula 2,
L₃₁ to L₃₃ are the same as described in connection with L₃, and
R₁₁ to R₁₅ are the same as described in connection with R₁₀.

In an embodiment, the heterocyclic compound may be selected from Compounds 1 to 42 below:

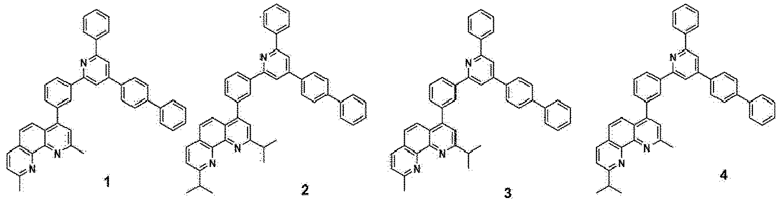

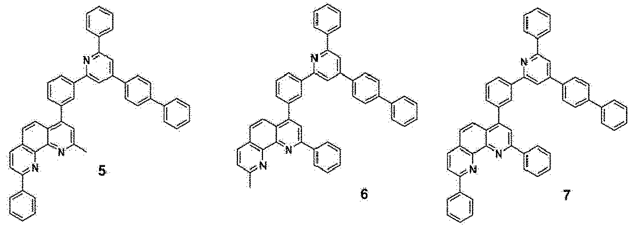

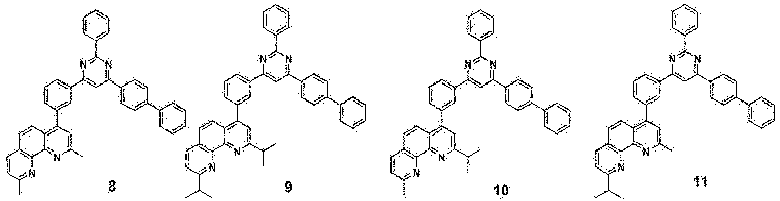

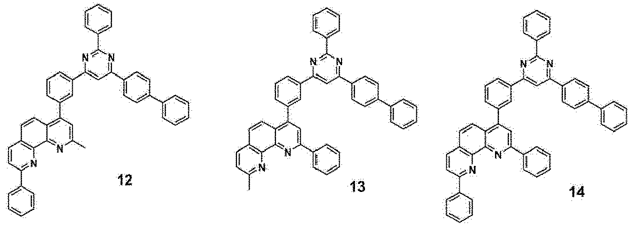

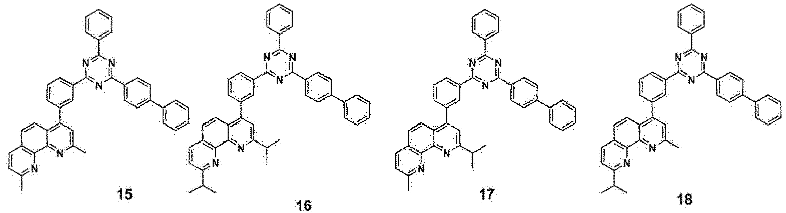

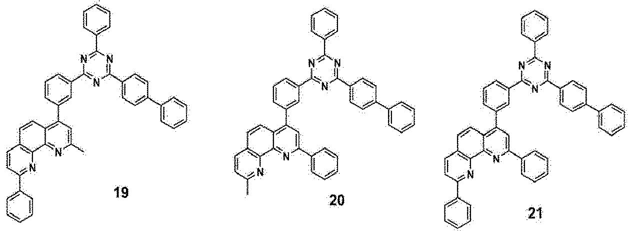

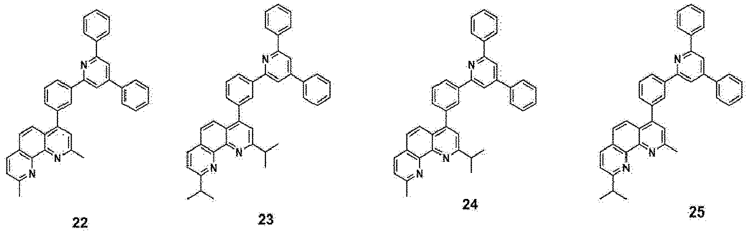

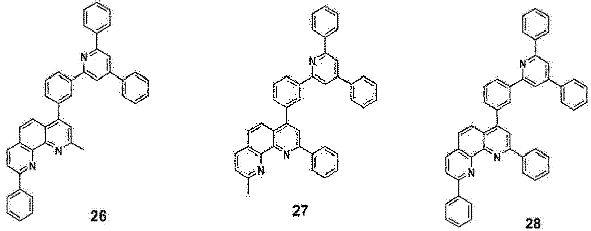

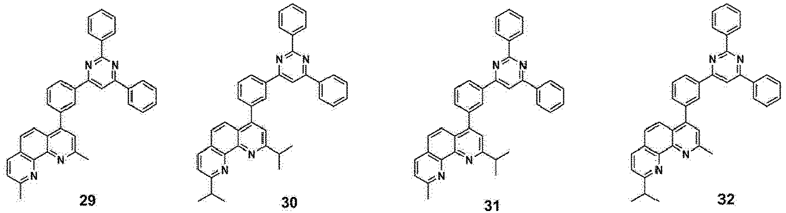

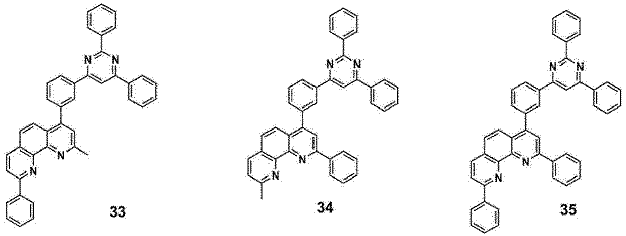

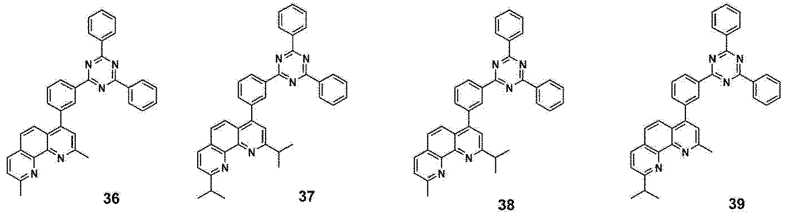

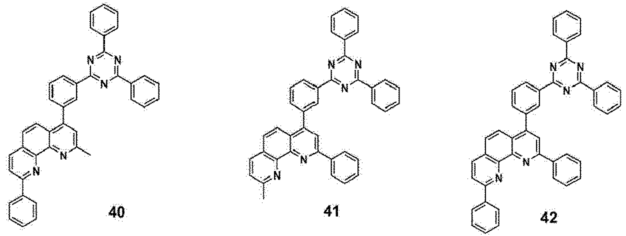

The heterocyclic compound represented by Formula 1 has a structure in which the 2 and 9 positions of the phenanthroline core are substituted. Accordingly, the structural stability of the compound itself is excellent since the 2 and 9 positions, where deterioration caused by negative polaron easily occurs in the organic light-emitting device, are substituted:

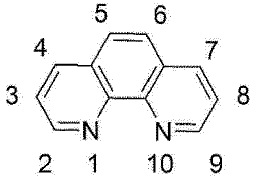

The heterocyclic compound represented by Formula 1 has a low (deep) LUMO energy level, so that an energy barrier at the interface with the p-type charge generation layer is lowered, thereby suppressing deterioration at the interface.

The heterocyclic compound represented by Formula 1 has excellent electron transport ability, and based on the structure described above, the heterocyclic compound may increase the mobility of electrons in the organic light-emitting device so that the reduction of the efficiency under low-current conditions may be prevented. Also, the charge balance in the organic light-emitting device may be properly adjusted under high-current conditions, thereby improving the lifespan and efficiency of the organic light-emitting device.

### [First electrode 110]

In FIG. 1, a substrate may be additionally disposed under the first electrode 110 or above the second electrode 190. The substrate may be a glass substrate or a plastic substrate, each having excellent mechanical strength, thermal stability, transparency, surface smoothness, ease of handling, and water resistance.

The first electrode 110 may be formed by depositing or sputtering a material for forming the first electrode 110 on the substrate. When the first electrode 110 is an anode, a material for forming the first electrode 110 may be selected from materials with a high work function to facilitate hole injection.

The first electrode 110 may be a reflective electrode, a semi-transmissive electrode, or a transmissive electrode. When the first electrode 110 is a transmissive electrode, a material for forming the first electrode 110 may be selected from indium tin oxide (ITO), indium zinc oxide (IZO), tin oxide (SnO₂), zinc oxide (ZnO), and any combinations thereof, but embodiments are not limited thereto. In embodiments, when the first electrode 110 is a semi-transmissive electrode or a reflective electrode, a material for forming the first electrode 110 may be selected from magnesium (Mg), silver (Ag), aluminium (Al), aluminium-lithium (Al-Li), calcium (Ca), magnesium-indium (Mg-In), magnesium-silver (Mg-Ag), and any combinations thereof, but embodiments are not limited thereto.

The first electrode 110 may have a single-layered structure or a multi-layered structure including two or more layers. For example, the first electrode 110 may have a three-layered structure of ITO/Ag/ITO, but the structure of the first electrode 110 is not limited thereto.

### [Organic layer 150]

The organic layer 150 is disposed on the first electrode 110. The organic layer 150 may include an emission layer.

The organic layer 150 may further include a hole transport region between the first electrode 110 and the emission layer and an electron transport region between the emission layer and the second electrode 190.

### [Hole transport region in organic layer 150]

The hole transport region may have i) a single-layered structure including a single layer including a single material, ii) a single-layered structure including a single layer including different materials, or iii) a multi-layered structure having multiple layers including different materials.

The hole transport region may include at least one selected from a hole injection layer, a hole transport layer, a buffer layer, an emission auxiliary layer, and an electron blocking layer.

For example, the hole transport region may have a single-layered structure including a single layer including different materials or a multi-layered structure having a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron blocking layer structure, wherein for each structure, constituting layers are sequentially stacked from the first electrode 110 in this stated order, but the structure of the hole transport region is not limited thereto.

In an embodiment, the hole transport region may include at least one selected from m-MTDATA, TDATA, 2-TNATA, NPB (NPD), β-NPB, TPD, spiro-TPD, spiro-NPB, methylated NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201 below, and a compound represented by Formula 202 below:

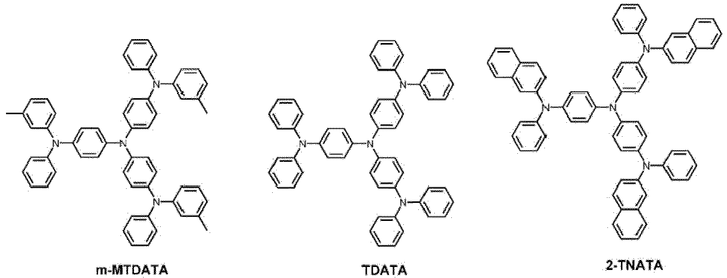

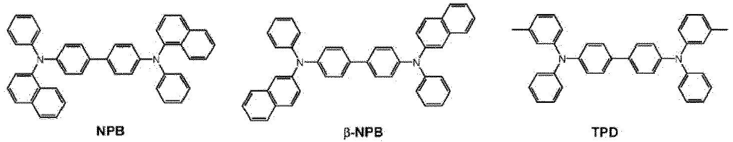

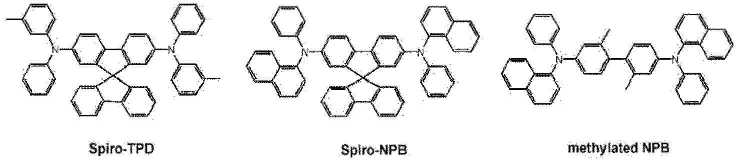

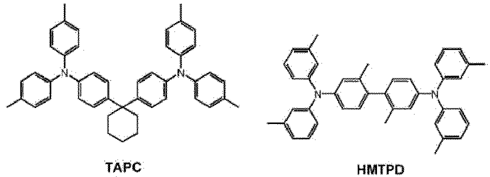

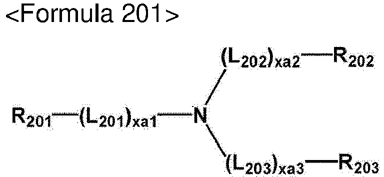

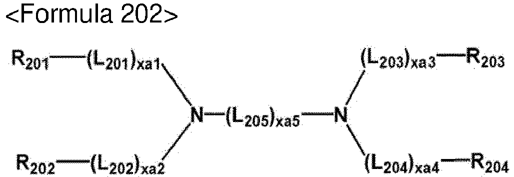

In Formulae 201 and 202,
L₂₀₁ to L₂₀₄ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (*e.g.* C₆-C₃₂) arylene group, a substituted or unsubstituted C₁-C₆₀ (*e.g*. C₁-C₂₀) heteroarylene group, a substituted or unsubstituted divalent non-aromatic condensed polycyclic group, and a substituted or unsubstituted divalent non-aromatic condensed heteropolycyclic group,
L₂₀₅ may be selected from *-O-*', *-S-*', *-N(Q₂₀₁)-*', a substituted or unsubstituted C₁-C₂₀ alkylene group, a substituted or unsubstituted C₂-C₂₀ alkenylene group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (*e.g*. C₆-C₃₂) arylene group, a substituted or unsubstituted C₁-C₆₀ (*e.g*. C₁-C₂₀) heteroarylene group, a substituted or unsubstituted divalent non-aromatic condensed polycyclic group, and a substituted or unsubstituted divalent non-aromatic condensed heteropolycyclic group,
xa1 to xa4 may each independently be an integer from 0 to 3,
xa5 may be an integer from 1 to 10, and
R₂₀₁ to R₂₀₄ and Q₂₀₁ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g.* C₆-C₃₂) aryl group, a substituted or unsubstituted C₆-C₆₀ (*e.g.* C₆-C₃₂) aryloxy group, a substituted or unsubstituted C₆-C₆₀ (*e.g*. C₆-C₃₂) arylthio group, a substituted or unsubstituted C₁-C₆₀ (*e.g*. C₁-C₂₀) heteroaryl group, a substituted or unsubstituted monovalent non-aromatic condensed polycyclic group, and a substituted or unsubstituted monovalent non-aromatic condensed heteropolycyclic group.

For example, in Formula 202, R₂₀₁ and R₂₀₂ may optionally be linked to each other via a single bond, a dimethyl-methylene group, or a diphenyl-methylene group, and R₂₀₃ and R₂₀₄ may optionally be linked to each other via a single bond, a dimethyl-methylene group, or a diphenyl-methylene group.

In an embodiment, in Formulae 201 and 202,
L₂₀₁ to L₂₀₅ may each independently be selected from:
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -CI, -Br, -I, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In embodiments, xa1 to xa4 may each independently be 0, 1, or 2.

In embodiments, xa5 may be 1, 2, 3, or 4.
In embodiments, R₂₀₁ to R₂₀₄ and Q₂₀₁ may each independently be selected from:
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -CI, -Br, -I, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂),
wherein Q₃₁ to Q₃₃ are the same as described above.

In embodiments, at least one selected from R₂₀₁ to R₂₀₃ in Formula 201 may each independently be selected from:
a fluorenyl group, a spiro-bifluorenyl group, a carbazolyl group, a dibenzofuranyl group, and a dibenzothiophenyl group; and
a fluorenyl group, a spiro-bifluorenyl group, a carbazolyl group, a dibenzofuranyl group, and a dibenzothiophenyl group, each substituted with at least one selected from deuterium, -F, -CI, -Br, -I, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group,
but embodiments are not limited thereto.

In embodiments, in Formula 202, i) R₂₀₁ and R₂₀₂ may be linked to each other via a single bond, and/or ii) R₂₀₃ and R₂₀₄ may be linked to each other via a single bond.

In embodiments, R₂₀₁ to R₂₀₄ in Formula 202 may be selected from:
a carbazolyl group; and
a carbazolyl group substituted with at least one selected from deuterium, -F, - Cl, -Br, -I, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group,
but embodiments are not limited thereto.

In embodiments, the compound represented by Formula 201 may be represented by Formula 201A below:

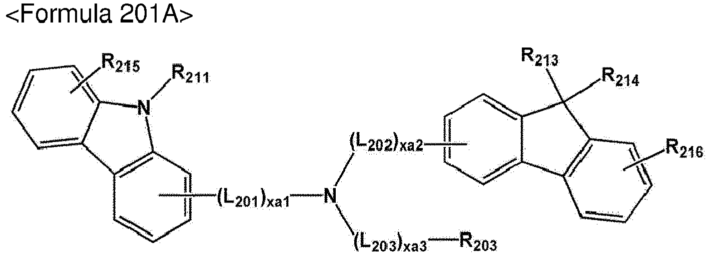

In embodiments, the compound represented by Formula 201 may be represented by Formula 201A(1) below, but embodiments are not limited thereto:

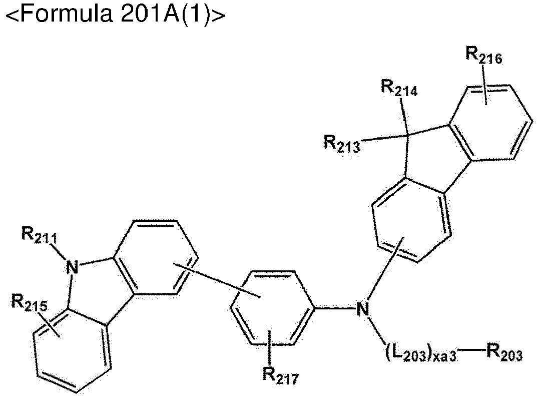

In embodiments, the compound represented by Formula 201 may be represented by Formula 201A-1 below, but embodiments are not limited thereto:

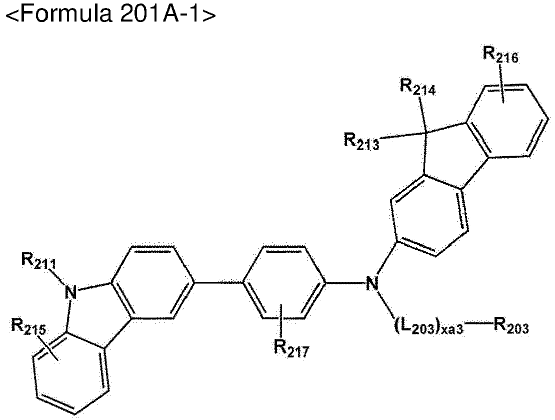

In embodiments, the compound represented by Formula 202 may be represented by Formula 202A below:

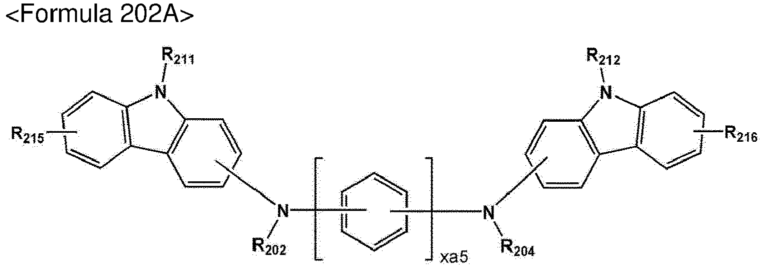

In embodiments, the compound represented by Formula 202 may be represented by Formula 202A-1 below:

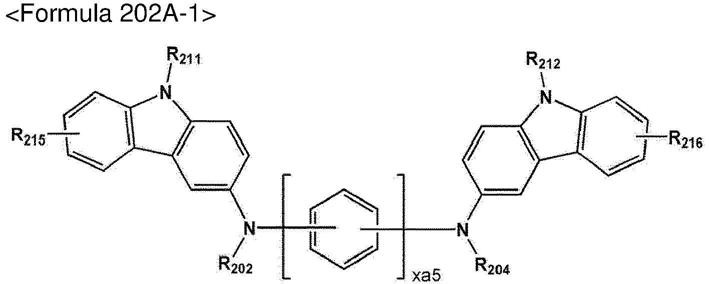

In Formulae 201A, 201A(1), 201A-1, 202A, and 202A-1,
L₂₀₁ to L₂₀₃, xa1 to xa3, xa5, and R₂₀₂ to R₂₀₄ are the same as described above,
R₂₁₁ and R₂₁₂ may be the same as described in connection with R₂₀₃, and
R₂₁₃ to R₂₁₇ may each independently be selected from hydrogen, deuterium, - F, -CI, -Br, -I, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

The hole transport region may include at least one compound selected from Compounds HT1 to HT39, but compounds to be included in the hole transport region are not limited thereto:

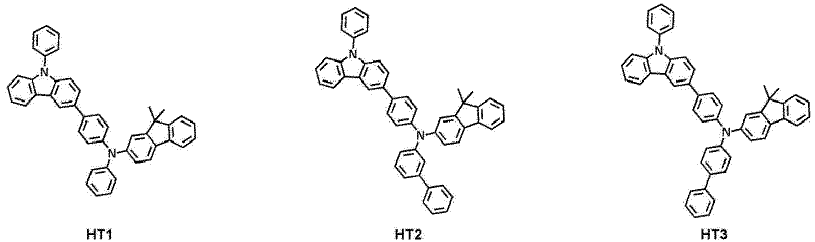

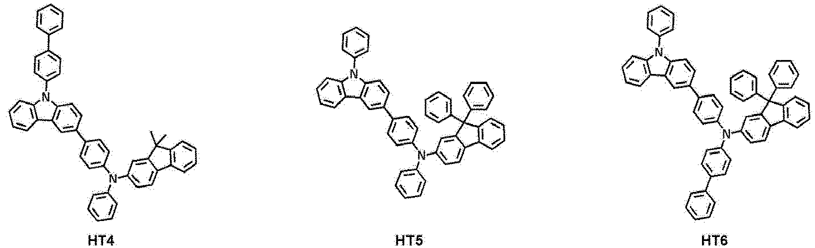

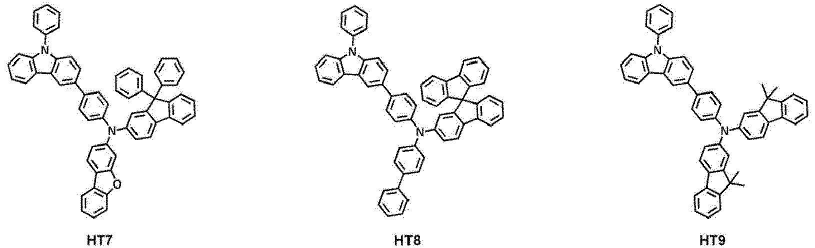

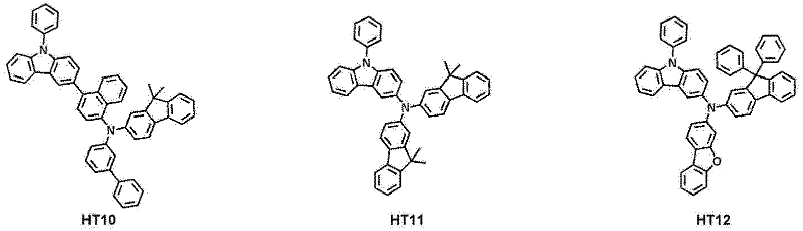

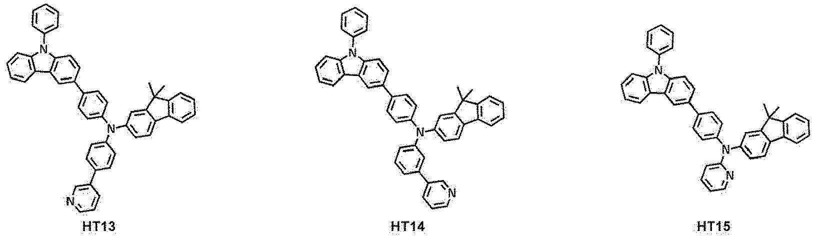

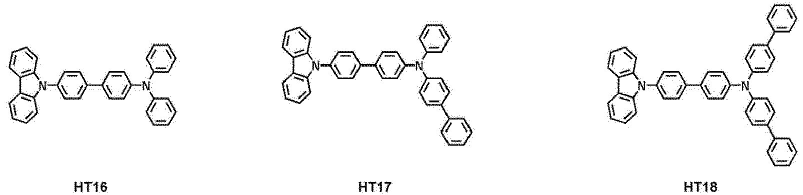

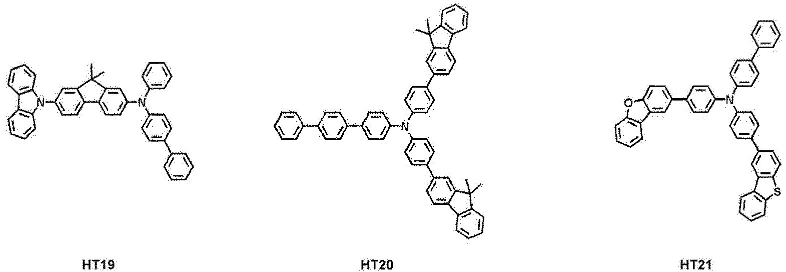

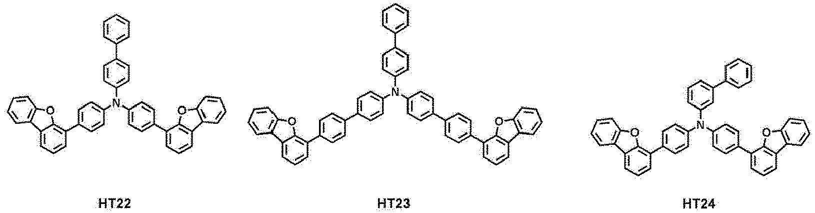

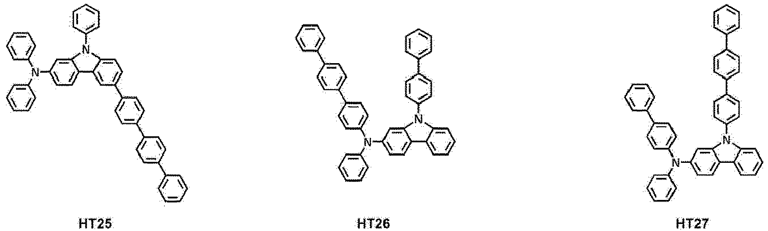

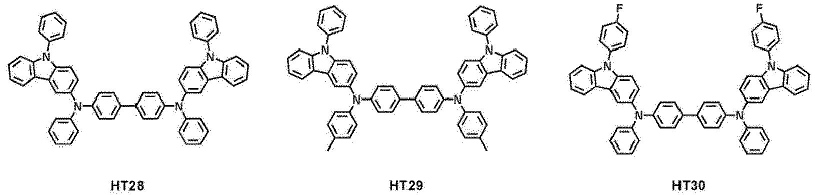

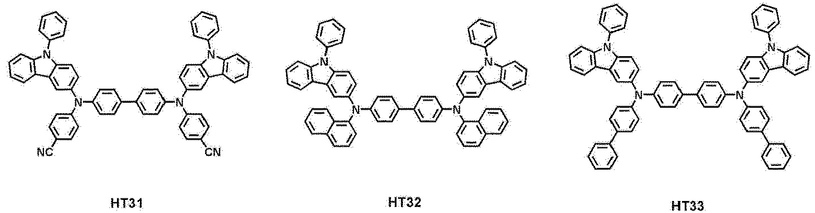

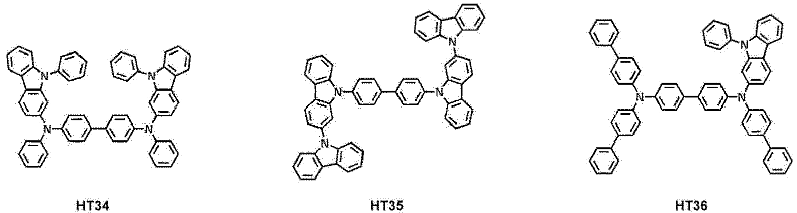

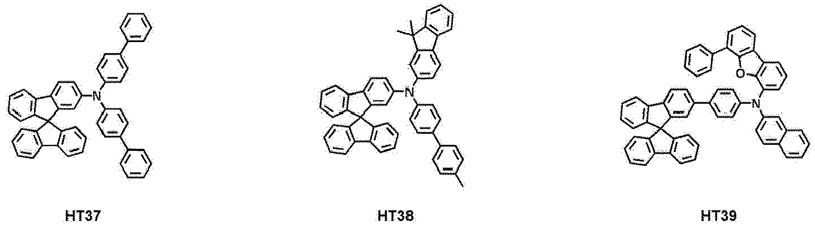

The thickness of the hole transport region may be in a range of about 100 Angstroms (Å) to about 10,000 Å. For example, thickness of the hole transport region may be in a range of about 100 Å to about 7,000 Å, about 100 Å to about 5,000 Å, or about 100 Å to about 1,000 Å. When the hole transport region includes at least one selected from a hole injection layer and a hole transport layer, the thickness of the hole injection layer may be in a range of about 50 Å to about 9,000 Å or about 100 Å to about 9,000 Å, and the thickness of the hole transport layer may be in a range of about 50 Å to about 2,000 Å. For example, the thickness of the hole injection layer may be in a range of about 50 Å to about 7,000 Å, about 50 Å to about 5,000 Å, about 50 Å to about 3,000 Å, about 50 Å to about 2,000 Å, or about 100 Å to about 1,000 Å. For example, the thickness of the hole transport layer may be in a range of about 100 Å to about 1,500 Å. When the thicknesses of the hole transport region, the hole injection layer, and the hole transport layer are within any of these ranges, excellent hole transport characteristics may be obtained without a substantial increase in driving voltage.

The emission auxiliary layer may increase light-emission efficiency by compensating for an optical resonance distance according to the wavelength of light emitted by an emission layer, and the electron blocking layer may block the flow of electrons from an electron transport region. The emission auxiliary layer and the electron blocking layer may include the aforementioned materials.

### [P-dopant]

The hole transport region may include a charge generation material as well as the aforementioned materials, to improve conductive properties of the hole transport region. The charge generation material may be substantially homogeneously or non-homogeneously dispersed in the hole transport region.

The charge generation material may include, for example, a p-dopant.

In an embodiment, a LUMO energy level of the p-dopant may be -3.5 eV or less.

The p-dopant may include at least one selected from a quinone derivative, a metal oxide, and a cyano group-containing compound, but embodiments are not limited thereto.

In an embodiment, the p-dopant may include at least one selected from:
a quinone derivative, such as tetracyanoquinodimethane (TCNQ) or 2,3,5,6-tetrafluoro-7,7,8,8-tetracyanoquinodimethane (F4-TCNQ);
a metal oxide, such as tungsten oxide or molybdenum oxide;
1,4,5,8,9,12-hexaazatriphenylene-hexacarbonitrile (HAT-CN); and
a compound represented by Formula 221 below,
but embodiments are not limited thereto:

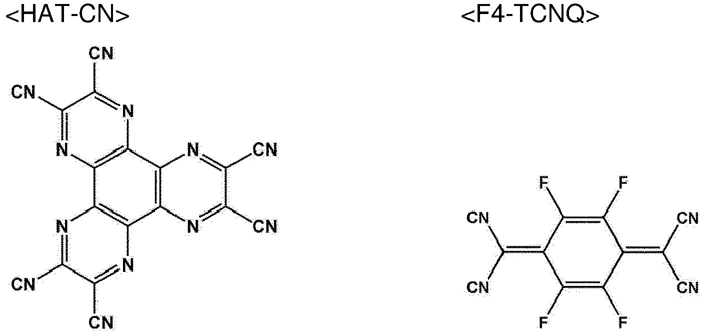

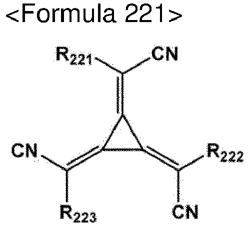

In Formula 221,
R₂₂₁ to R₂₂₃ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g.* C₆-C₃₂) aryl group, a substituted or unsubstituted C₁-C₆₀ (*e.g.* C₁-C₂₀) heteroaryl group, a substituted or unsubstituted monovalent non-aromatic condensed polycyclic group, and a substituted or unsubstituted monovalent non-aromatic condensed heteropolycyclic group, and at least one selected from R₂₂₁ to R₂₂₃ may have at least one substituent selected from a cyano group, -F, -CI, -Br, -I, a C₁-C₂₀ alkyl group substituted with -F, a C₁-C₂₀ alkyl group substituted with -CI, a C₁-C₂₀ alkyl group substituted with -Br, and a C₁-C₂₀ alkyl group substituted with -I.

### [Emission layer in emission unit]

When the organic light-emitting device 10 is a full-color organic light-emitting device, the emission layer may be patterned into a red emission layer, a green emission layer, or a blue emission layer, according to a sub-pixel. In embodiments, the emission layer may have a stacked structure of two or more layers selected from a red emission layer, a green emission layer, and a blue emission layer, in which the two or more layers contact each other or are separated from each other. In embodiments, the emission layer may include two or more materials selected from a red light-emitting material, a green light-emitting material, and a blue light-emitting material, in which the two or more materials are mixed with each other in a single layer to emit white light.

The emission layer may include a host and a dopant. The dopant may include at least one selected from a phosphorescent dopant and a fluorescent dopant.

The amount of the dopant in the emission layer may be, based on about 100 parts by weight of the host, in a range of about 0.01 parts by weight to about 15 parts by weight, but embodiments are not limited thereto.

The thickness of the emission layer may be in a range of about 100 Å to about 1,000 Å. In embodiments, the thickness of the emission layer may be in a range of about 100 Å to about 800 Å, about 150 Å to about 800 Å, about 150 Å to about 700 Å, about 150 Å to about 650 Å, about 200 Å to about 600 Å or about 200 Å to about 400 Å. When the thickness of the emission layer is within any of these ranges, improved luminescence characteristics may be obtained without a substantial increase in driving voltage.

### [Host in emission layer]

In an embodiment, the host may include a compound represented by Formula 301 below:

<Formula 301> [Ar₃₀₁]_{xb11}-[(L₃₀₁)_{xb1}-R₃₀₁]_{xb21}.

In Formula 301,
Ar₃₀₁ may be a substituted or unsubstituted C₅-C₆₀ carbocyclic group or a substituted or unsubstituted C₁-C₆₀ heterocyclic group,
xb11 may be 1, 2, or 3,
L₃₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xb1 may be an integer from 0 to 5,
R₃₀₁ may be selected from deuterium, -F, -CI, -Br, -I, a hydroxyl group, a cyano group, a nitro group, an amidino group, a hydrazine group, a hydrazone group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), and - P(=O)(Q₃₀₁)(Q₃₀₂), and
xb21 may be an integer from 1 to 5,
wherein Q₃₀₁ to Q₃₀₃ may each independently be selected from a C₁-C₁₀ alkyl group, a C₁-C₁₀ alkoxy group, a phenyl group, a biphenyl group, a terphenyl group, and a naphthyl group, but embodiments are not limited thereto.

For example, Ar₃₀₁ may be a substituted or unsubstituted C₆-C₃₂ carbocyclic group or a substituted or unsubstituted C₁-C₁₂ heterocyclic group, but embodiments are not limited thereto.

In an embodiment, Ar₃₀₁ in Formula 301 may be selected from:
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group; and
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group, each substituted with at least one selected from deuterium, - F, -CI, -Br, -I, a hydroxyl group, a cyano group, a nitro group, an amidino group, a hydrazine group, a hydrazone group, a C₁₋C₂₀ alkyl group, a C₁-C₂₀ alkoxy group, a phenyl group, a biphenyl group, a terphenyl group, a naphthyl group, -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂);
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group, but embodiments are not limited thereto.

When xb11 in Formula 301 is 2 or more, two or more Ar₃₀₁(s) may be linked to each other via a single bond.

In embodiments, the compound represented by Formula 301 may be represented by Formula 301-1 or 301-2 below:

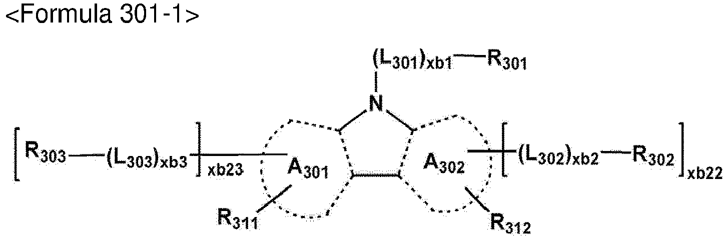

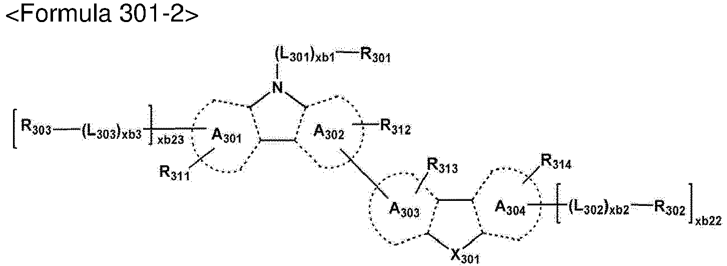

In Formulae 301-1 and 301-2,
A₃₀₁ to A₃₀₄ may each independently be selected from a benzene, a naphthalene, a phenanthrene, a fluoranthene, a triphenylene, a pyrene, a chrysene, a pyridine, a pyrimidine, an indene, a fluorene, a spiro-bifluorene, a benzofluorene, a dibenzofluorene, an indole, a carbazole, a benzocarbazole, dibenzocarbazole, a furan, a benzofuran, a dibenzofuran, a naphthofuran, a benzonaphthofuran, a dinaphthofuran, a thiophene, a benzothiophene, a dibenzothiophene, a naphthothiophene, a benzonaphthothiophene, and a dinaphthothiophene,
X₃₀₁ may be O, S, or N-[(L₃₀₄)_{xb4}-R₃₀₄],
R₃₁₁ to R₃₁₄ may each independently be selected from hydrogen, deuterium, - F, -CI, -Br, -I, a hydroxyl group, a cyano group, a nitro group, an amidino group, a hydrazine group, a hydrazone group, a C₁-C₂₀ alkyl group, a C₁-C₂₀ alkoxy group, a phenyl group, a biphenyl group, a terphenyl group, a naphthyl group -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
xb22 and xb23 may each independently be 0, 1, or 2,
L₃₀₁, xb1, R₃₀₁, and Q₃₁ to Q₃₃ are the same as described above,
L₃₀₂ to L₃₀₄ may each independently be the same as described in connection with L₃₀₁,
xb2 to xb4 may each independently be the same as described in connection with xb1, and
R₃₀₂ to R₃₀₄ may each independently be the same as described in connection with R₃₀₁.

For example, L₃₀₁ to L₃₀₄ in Formulae 301, 301-1, and 301-2 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -CI, -Br, -I, a hydroxyl group, a cyano group, a nitro group, an amidino group, a hydrazine group, a hydrazone group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
wherein Q₃₁ to Q₃₃ are the same as described above.

As another example, R₃₀₁ to R₃₀₄ in Formulae 301,301-1, and 301-2 may each independently be selected from: deuterium, -F, -CI, -Br, -I, a hydroxyl group, a cyano group, a nitro group, an amidino group, a hydrazino group, a hydrazono group, a substituted or unsubstituted C₁-C₆₀ (*e.g*. C₁-C₁₀) alkyl group, a substituted or unsubstituted C₂-C₆₀ (*e.g.* C₂-C₁₀) alkenyl group, a substituted or unsubstituted C₂-C₆₀ (*e.g.* C₂-C₁₀) alkynyl group, a substituted or unsubstituted C₁-C₆₀ (*e.g.* C₁-C₁₀) alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₃₀ aryl group, a substituted or unsubstituted C₆-C₃₀ aryloxy group, a substituted or unsubstituted C₆-C₃₀ arylthio group, a substituted or unsubstituted C₁-C₂₀ heteroaryl group, a substituted or unsubstituted monovalent non-aromatic condensed polycyclic group, a substituted or unsubstituted monovalent non-aromatic condensed heteropolycyclic group, -Si(Q₃₀₁)(Q₃₀₂)(Q₃₀₃), -N(Q₃₀₁)(Q₃₀₂), - B(Q₃₀₁)(Q₃₀₂), -C(=O)(O₃₀₁), -S(=O)₂(Q₃₀₁), and -P(=O)(Q₃₀₁)(Q₃₀₂), but embodiments are not limited thereto.

In an embodiment, R₃₀₁ to R₃₀₄ in Formulae 301, 301-1, and 301-2 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -CI, -Br, -I, a hydroxyl group, a cyano group, a nitro group, an amidino group, a hydrazine group, a hydrazone group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
wherein Q₃₁ to Q₃₃ are the same as described above.

In embodiments, the host may include an alkaline earth metal complex. For example, the host may include a complex selected from a Be complex (for example, Compound H55), a Mg complex, and a Zn complex. For example, the host may be selected from a Be complex (for example, Compound H55), an Mg complex, and a Zn complex.

The host may include at least one selected from 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), and Compounds H1 to H56, but embodiments are not limited thereto:

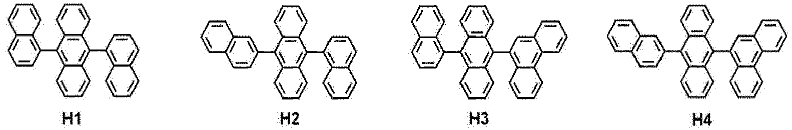

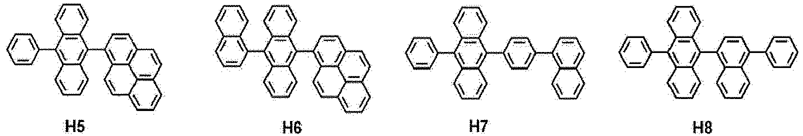

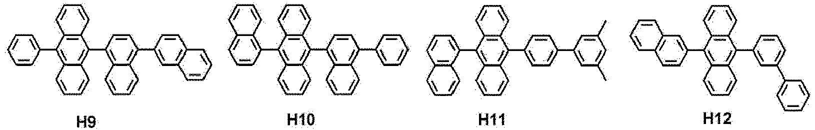

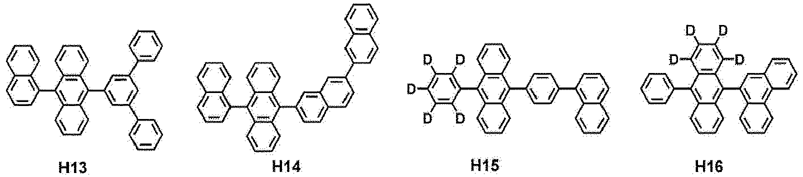

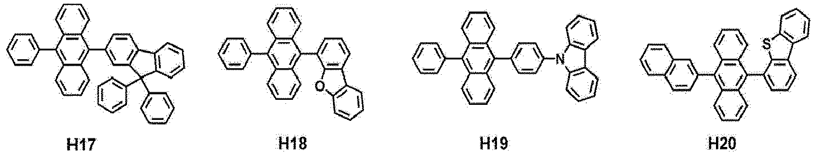

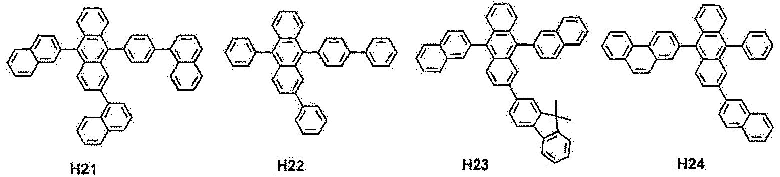

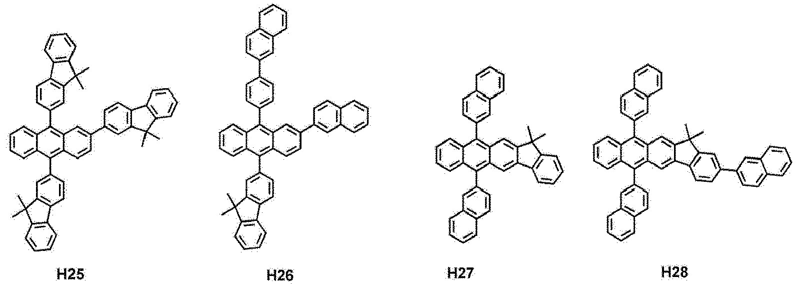

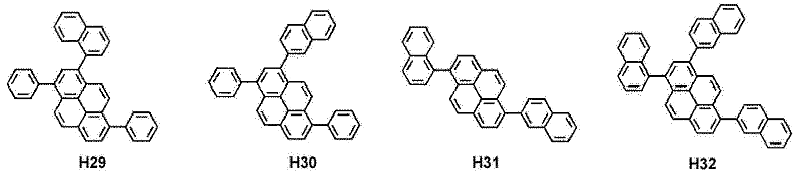

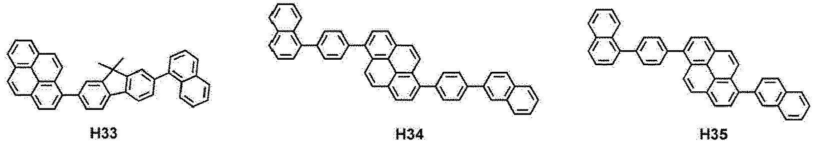

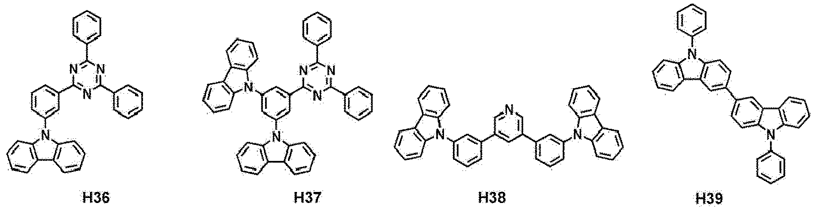

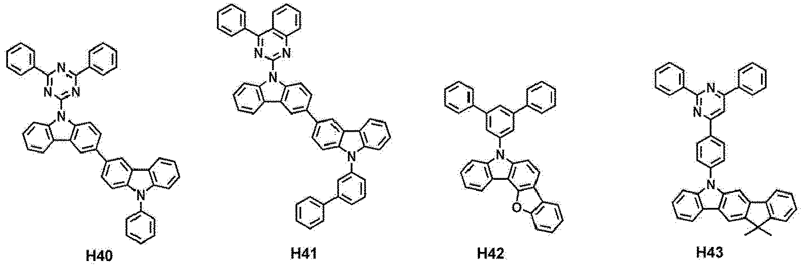

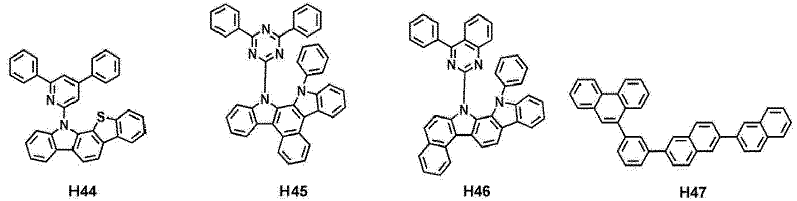

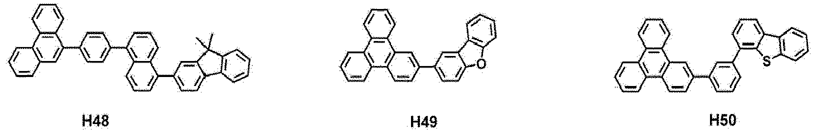

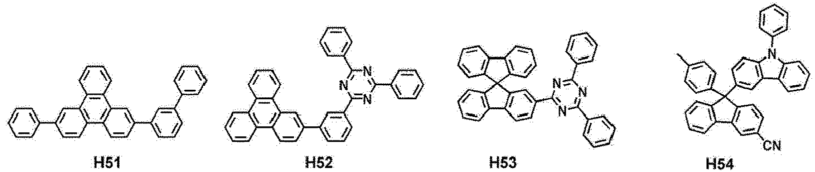

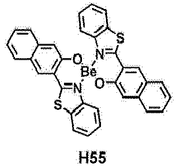

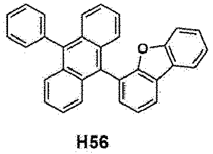

### [Phosphorescent dopant included in emission layer]

The phosphorescent dopant may include an organometallic complex represented by Formula 401 below:

<Formula 401 > M(L₄₀₁)_{xc1}(L₄₀₂)_{xc2˙}

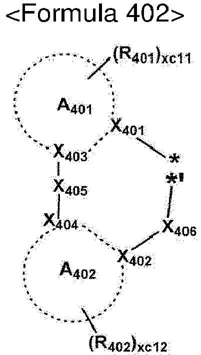

In Formulae 401 and 402,
M may be selected from iridium (Ir), platinum (Pt), palladium (Pd), osmium (Os), titanium (Ti), zirconium (Zr), hafnium (Hf), europium (Eu), terbium (Tb), rhodium (Rh), and thulium (Tm),
L₄₀₁ may be a ligand represented by Formula 402, and xc1 may be 1, 2, or 3, wherein, when xc1 is 2 or more, two or more L₄₀₁(s) may be identical to or different from each other,
L₄₀₂ may be an organic ligand, and xc2 may be an integer from 0 to 4, wherein, when xc2 may be two or more, two or more L₄₀₂(s) may be identical to or different from each other,
X₄₀₁ to X₄₀₄ may each independently be nitrogen or carbon,
X₄₀₁ and X₄₀₃ may be linked to each other via a single bond or a double bond, and X₄₀₂ and X₄₀₄ may be linked to each other via a single bond or a double bond,
A₄₀₁ and A₄₀₂ may each independently be a C₅-C₆₀ (*e.g*. C₅-C₃₀) carbocyclic group or a C₁-C₆₀ (*e.g*. C₁-C₂₀) heterocyclic group,
X₄₀₅ may be a single bond, *-O-*', *-S-*', *-C(=O)-*', *-N(Q₄₁₁)-*', *-C(Q₄₁₁)(Q₄₁₂)-*', *-C(Q₄₁₁)-C(Q₄₁₂)-*', *-C(Q₄₁₁)=*', or *=C=*', wherein Q₄₁₁ and Q₄₁₂ may be hydrogen, deuterium, a C₁-C₂₀ alkyl group, a C₁-C₂₀ alkoxy group, a phenyl group, a biphenyl group, a terphenyl group, or a naphthyl group,
X₄₀₆ may be a single bond, O, or S,
R₄₀₁ and R₄₀₂ may each independently be selected from hydrogen, deuterium, -F, -CI, -Br, -I, a hydroxyl group, a cyano group, a nitro group, an amidino group, a hydrazine group, a hydrazone group, a substituted or unsubstituted C₁-C₂₀ alkyl group, a substituted or unsubstituted C₁-C₂₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g*. C₆-C₃₀) aryl group, a substituted or unsubstituted C₆-C₆₀ (*e.g*. C₆-C₃₀) aryloxy group, a substituted or unsubstituted C₆-C₆₀ (*e.g*. C₆-C₃₀) arylthio group, a substituted or unsubstituted C₁-C₆₀ (*e.g*. C₁-C₂₀) heteroaryl group, a substituted or unsubstituted monovalent non-aromatic condensed polycyclic group, and a substituted or unsubstituted monovalent non-aromatic condensed heteropolycyclic group, - Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁), and - P(=O)(Q₄₀₁)(Q₄₀₂), wherein Q₄₀₁ to Q₄₀₃ may each independently be selected from a C₁-C₁₀ alkyl group, a C₁-C₁₀ alkoxy group, a C₆-C₂₀ aryl group, and a C₁-C₂₀ heteroaryl group,
xc11 and xc12 may each independently be an integer from 0 to 10, and
* and *' in Formula 402 each indicate a binding site to M in Formula 401.

In an embodiment, A₄₀₁ and A₄₀₂ in Formula 402 may each independently be selected from a benzene group, a naphthalene group, a fluorene group, a spiro-bifluorene group, an indene group, a pyrrole group, a thiophene group, a furan group, an imidazole group, a pyrazole group, a thiazole group, an isothiazole group, an oxazole group, an isoxazole group, a pyridine group, a pyrazine group, a pyrimidine group, a pyridazine group, a quinoline group, an isoquinoline group, a benzoquinoline group, a quinoxaline group, a quinazoline group, a carbazole group, a benzimidazole group, a benzofuran group, a benzothiophene group, an isobenzothiophene group, a benzoxazole group, an isobenzoxazole group, a triazole group, a tetrazole group, an oxadiazole group, a triazine group, a dibenzofuran group, and a dibenzothiophene group.

In embodiments, in Formula 402, i) X₄₀₁ may be nitrogen and X₄₀₂ may be carbon, or ii) X₄₀₁ and X₄₀₂ may each be nitrogen at the same time.

In embodiments, R₄₀₁ and R₄₀₂ in Formula 402 may each independently be selected from:
hydrogen, deuterium, -F, -CI, -Br, -I, a hydroxyl group, a cyano group, a nitro group, an amidino group, a hydrazino group, a hydrazono group, a C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -CI, -Br, -I, a hydroxyl group, a cyano group, a nitro group, an amidino group, a hydrazino group, a hydrazono group, a phenyl group, a naphthyl group, a cyclopentyl group, a cyclohexyl group, an adamantanyl group, a norbornanyl group, and a norbornenyl group;
a cyclopentyl group, a cyclohexyl group, an adamantanyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group;
a cyclopentyl group, a cyclohexyl group, an adamantanyl group, a norbornanyl group, a norbornenyl group a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group, each substituted with at least one selected from deuterium, -F, -CI, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, an adamantanyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group; and
-Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), and -P(=O)(Q₄₀₁)(Q₄₀₂),
wherein Q₄₀₁ to Q₄₀₃ may each independently be selected from a C₁-C₁₀ alkyl group, a C₁-C₁₀ alkoxy group, a phenyl group, a biphenyl group, and a naphthyl group, but embodiments are not limited thereto.

In embodiments, when xc1 in Formula 401 is 2 or more, two A₄₀₁(s) in two or more L₄₀₁(s) may optionally be linked to each other via X₄₀₇, which is a linking group, two A402(s) may optionally be linked to each other via X₄₀₈, which is a linking group (see Compounds PD1 to PD4 and PD7 below). X₄₀₇ and X₄₀₈ may each independently be a single bond, *-O-*', *-S-*', *-C(=O)-*', *-N(Q₄₁₃)-*', *-C(Q₄₁₃)(Q₄₁₄)-*', or *-C(Q₄₁₃)=C(Q₄₁₄)-*' (wherein Q₄₁₃ and Q₄₁₄ may each independently be hydrogen, deuterium, a C₁-C₂₀ alkyl group, a C₁-C₂₀ alkoxy group, a phenyl group, a biphenyl group, a terphenyl group, or a naphthyl group), but embodiments are not limited thereto.

L₄₀₂ in Formula 401 may be a monovalent, divalent, or trivalent organic ligand. For example, L₄₀₂ may be selected from halogen, diketone (for example, acetylacetonate), carboxylic acid (for example, picolinate), -C(=O), isonitrile, -CN, and phosphorus (for example, phosphine or phosphite), but embodiments are not limited thereto.

In embodiments, the phosphorescent dopant may be selected from, for example, Compounds PD1 to PD25 below, but embodiments are not limited thereto:

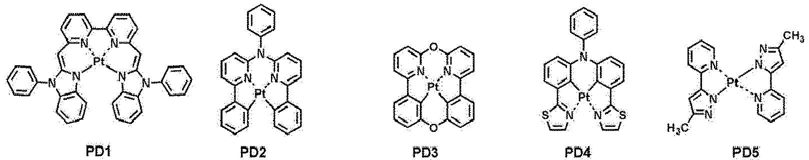

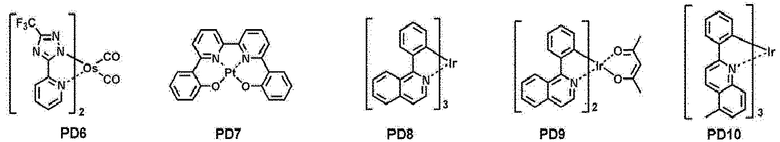

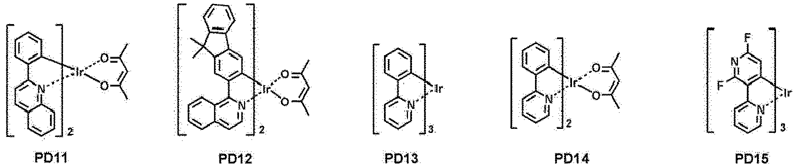

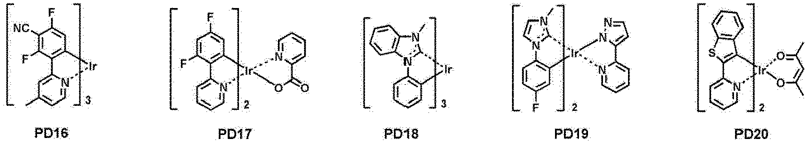

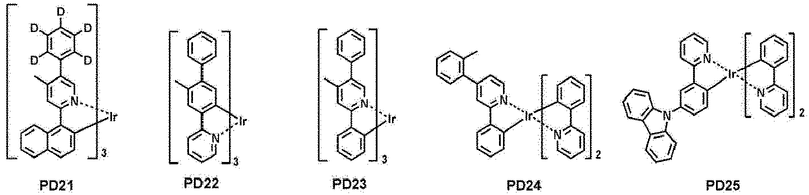

### [Fluorescent dopant included in emission layer]

The fluorescent dopant may include an arylamine compound or a styrylamine compound.

The fluorescent dopant may include a compound represented by Formula 501 below:

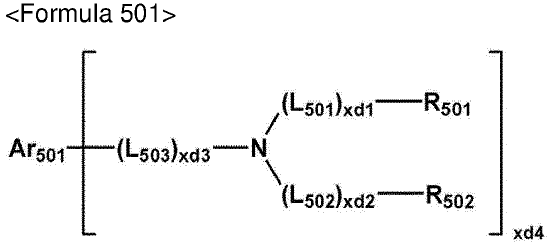

In Formula 501,
Ar₅₀₁ may be a substituted or unsubstituted C₅-C₆₀ carbocyclic group or a substituted or unsubstituted C₁-C₆₀ heterocyclic group,
L₅₀₁ to L₅₀₃ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (*e.g*. C₆-C₃₀) arylene group, a substituted or unsubstituted C₁-C₆₀ (*e.g*. C₁-C₂₀) heteroarylene group, a substituted or unsubstituted divalent non-aromatic condensed polycyclic group, and a substituted or unsubstituted divalent non-aromatic condensed heteropolycyclic group,
xd1 to xd3 may each independently be an integer from 0 to 3,
R₅₀₁ and R₅₀₂ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g.* C₆-C₃₀) aryl group, a substituted or unsubstituted C₆-C₆₀ (*e.g.* C₆-C₃₀) aryloxy group, a substituted or unsubstituted C₆-C₆₀ (*e.g*. C₆-C₃₀) arylthio group, a substituted or unsubstituted C₁-C₆₀ (*e.g*. C₁-C₂₀) heteroaryl group, a substituted or unsubstituted monovalent non-aromatic condensed polycyclic group, and a substituted or unsubstituted monovalent non-aromatic condensed heteropolycyclic group, and
xd4 may be an integer from 1 to 6.

For example, Ar₅₀₁ may be a substituted or unsubstituted C₅-C₃₀ carbocyclic group or a substituted or unsubstituted C₁-C₂₀ heterocyclic group, but embodiments are not limited thereto.

In an embodiment, Ar₅₀₁ in Formula 501 may be selected from:
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group; and
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group, each substituted with at least one selected from deuterium, -F, -CI, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

In embodiments, L₅₀₁ to L₅₀₃ in Formula 501 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -CI, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

In embodiments, R₅₀₁ and R₅₀₂ in Formula 501 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -CI, - Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, and - Si(Q₃₁)(Q₃₂)(Q₃₃),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In embodiments, xd4 in Formula 501 may be 2, but embodiments are not limited thereto.

For example, the fluorescent dopant may be selected from Compounds FD1 to FD23 below:

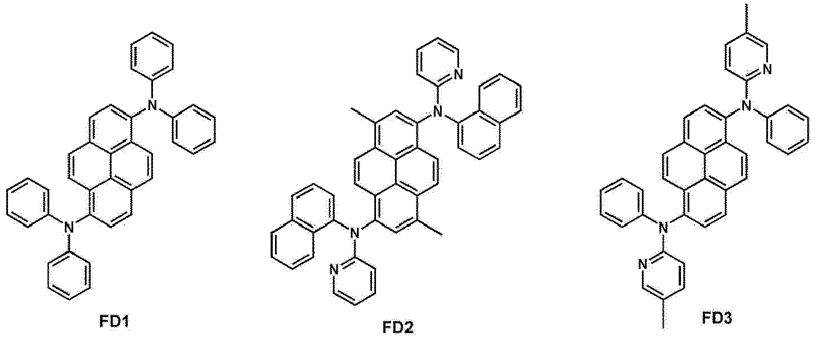

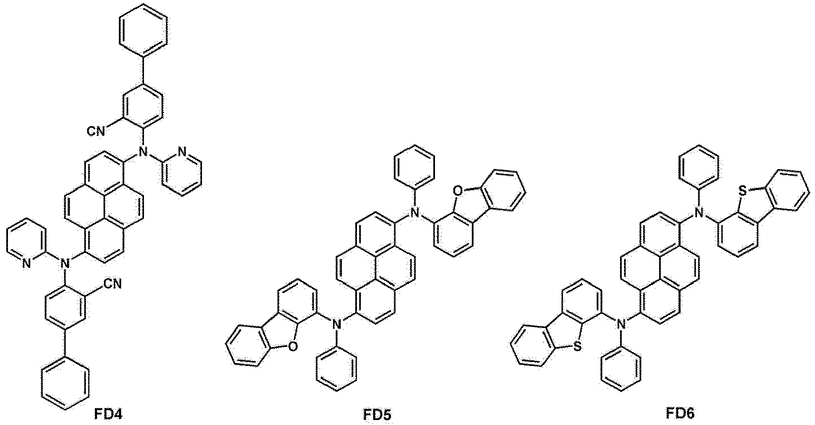

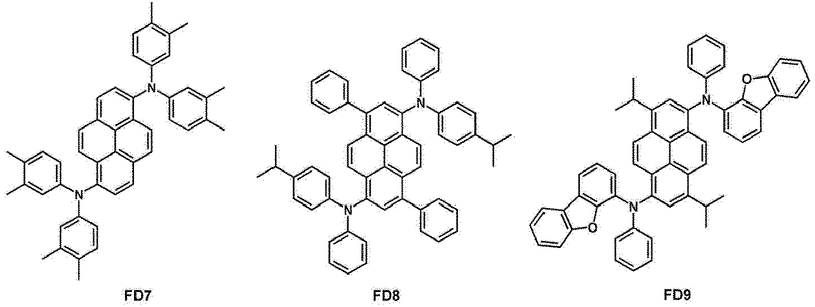

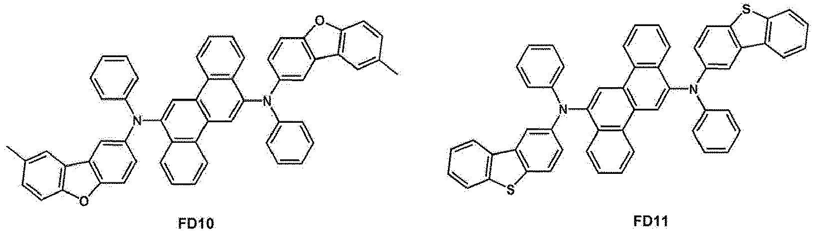

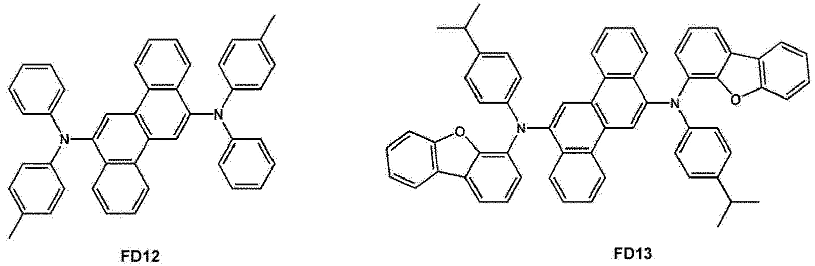

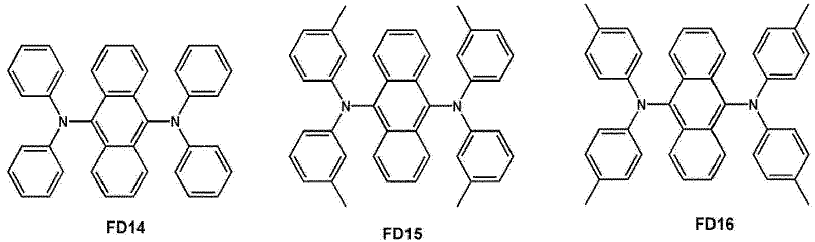

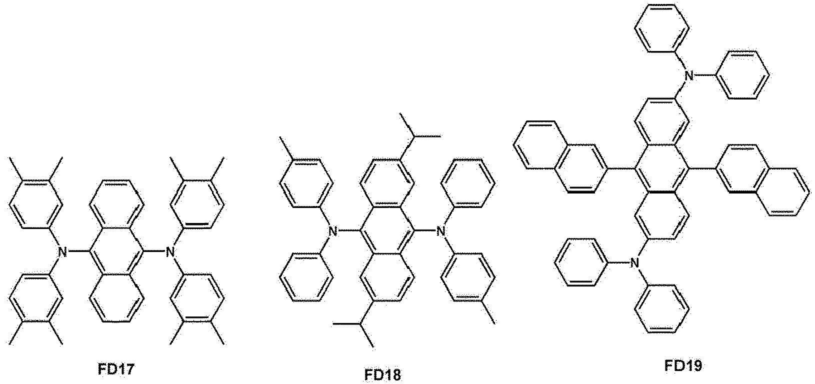

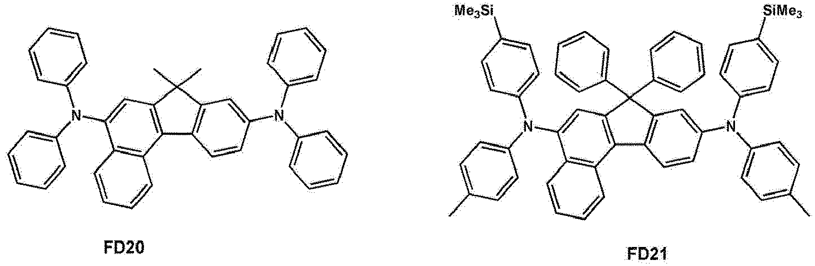

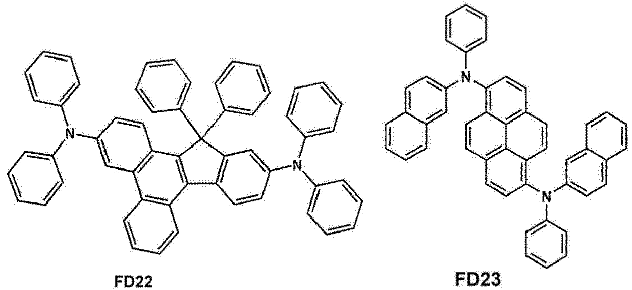

In embodiments, the fluorescent dopant may be selected from the following compounds, but embodiments are not limited thereto:

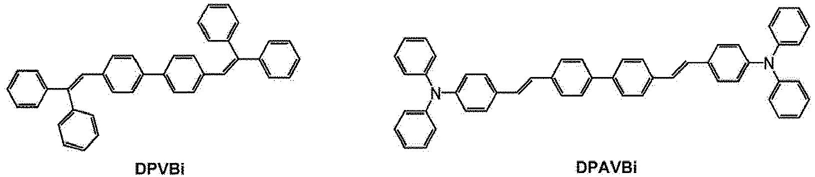

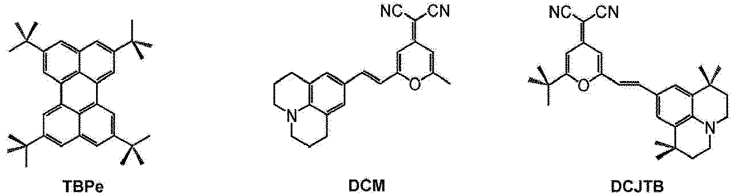

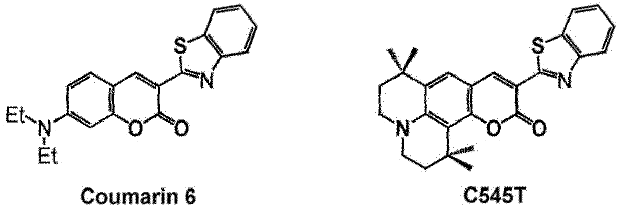

### [Electron transport region in emission unit]

The electron transport region may have i) a single-layered structure including a single layer including a single material, ii) a single-layered structure including a single layer including different materials, or iii) a multi-layered structure having multiple layers including different materials.

The electron transport region may include at least one selected from a buffer layer, a hole blocking layer, an electron control layer, an electron transport layer, and an electron injection layer, but embodiments are not limited thereto.

In embodiments, the electron transport region may include at least one selected from a hole blocking layer, an electron transport layer, and an electron injection layer.

For example, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein for each structure, constituting layers are sequentially stacked from an emission layer. However, embodiments of the structure of the electron transport region are not limited thereto.

The electron transport region (for example, a buffer layer, a hole blocking layer, an electron control layer, or an electron transport layer in the electron transport region) may include an electron transport compound.

The electron transport compound may be a metal-free compound including at least one π electron-deficient nitrogen-containing ring.

The "π electron-depleted nitrogen-containing ring" indicates a C₁-C₆₀ (*e.g*. a C₁-C₃₀) heterocyclic group having at least one *-N=*' moiety as a ring-forming moiety.

For example, the "π electron-depleted nitrogen-containing ring" may be i) a 5-membered to 7-membered heteromonocyclic group having at least one *-N=*' moiety, ii) a heteropolycyclic group in which two or more 5-membered to 7-membered heteromonocyclic groups each having at least one *-N=*' moiety are condensed with each other, or iii) a heteropolycyclic group in which at least one of 5-membered to 7-membered heteromonocyclic groups, each having at least one *-N=*' moiety, is condensed with at least one C₅-C₆₀ (*e.g*. a C₅-C₃₀) carbocyclic group.

Examples of the π electron-deficient nitrogen-containing ring include an imidazole ring, a pyrazole ring, a thiazole ring, an isothiazole ring, an oxazole ring, an isoxazole ring, a pyridine ring, a pyrazine ring, a pyrimidine ring, a pyridazine ring, an indazole ring, a purine ring, a quinoline ring, an isoquinoline ring, a benzoquinoline ring, a phthalazine ring, a naphthyridine ring, a quinoxaline ring, a quinazoline ring, a cinnoline ring, a phenanthridine ring, an acridine ring, a phenanthroline ring, a phenazine ring, a benzimidazole ring, an isobenzothiazole ring, a benzoxazole ring, an isobenzoxazole ring, a triazole ring, a tetrazole ring, an oxadiazole ring, a triazine ring, a thiadiazole ring, an imidazopyridine ring, an imidazopyrimidine ring, and an azacarbazole ring, but are not limited thereto.

For example, the electron transport region may include an electron transport compound.

In an embodiment, the electron transport compound may be a compound represented by Formula 601 below:

<Formula 601> [Ar₆₀₁]ₓₑ₁₁-[(L₆₀₁)ₓₑ₁-R₆₀₁]ₓₑ₂₁.

In Formula 601,
Ar₆₀₁ may be a substituted or unsubstituted C₅-C₆₀ carbocyclic group or a substituted or unsubstituted C₁-C₆₀ heterocyclic group,
xe11 may be 1, 2, or 3,
L₆₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xe1 may be an integer from 0 to 5,
R₆₀₁ may be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₆₀₁)(Q₆₀₂)(Q₆₀₃), -C(=O)(Q₆₀₁), -S(=O)₂(Q₆₀₁), and -P(=O)(Q₆₀₁)(Q₆₀₂),
Q₆₀₁ to Q₆₀₃ may each independently be a C₁-C₁₀ alkyl group, a C₁-C₁₀ alkoxy group, a phenyl group, a biphenyl group, a terphenyl group, or a naphthyl group, and
xe21 may be an integer from 1 to 5.

In an embodiment, at least one of Ar₆₀₁(s) in the number of xe11 and R₆₀₁(s) in the number of xe21 may include the π electron-deficient nitrogen-containing ring.

For example, Ar₆₀₁ may be a substituted or unsubstituted C₅-C₃₀ carbocyclic group or a substituted or unsubstituted C₁-C₂₀ heterocyclic group, but embodiments are not limited thereto.

In an embodiment, ring Ar₆₀₁ in Formula 601 may be selected from:
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group; and
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group, each substituted with at least one selected from deuterium, -F, -CI, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -S(=O)₂(Q₃₁), and -P(=O)(Q₃₁)(Q₃₂),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

When xe11 in Formula 601 is 2 or more, two or more Ar₆₀₁(s) may be linked to each other via a single bond.

In embodiments, Ar₆₀₁ in Formula 601 may be an anthracene group.

In embodiments, the compound represented by Formula 601 may be represented by Formula 601-1 below:

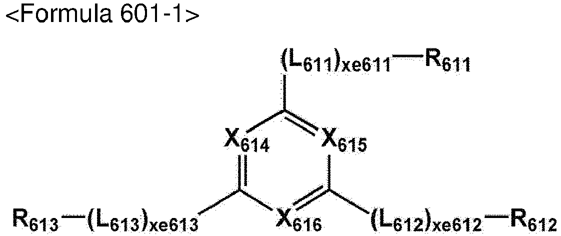

In Formula 601-1,
X₆₁₄ may be N or C(R₆₁₄), X₆₁₅ may be N or C(R₆₁₅), X₆₁₆ may be N or C(R₆₁₆), and at least one of X₆₁₄ to X₆₁₆ may be N,
L₆₁₁ to L₆₁₃ may each independently be the same as described in connection with L₆₀₁,
xe611 to xe613 may each independently be the same as described in connection with xe1,
R₆₁₁ to R₆₁₃ may each independently be the same as described in connection with R₆₀₁, and
R₆₁₄ to R₆₁₆ may each independently be selected from hydrogen, deuterium, - F, -CI, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

For example, L₆₀₁ and L₆₁₁ to L₆₁₃ in Formulae 601 and 601-1 may each independently be selected from: a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₃₀ arylene group, a substituted or unsubstituted C₁-C₂₀ heteroarylene group, a substituted or unsubstituted divalent non-aromatic condensed polycyclic group, and a substituted or unsubstituted divalent non-aromatic condensed heteropolycyclic group, but embodiments are not limited thereto.

In an embodiment, L₆₀₁ and L₆₁₁ to L₆₁₃ in Formulae 601 and 601-1 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -CI, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group,
but embodiments are not limited thereto.

In embodiments, xe1 and xe611 to xe613 in Formulae 601 and 601-1 may each independently be 0, 1, or 2.

For example, R₆₀₁ and R₆₁₁ to R₆₁₃ in Formulae 601 and 601-1 may each independently be selected from: a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₃₀ aryl group, a substituted or unsubstituted C₆-C₃₀ aryloxy group, a substituted or unsubstituted C₆-C₃₀ arylthio group, a substituted or unsubstituted C₁-C₂₀ heteroaryl group, a substituted or unsubstituted monovalent non-aromatic condensed polycyclic group, a substituted or unsubstituted monovalent non-aromatic condensed heteropolycyclic group, - Si(Q₆₀₁)(Q₆₀₂)(Q₆₀₃), -C(=O)(O₆₀₁), -S(=O)₂(Q₆₀₁), and -P(=O)(O₆₀₁)(O₆₀₂).

In embodiments, R₆₀₁ and R₆₁₁ to R₆₁₃ in Formulae 601 and 601-1 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group;
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -CI, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
-S(=O)₂(Q₆₀₁) and -P(=O)(Q₆₀₁)(Q₆₀₂),
wherein Q₆₀₁ and Q₆₀₂ are the same as described above.

In an embodiment, the electron transport compound may include at least one compound selected from Compounds ET1 to ET39 below:

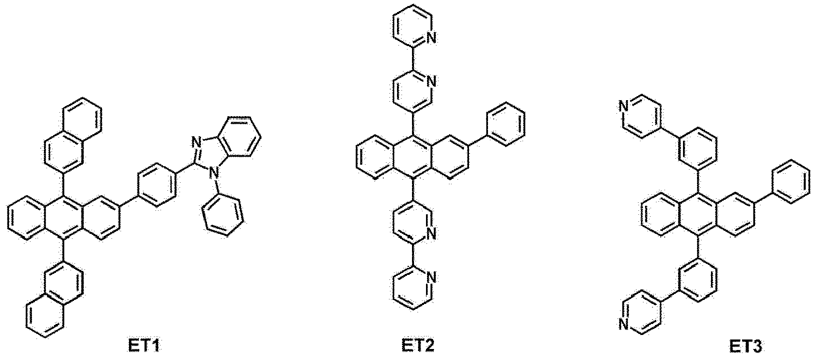

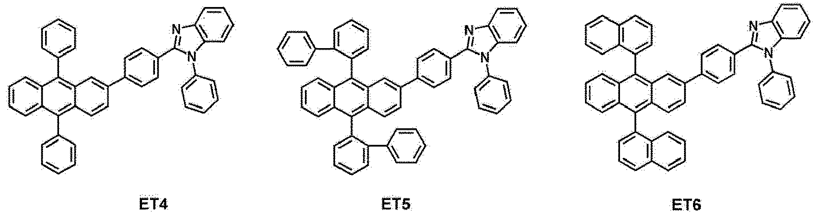

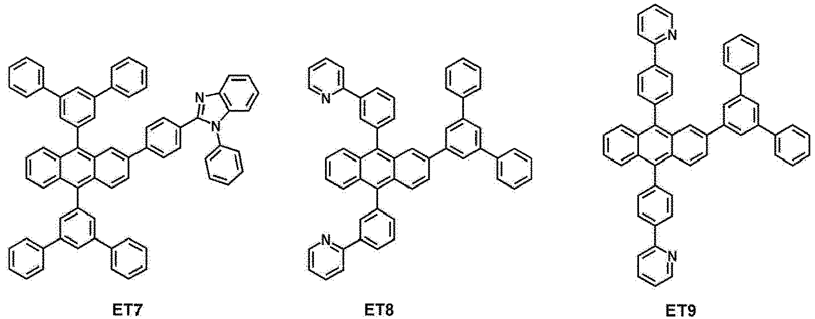

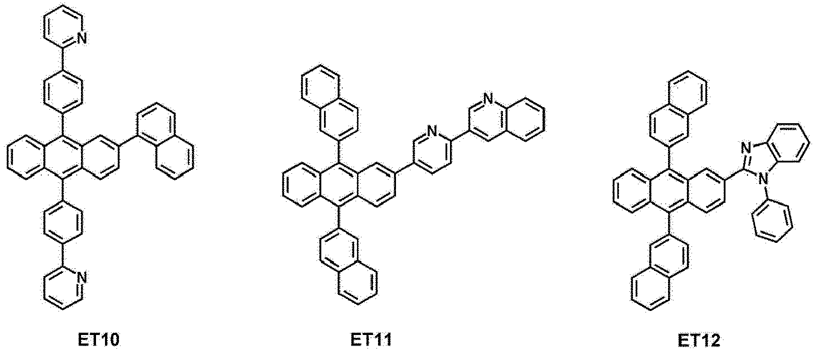

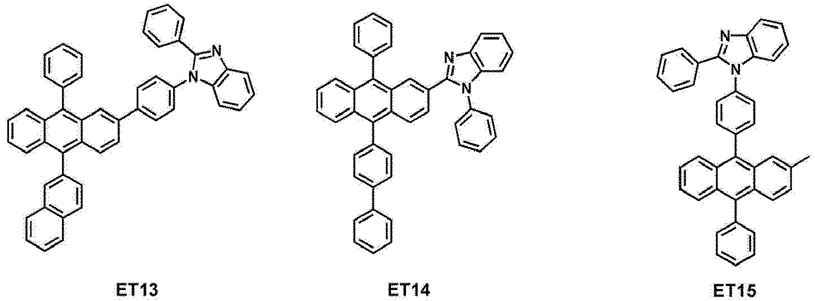

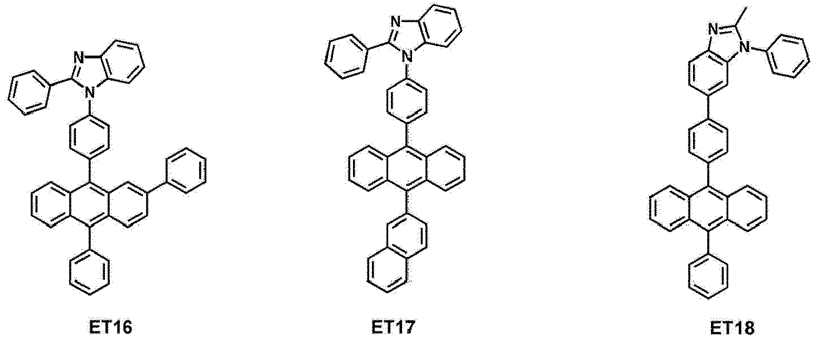

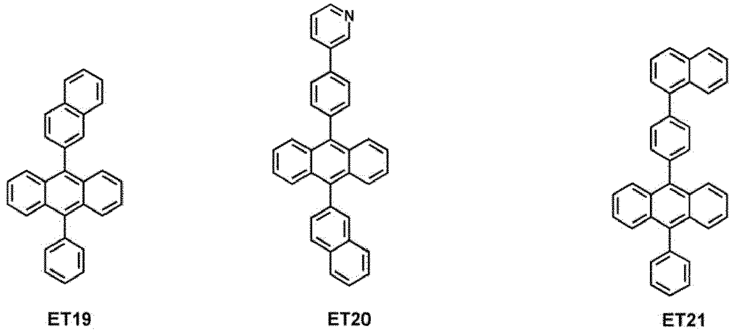

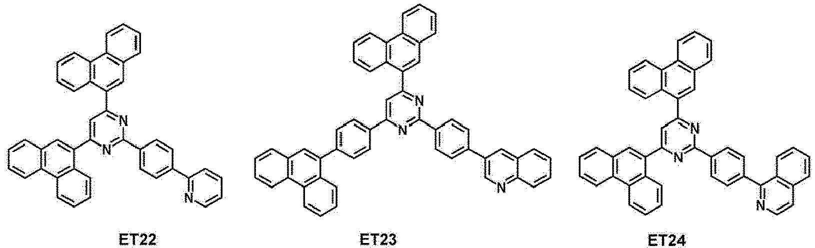

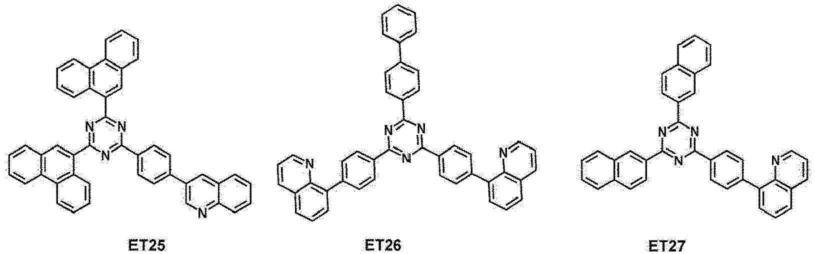

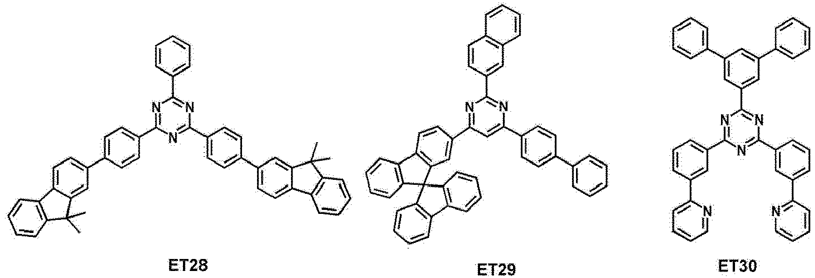

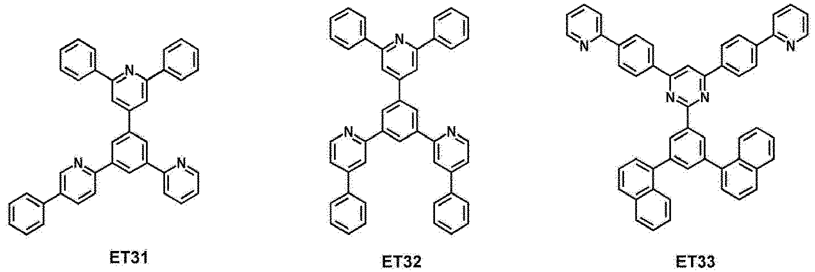

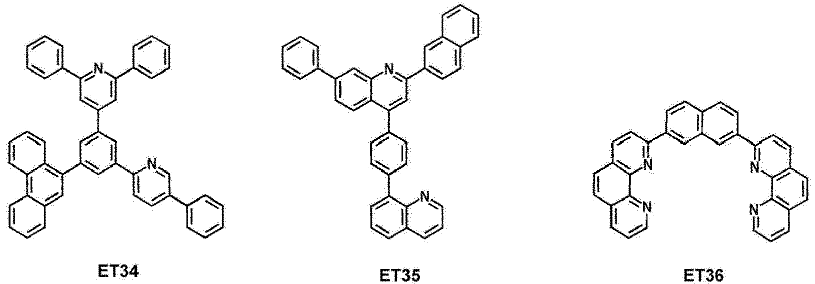

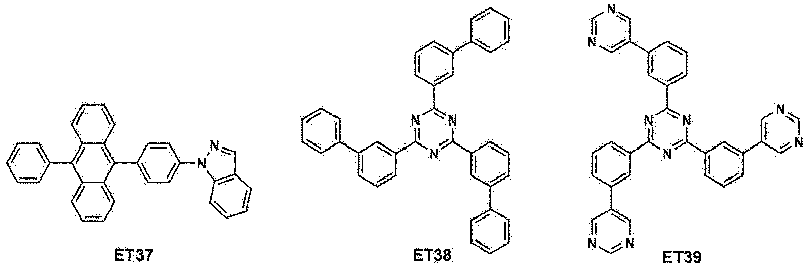

In embodiments, the electron transport compound may include at least one compound selected from 2,9-dimethyl-4,7-diphenyl-1,10-phenanthroline (BCP), 4,7-diphenyl-1,10-phenanthroline (Bphen), Alq₃, BAlq, 3-(biphenyl-4-yl)-5-(4-tert-butylphenyl)-4-phenyl-4H-1,2,4-triazole (TAZ), and NTAZ below:

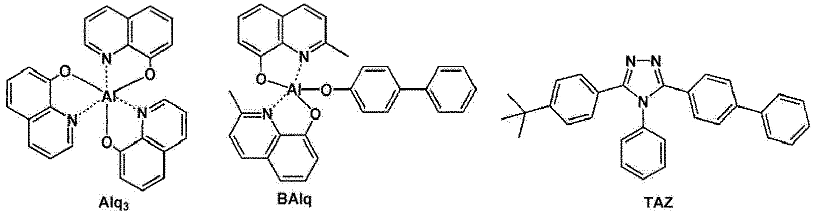

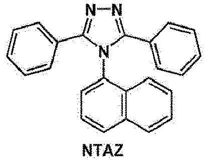

The thicknesses of the buffer layer, the hole blocking layer, and the electron control layer may each independently be in a range of about 20 Å to about 1,000 Å. For example, the thickness of the buffer layer may be in a range of about 20 Å to about 700 Å, about 20 Å to about 500 Å, about 20 Å to about 400 Å, about 30 Å to about 400 Å or about 30 Å to about 300 Å. For example, the thickness of the hole blocking layer may be in a range of about 20 Å to about 700 Å, about 20 Å to about 500 Å, about 20 Å to about 400 Å, about 30 Å to about 400 Å or about 30 Å to about 300 Å. For example, the thickness of the electron control layer may be in a range of about 20 Å to about 700 Å, about 20 Å to about 500 Å, about 20 Å to about 400 Å, about 30 Å to about 400 Å or about 30 Å to about 300 Å. When the thicknesses of the buffer layer, the hole blocking layer, and the electron control layer are within these ranges, excellent hole blocking characteristics or excellent electron control characteristics may be obtained without a substantial increase in driving voltage.

The thickness of the electron transport layer may be in a range of about 100 Å to about 1,000 Å. In embodiments, the thickness of the electron transport layer may be in a range of about 100 Å to about 700 Å, about 100 Å to about 600 Å, about 150 Å to about 600 Å, about 150 Å to about 500 Å or about 200 Å to about 400 Å. When the thickness of the electron transport layer is within any of these ranges, excellent electron transport characteristics may be obtained without a substantial increase in driving voltage.

The electron transport region (for example, the electron transport layer in the electron transport region) may further include, in addition to the materials described above, a metal-containing material.

The metal-containing material may include at least one selected from alkali metal complex and alkaline earth-metal complex. The alkali metal complex may include a metal ion selected from a Li ion, a Na ion, a K ion, a Rb ion, and a Cs ion, and the alkaline earth-metal complex may include a metal ion selected from a Be ion, a Mg ion, a Ca ion, a Sr ion, and a Ba ion. A ligand coordinated with the metal ion of the alkali metal complex or the alkaline earth-metal complex may be selected from a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy diphenyloxadiazole, a hydroxy diphenylthiadiazole, a hydroxy phenylpyridine, a hydroxy phenylbenzimidazole, a hydroxy phenylbenzothiazole, a bipyridine, a phenanthroline, and a cyclopentadiene, but embodiments are not limited thereto.

For example, the metal-containing material may include a Li complex. The Li complex may include, for example, Compound ET-D1 (lithium quinolate, LiQ) or ET-D2:

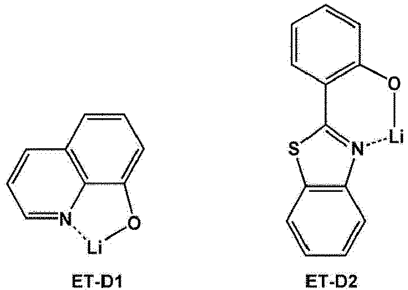

The electron transport region may include an electron injection layer that facilitates electron injection from the second electrode 190. The electron injection layer may directly contact the second electrode 190.

The electron injection layer may have i) a single-layered structure including a single layer including a single material, ii) a single-layered structure including a single layer including different materials, or iii) a multi-layered structure having multiple layers including different materials.

The electron injection layer may include an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof.

The alkali metal may be selected from Li, Na, K, Rb, and Cs. In an embodiment, the alkali metal may be Li, Na, or Cs. In embodiments, the alkali metal may be Li or Cs, but embodiments are not limited thereto.

The alkaline earth metal may be selected from Mg, Ca, Sr, and Ba.

The rare earth metal may be selected from Sc, Y, Ce, Tb, Yb, and Gd.

The alkali metal compound, the alkaline earth-metal compound, and the rare earth metal compound may be selected from oxides and halides (for example, fluorides, chlorides, bromides, or iodides) of the alkali metal, the alkaline earth-metal, and the rare earth metal.

The alkali metal compound may be selected from alkali metal oxides, such as Li₂O, CS₂O, or K₂O, and alkali metal halides, such as LiF, NaF, CsF, KF, Lil, Nal, Csl, or Kl. In an embodiment, the alkali metal compound may be selected from LiF, Li₂O, NaF, Lil, Nal, Csl, and Kl, but embodiments are not limited thereto.

The alkaline earth-metal compound may be selected from alkaline earth-metal oxides, such as BaO, SrO, CaO, BaₓSr₁₋ₓO (0<x<1), or BaₓCa₁₋ₓO (0<x<1). In an embodiment, the alkaline earth-metal compound may be selected from BaO, SrO, and CaO, but embodiments are not limited thereto.

The rare earth metal compound may be selected from YbF₃, ScF₃, SC₂O₃, Y₂O₃, Ce₂O₃, GdF₃ and TbF₃. In an embodiment, the rare earth metal compound may be selected from YbF₃, ScF₃, TbF₃, YbI₃, ScI₃, and TbI₃, but embodiments are not limited thereto.

The alkali metal complex, the alkaline earth-metal complex, and the rare earth metal complex may include an ion of alkali metal, alkaline earth-metal, and rare earth metal as described above, and a ligand coordinated with a metal ion of the alkali metal complex, the alkaline earth-metal complex, or the rare earth metal complex may be selected from hydroxy quinoline, hydroxy isoquinoline, hydroxy benzoquinoline, hydroxy acridine, hydroxy phenanthridine, hydroxy phenyloxazole, hydroxy phenylthiazole, hydroxy diphenyloxadiazole, hydroxy diphenylthiadiazole, hydroxy phenylpyridine, hydroxy phenylbenzimidazole, hydroxy phenylbenzothiazole, bipyridine, phenanthroline, and cyclopentadiene, but embodiments are not limited thereto.

In an embodiment, the electron injection layer may include a combination of an alkali metal compound and a rare earth metal compound. In an embodiment, the electron injection layer may be formed by co-deposition of RbI and Yb. In embodiments, the electron injection layer may be formed by co-deposition of Kl and Yb.

The electron injection layer may consist of an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof, as described above. In embodiments, the electron injection layer may further include an organic material. When the electron injection layer further includes an organic material, an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combination thereof may be homogeneously or non-homogeneously dispersed in a matrix including the organic material.

The thickness of the electron injection layer may be in a range of about 1 Å to about 100 Å. In embodiments, the thickness of the electron injection layer may be in a range of about 3 Å to about 90 Å or about 3 Å to about 20 Å. When the thickness of the electron injection layer is within any of these ranges, excellent electron injection characteristics may be obtained without a substantial increase in driving voltage.

### [Second electrode 190]

The second electrode 190 is disposed on the organic layer 150 having such a structure. The second electrode 190 may be a cathode which is an electron injection electrode, and in this regard, a material for forming the second electrode 190 may be selected from a metal, an alloy, an electrically conductive compound, and a combination thereof, which have a relatively low work function.

The second electrode 190 may include at least one selected from lithium (Li), silver (Ag), magnesium (Mg), aluminium (Al), aluminium-lithium (Al-Li), calcium (Ca), magnesium-indium (Mg-In), magnesium-silver (Mg-Ag), silver-magnesium (Ag-Mg), ytterbium (Yb), silver-ytterbium (Ag-Yb), ITO, and IZO, but embodiments are not limited thereto.

The second electrode 190 may be a transmissive electrode, a semi-transmissive electrode, or a reflective electrode.

The second electrode 190 may have a single-layered structure or a multi-layered structure including two or more layers.

### [Capping layer]

The organic light-emitting device may further include a capping layer, and the capping layer may be disposed on a surface of the first electrode and/or the second electrode, opposite to the surface on which the organic layer is disposed.

For example, the organic light-emitting device may have a structure in which a first capping layer, the first electrode, the organic layer, and the second electrode are sequentially stacked in this stated order, a structure in which the first electrode, the organic layer, the second electrode, and a second capping layer are sequentially stacked in this stated order, or a structure in which a first capping layer, the first electrode, the organic layer, the second electrode, and a second capping layer are sequentially stacked in this stated order.

Light generated from the emission layer included in the organic layer of the organic light-emitting device may pass through the first electrode and the first capping layer toward the outside, wherein the first electrode may be a semi-transmissive electrode or a transmissive electrode, or may pass through the second electrode and the second capping layer toward the outside, wherein the second electrode may be a semi-transmissive electrode or a transmissive electrode.

Each of the first capping layer and the second capping layer may increase external luminescence efficiency according to the principle of constructive interference.

The first capping layer and the second capping layer may each independently be an organic capping layer including an organic material, an inorganic capping layer including an inorganic material, or a composite capping layer including an organic material and an inorganic material.

At least one selected from the first capping layer and the second capping layer may each independently include at least one material selected from a carbocyclic compound, a heterocyclic compound, an amine-based compound, a porphyrine derivative, a phthalocyanine derivative, a naphthalocyanine derivative, an alkali metal complex, and an alkaline earth-based complex. The carbocyclic compound, the heterocyclic compound, and the amine-based compound may be optionally substituted with a substituent containing at least one element selected from O, N, S, Se, Si, F, Cl, Br, and I. In an embodiment, at least one of the first capping layer and the second capping layer may each independently include an amine-based compound.

In an embodiment, at least one selected from the first capping layer and the second capping layer may each independently include the compound represented by Formula 201 or the compound represented by Formula 202.

In embodiments, at least one selected from the first capping layer and the second capping layer may each independently include a compound selected from Compounds HT28 to HT33 above and Compounds CP1 to CP5 below, but embodiments are not limited thereto:

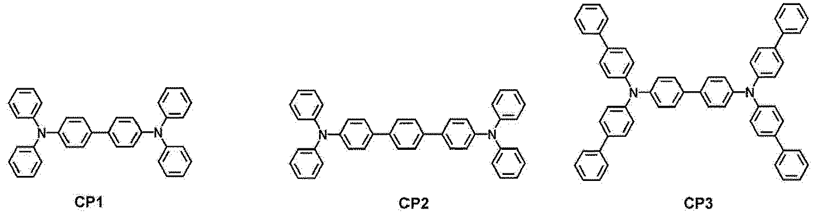

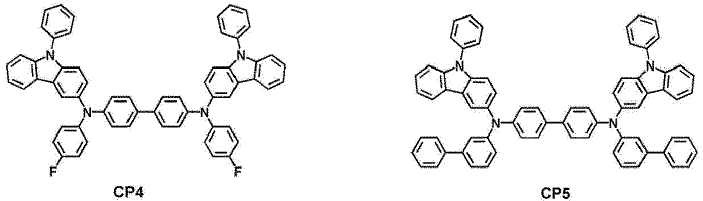

### [General preparation methods]

Layers constituting the hole transport region, the emission layer, and layers constituting the electron transport region may be formed in a certain region by using one or more suitable methods selected from vacuum deposition, spin coating, casting, Langmuir-Blodgett (LB) deposition, ink-jet printing, laser-printing, and laser-induced thermal imaging.

When layers constituting the hole transport region, the emission layer, and layers constituting the electron transport region are formed by vacuum deposition, the deposition may be performed at a deposition temperature of about 100 Å to about 500 Å, a vacuum degree of about 10⁻⁸ torr to about 10⁻³ torr, and a deposition speed of about 0.01 Å/sec to about 100 Å/sec by taking into account a material to be included in a layer to be formed and the structure of a layer to be formed.

When layers constituting the hole transport region, the emission layer, and layers constituting the electron transport region are formed by spin coating, the spin coating may be performed at a coating speed of about 2,000 rpm to about 5,000 rpm and at a heat treatment temperature of about 80 °C to 200 °C by taking into account a material to be included in a layer to be formed and the structure of a layer to be formed.

### [Apparatus]

The organic light-emitting device may be included in various apparatuses.

According to an embodiment, provided is an apparatus including the organic light-emitting device.

For example, the apparatus may be a light-emitting apparatus, an authentication apparatus, or an electronic apparatus, but embodiments are not limited thereto.

The light-emitting apparatus may be used as various displays, light sources, and the like.

The authentication apparatus may be, for example, a biometric authentication apparatus for authenticating an individual by using biometric information of a biometric body (for example, a fingertip, a pupil, or the like).

The authentication apparatus may further include, in addition to the organic light-emitting device, a biometric information collector.

The electronic apparatus may be applied to personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram (ECG) displays, ultrasonic diagnostic devices, or endoscope displays), fish finders, various measuring instruments, meters (for example, meters for a vehicle, an aircraft, and a vessel), projectors, and the like, but embodiments are not limited thereto.

In an embodiment, the apparatus may further include, in addition to the organic light-emitting device, a thin-film transistor. Here, the thin-film transistor may include a source electrode, an activation layer, and a drain electrode, wherein the first electrode of the organic light-emitting device may be in electrical contact with one of the source electrode and the drain electrode of the thin-film transistor.

### [Electronic apparatus]

According to an embodiment, provided is an electronic apparatus including a substrate and an organic light-emitting device disposed on the substrate. The organic light-emitting device is the same as described above.

In an embodiment, the electronic apparatus may include a color conversion layer disposed on at least one traveling direction of light emitted from the organic light-emitting device, and the color conversion layer may include quantum dots.

### [Quantum dot]

The emission layer included in the organic light-emitting device of the disclosure may include quantum dots.

In an embodiment, the color conversion layer included in the electronic apparatus may include a quantum dot material.

The quantum dot is a particle having a crystal structure of several to several tens of nanometers, and includes hundreds to thousands of atoms.

Since the quantum dot is very small in size, a quantum confinement effect may occur. The quantum confinement effect refers to a phenomenon in which a band gap of an object becomes large when the object becomes smaller than a nanometer size. Accordingly, when light having a wavelength having an energy intensity that is greater than the band gap of the quantum dot is irradiated to the quantum dot, the quantum dot is excited by absorbing the light and emits light having a specific wavelength and transits to the ground state. The wavelength of the emitted light has a value corresponding to the band gap.

The core of the quantum dot may include a Group II-VI compound, a Group III-VI compound, a Group III-V compound, a Group IV-VI compound, a Group IV element or compound, a Group I-III-VI compound, or any combination thereof.

The Group II-VI compound may be selected from a binary compound selected from CdS, CdSe, CdTe, ZnS, ZnSe, ZnTe, ZnO, HgS, HgSe, HgTe, MgSe, MgS, and any mixture thereof; a ternary compound selected from CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, any mixture thereof; and a quaternary compound selected from CdZnSeS, CdZnSeTe, CdZnSTe, CdHgSeS, CdHgSeTe, CdHgSTe, HgZnSeS, HgZnSeTe, HgZnSTe, and any mixture thereof.

The Group III-VI compound may include: a binary compound, such as In₂S₃ or In₂Se; a ternary compound, such as InGaS₃ or InGaSe₃; or any combination thereof.

For example, the Group III-V compound may be selected from: a binary compound selected from GaN, GaP, GaAs, GaSb, AlN, AlP, AlAs, AlSb, InN, InP, InAs, InSb, and any mixture thereof; a ternary compound selected from GaNP, GaNAs, GaNSb, GaPAs, GaPSb, AlNP, AlNAs, AlNSb, AlPAs, AlPSb, InGaP, InAlP, InNP, InNAs, InNSb, InPAs, InPSb, and any mixture thereof; and a quaternary compound selected from GaAlNP, GaAlNAs, GaAlNSb, GaAlPAs, GaAlPSb, GalnNP, GalnNAs, GalnNSb, GalnPAs, GalnPSb, InAlNP, InAlNAs, InAlNSb, InAlPAs, InAlPSb, and any mixture thereof, but embodiments are not limited thereto. The Group III-V semiconductor compound may further include Group II metal (for example, InZnP, etc.).

The IV-VI group compound may be selected from: a binary compound selected from SnS, SnSe, SnTe, PbS, PbSe, PbTe, and any mixture thereof; a ternary compound selected from SnSeS, SnSeTe, SnSTe, PbSeS, PbSeTe, PbSTe, SnPbS, SnPbSe, SnPbTe, and any mixture thereof; and a quaternary compound selected from SnPbSSe, SnPbSeTe, SnPbSTe, and any mixture thereof. The IV group element may be selected from Si, Ge, and any mixture thereof. The IV group compound may be a binary compound selected from SiC, SiGe, and any mixture thereof.

The Group I-III-VI semiconductor compound may include a ternary compound, such as AglnS, AglnS₂, CulnS, CulnS₂, CuGaO₂, AgGaO₂, or AgAlO₂; or any combination thereof.

The binary compound, the ternary compound, or the quaternary compound may exist in particles at uniform concentration, or may exist in the same particle in a state in which a concentration distribution is partially different. The binary compound, the ternary compound, or the quaternary compound may have a core-shell structure in which one quantum dot surrounds another quantum dot. An interface between the core and the shell may have a concentration gradient in which the concentration of elements existing in the shell decreases toward the center.

In embodiments, the quantum dot may have a core-shell structure including a core with the above-described nanoparticles and a shell surrounding the core. The shell of the quantum dot may serve as a protective layer for maintaining semiconductor characteristics by preventing chemical degeneration of the core and/or may serve as a charging layer for imparting electrophoretic characteristics to the quantum dot. The shell may be a single layer or a multilayer. An interface between the core and the shell may have a concentration gradient in which the concentration of elements existing in the shell decreases toward the center. Examples of the shell of the quantum dot may include a metal or non-metal oxide, a semiconductor compound, or any combination thereof.

Examples of the metal or non-metal oxide are binary compounds, such as SiO₂, Al₂O₃, TiO₂, ZnO, MnO, Mn₂O₃, Mn₃O₄, CuO, FeO, Fe₂O₃, Fe₃O₄, CoO, Co₃O₄, and NiO, or ternary compounds, such as MgAl₂O₄, CoFe₂O₄, NiFe₂O₄, and CoMn₂O₄, but embodiments are not limited thereto.

Examples of the semiconductor compound are CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, and the like, but embodiments are not limited thereto.

A full width of half maximum (FWHM) of an emission wavelength spectrum of the quantum dot may be less than or equal to about 45 nm. For example, the full width of half maximum (FWHM) of an emission wavelength spectrum of the quantum dot may be less than or equal to about 40 nm. For example, the full width of half maximum (FWHM) of an emission wavelength spectrum of the quantum dot may be less than or equal to about 30 nm. Light emitted through such quantum dot is irradiated omnidirectionally, thereby improving a wide viewing angle.

The shape of the quantum is not particularly limited, but is one that is commonly used in the art. More specifically, a spherical, pyramidal, multi-arm, or cubic nanoparticle, nanotube, nanowire, nanofiber, or nanoplate particle may be used.

The quantum dot may adjust the color of emitted light according to the particle size. Therefore, the quantum dot may emit various emission colors such as blue, red, or green.

### [Definitions of substituents]

The term "C₁-C₆₀ alkyl group" as used herein refers to a linear or branched aliphatic saturated hydrocarbon monovalent group having 1 to 60 carbon atoms, and examples thereof include a methyl group, an ethyl group, a propyl group, an isobutyl group, a sec-butyl group, a tert-butyl group, a pentyl group, an isoamyl group, and a hexyl group. The term "C₁-C₆₀ alkylene group" as used herein refers to a divalent group having the same structure as the C₁-C₆₀ alkyl group. Corresponding definitions apply to other ranges given for the number of carbon atoms in an alkyl/alkylene group.

The term "C₂-C₆₀ alkenyl group" as used herein refers to a hydrocarbon group having at least one carbon-carbon double bond in the middle or at the terminus of the C₂-C₆₀ alkyl group, and examples thereof include an ethenyl group, a propenyl group, and a butenyl group. The term "C₂-C₆₀ alkenylene group" as used herein refers to a divalent group having the same structure as the C₂-C₆₀ alkenyl group. Corresponding definitions apply to other ranges given for the number of carbon atoms in an alkenyl/alkenylene group.

The term "C₂-C₆₀ alkynyl group" as used herein refers to a hydrocarbon group having at least one carbon-carbon triple bond in the middle or at the terminus of the C₂-C₆₀ alkyl group, and examples thereof include an ethynyl group, and a propynyl group. The term "C₂-C₆₀ alkynylene group" as used herein refers to a divalent group having the same structure as the C₂-C₆₀ alkynyl group. Corresponding definitions apply to other ranges given for the number of carbon atoms in an alkynyl/alkynylene group.

The term "C₁-C₆₀ alkoxy group" as used herein refers to a monovalent group represented by -OA₁₀₁ (wherein A₁₀₁ is the C₁-C₆₀ alkyl group), and examples thereof include a methoxy group, an ethoxy group, and an isopropyloxy group. Corresponding definitions apply to other ranges given for the number of carbon atoms in an alkoxy group.

The term "C₃-C₁₀ cycloalkyl group" as used herein refers to a monovalent saturated hydrocarbon monocyclic group having 3 to 10 carbon atoms, and examples thereof include a cyclopropyl group, a cyclobutyl group, a cyclopentyl group, a cyclohexyl group, and a cycloheptyl group. The term "C₃-C₁₀ cycloalkylene group" as used herein refers to a divalent group having the same structure as the C₃-C₁₀ cycloalkyl group.

The term "C₁-C₁₀ heterocycloalkyl group" as used herein refers to a monovalent monocyclic group having at least one heteroatom selected from N, O, Si, P, and S as a ring-forming atom and 1 to 10 carbon atoms, and examples thereof include a 1,2,3,4-oxatriazolidinyl group, a tetrahydrofuranyl group, and a tetrahydrothiophenyl group. The term "C₁-C₁₀ heterocycloalkylene group" as used herein refers to a divalent group having the same structure as the C₁-C₁₀ heterocycloalkyl group.

The term "C₃-C₁₀ cycloalkenyl group" as used herein refers to a monovalent monocyclic group that has 3 to 10 carbon atoms and at least one carbon-carbon double bond in the ring thereof and no aromaticity, and examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent monocyclic group that has at least one heteroatom selected from N, O, Si, P, and S as a ring-forming atom, 1 to 10 carbon atoms, and at least one carbon-carbon double bond in its ring. Examples of the C₁-C₁₀ heterocycloalkenyl group include a 4,5-dihydro-1,2,3,4-oxatriazolyl group, a 2,3-dihydrofuranyl group, and a 2,3-dihydrothiophenyl group. The term "C₁-C₁₀ heterocycloalkylene group" as used herein refers to a divalent group having the same structure as the C₁-C₁₀ heterocycloalkyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the two or more rings may be fused to each other. Corresponding definitions apply to other ranges given for the number of carbon atoms in an aryl/arylene group.

The term "C₁-C₆₀ heteroaryl group" as used herein refers to a monovalent group having a carbocyclic aromatic system that has at least one heteroatom selected from N, O, Si, P, and S as a ring-forming atom, in addition to 1 to 60 carbon atoms. The term "C₁-C₆₀ heteroarylene group" as used herein refers to a divalent group having a carbocyclic aromatic system that has at least one heteroatom selected from N, O, Si, P, and S as a ring-forming atom, in addition to 1 to 60 carbon atoms. Examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the two or more rings may be condensed with each other. Corresponding definitions apply to other ranges given for the number of carbon atoms in an heteroaryl/heteroarylene group.

The term "C₆-C₆₀ aryloxy group" as used herein refers to -OA₁₀₂ (wherein A₁₀₂ is the C₆-C₆₀ aryl group), and the term "C₆-C₆₀ arylthio group" as used herein refers to -SA₁₀₃ (wherein A₁₀₃ is the C₆-C₆₀ aryl group). Corresponding definitions apply to other ranges given for the number of carbon atoms in an aryloxy group and an arylthio group.

The term "monovalent non-aromatic condensed polycyclic group" as used herein refers to a monovalent group (for example, having 8 to 60 carbon atoms) having two or more rings condensed with each other, only carbon atoms as ring-forming atoms, and no aromaticity in its entire molecular structure. A detailed example of the monovalent non-aromatic condensed polycyclic group is a fluorenyl group. The term "divalent non-aromatic condensed polycyclic group" as used herein refers to a divalent group having substantially the same structure as the monovalent non-aromatic condensed polycyclic group.

The term "monovalent non-aromatic condensed heteropolycyclic group" as used herein refers to a monovalent group (for example, having 1 to 60 carbon atoms) having two or more rings condensed to each other, at least one heteroatom selected from N, O, Si, P, and S, other than carbon atoms, as a ring-forming atom, and no aromaticity in its entire molecular structure. An example of the monovalent non-aromatic condensed heteropolycyclic group is a carbazolyl group. The term "divalent non-aromatic condensed heteropolycyclic group" as used herein refers to a divalent group having substantially the same structure as the monovalent non-aromatic condensed heteropolycyclic group.

The term "C₅-C₆₀ carbocyclic group" as used herein refers to a monocyclic or polycyclic group that includes only carbon as a ring-forming atom and consists of 5 to 60 carbon atoms. The C₅-C₆₀ carbocyclic group may be an aromatic carbocyclic group or a non-aromatic carbocyclic group. The C₅-C₆₀ carbocyclic group may be a ring, such as benzene, a monovalent group, such as a phenyl group, or a divalent group, such as a phenylene group. In embodiments, depending on the number of substituents connected to the C₅-C₆₀ carbocyclic group, the C₅-C₆₀ carbocyclic group may be a trivalent group or a quadrivalent group. For example, the term "benzene group" as used herein may represent a benzene ring, a phenyl group, a phenylene group, or a trivalent or tetravelent group corresponding thereto. Corresponding definitions apply to other ranges given for the number of carbon atoms in a carbocyclic group.

The term "C₁-C₆₀ heterocyclic group" as used herein refers to a group having the same structure as the C₅-C₆₀ carbocyclic group, except that as a ring-forming atom, at least one heteroatom selected from N, O, Si, P, and S is used in addition to carbon (the number of carbon atoms may be in a range of 1 to 60). Corresponding definitions apply to other ranges given for the number of carbon atoms in a heterocyclic group.

In the specification, at least one substituent of the substituted C₅-C₆₀ (*e.g*. C₅-C₃₀) carbocyclic group, the substituted C₁-C₆₀ (*e.g*. C₁-C₂₀) heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ (*e.g*. C₆-C₃₀) arylene group, the substituted C₁-C₆₀ (*e.g.* C₁-C₂₀) heteroarylene group, the substituted divalent non-aromatic condensed polycyclic group, the substituted divalent non-aromatic condensed heteropolycyclic group, the substituted C₁-C₆₀ (*e.g*. C₁-C₂₀) alkyl group, the substituted C₂-C₆₀ (*e.g.* C₂-C₂₀) alkenyl group, the substituted C₂-C₆₀ (*e.g.* C₂-C₂₀) alkynyl group, the substituted C₁-C₆₀ (*e.g*. C₁-C₂₀) alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ (*e.g.* C₆-C₃₀) aryl group, the substituted C₆-C₆₀ (*e.g.* C₆-C₃₀) aryloxy group, the substituted C₆-C₆₀ (*e.g.* C₆-C₃₀) arylthio group, the substituted C₁-C₆₀ (*e.g.* C₁-C₂₀) heteroaryl group, the substituted monovalent non-aromatic condensed polycyclic group, and the substituted monovalent non-aromatic condensed heteropolycyclic group may be selected from:
deuterium, -F, -CI, -Br, -I, a hydroxyl group, a cyano group, a nitro group, an amidino group, a hydrazino group, a hydrazono group, a C₁-C₆₀ (*e.g*. C₁-C₂₀) alkyl group, a C₂-C₆₀ (*e.g.* C₂-C₂₀) alkenyl group, a C₂-C₆₀ (*e.g.* C₂-C₂₀) alkynyl group, and a C₁-C₆₀ (*e.g*. C₁-C₂₀) alkoxy group;
a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, and a C₁-C₆₀ (*e.g.* C₁-C₂₀) alkoxy group, each substituted with at least one selected from deuterium, -F, -CI, -Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, a monovalent non-aromatic condensed heteropolycyclic group, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), and -P(=O)(Q₁₁)(Q₁₂),
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g*. C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g*. C₁-C₂₀) heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g*. C₆-C₃₀) aryl group, a C₆-C₆₀ (*e.g.* C₆-C₃₀) aryloxy group, a C₆-C₆₀ (*e.g.* C₆-C₃₀) arylthio group, a C1-C₆₀ (*e.g.* C₁-C₂₀) heteroaryl group, a monovalent non-aromatic condensed polycyclic group, and a monovalent non-aromatic condensed heteropolycyclic group, each substituted with at least one selected from deuterium, -F, -CI, -Br, -I, a hydroxyl group, a cyano group, a nitro group, an amidino group, a hydrazino group, a hydrazono group, a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, a monovalent non-aromatic condensed heteropolycyclic group, -Si(Q₂₁)(Q₂₂)(Q₂₃), -N(Q₂₁)(Q₂₂), - B(Q₂₁)(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
wherein Q₁₁ to Q₁₃, Q₂₁ to Q₂₃, and Q₃₁ to Q₃₃ may each independently be selected from hydrogen, deuterium, -F, -CI, -Br, -I, a hydroxyl group, a cyano group, a nitro group, an amidino group, a hydrazino group, a hydrazono group, a C₁-C₆₀ *(e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g*. C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₁-C₆₀ (*e.g.* C₁-C₂₀) heteroaryl group, a monovalent non-aromatic condensed polycyclic group, a monovalent non-aromatic condensed heteropolycyclic group, a biphenyl group, and a terphenyl group.

The term "Ph" as used herein refers to a phenyl group, the term "Me" as used herein refers to a methyl group, the term "Et" as used herein refers to an ethyl group, the term "ter-Bu" or "Bu^{t}" as used herein refers to a tert-butyl group, and the term "OMe" as used herein refers to a methoxy group.

The term "biphenyl group" as used herein refers to "a phenyl group substituted with a phenyl group". In other words, the "biphenyl group" is a substituted phenyl group having a C₆-C₆₀ aryl group as a substituent.

The term "terphenyl group" as used herein refers to "a phenyl group substituted with a biphenyl group". In other words, the "terphenyl group" is a substituted phenyl group having, as a substituent, a C₆-C₆₀ aryl group substituted with a C₆-C₆₀ aryl group.

* and *' as used herein, unless defined otherwise, each refer to a binding site to a neighboring atom in a corresponding formula.

Hereinafter, a compound according to embodiments and an organic light-emitting device according to embodiments will be described in detail with reference to Examples. The wording "B was used instead of A" used in describing Examples refers to that an identical molar equivalent of B was used in place of A.

### Examples

### Example 1

A 15 Ω/cm² (800 Å) ITO/Ag/ITO glass substrate (a product of Corning Inc.) was cut to a size of 50 mm x 50 mm x 0.7 mm, sonicated with isopropyl alcohol and pure water each for 5 minutes, and cleaned by exposure to ultraviolet rays and ozone for 15 minutes. The resultant glass substrate was loaded onto a vacuum deposition apparatus.

HAT-CN was deposited on the ITO/Ag/ITO anode of the glass substrate to form a hole injection layer having a thickness of 50 Å, NPB was deposited on the hole injection layer to form a hole transport layer having a thickness of 250 Å, TCTA was deposited on the hole transport layer to form an electron blocking layer having a thickness of 50 Å, HT56 and FD23 were co-deposited on the electron blocking layer at a volume ratio of 97:3 to form an emission layer having a thickness of 200 Å, T2T was deposited on the emission layer to form a hole blocking layer having a thickness of 50 Å, and TPM-TAZ and LiQ were co-deposited on the hole blocking layer at a volume ratio of 1:1 to form an electron transport layer having a thickness of 250 Å, thereby forming a first emission unit.

Compound 1 and Li were co-deposited on the first emission unit at a volume ratio of 99:1 to form an n-type charge generation layer having a thickness of 150 Å, and HAT-CN was deposited on the n-type charge generation layer to form a p-type charge generation layer having a thickness of 50 Å, thereby forming a first charge generation unit.

NPB was deposited on the first charge generation unit to form a hole transport layer having a thickness of 500 Å, TCTA was deposited on the hole transport layer to form an electron blocking layer having a thickness of 50 Å, HT56 and FD23 were co-deposited on the electron blocking layer at a volume ratio of 97:3 to form an emission layer having a thickness of 200 Å, T2T was deposited on the emission layer to form a hole blocking layer having a thickness of 50 Å, and TPM-TAZ and LiQ were co-deposited on the hole blocking layer at a volume ratio of 1:1 to form an electron transport layer having a thickness of 250 Å, thereby forming a second emission unit.

Compound 1 and Li were co-deposited on the second emission unit at a volume ratio of 99:1 to form an n-type charge generation layer having a thickness of 150 Å, and HAT-CN was deposited on the n-type charge generation layer to form a p-type charge generation layer having a thickness of 50 Å, thereby forming a second charge generation unit.

NPB was deposited on the second charge generation unit to form a hole transport layer having a thickness of 400 Å, TCTA was deposited on the hole transport layer to form an electron blocking layer having a thickness of 50 Å, HT56 and FD23 were co-deposited on the electron blocking layer at a volume ratio of 97:3 to form an emission layer having a thickness of 200 Å, T2T was deposited on the emission layer to form a hole blocking layer having a thickness of 50 Å, and TPM-TAZ and LiQ were co-deposited on the hole blocking layer at a volume ratio of 1:1 to form an electron transport layer having a thickness of 300 Å, thereby forming a third emission unit.

Yb was deposited on the third emission unit to a thickness of 10 Å, and Ag and Mg were co-deposited thereon at a volume ratio of 9:1 to form a cathode having a thickness of 100 Å, thereby manufacturing a tandem organic light-emitting device.

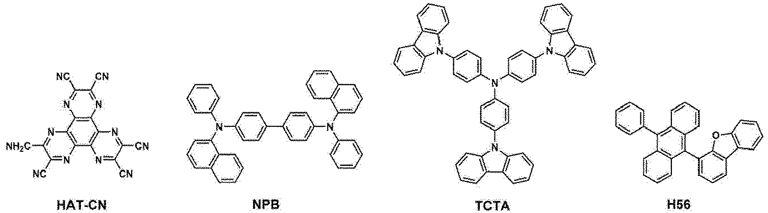

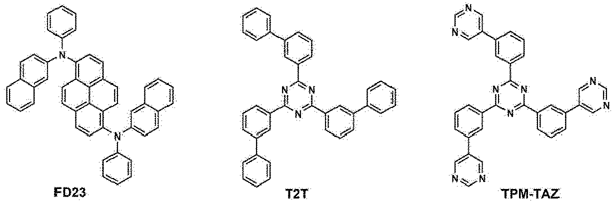

### Examples 2 to 42 and Comparative Examples 1 to 3 and 5 to 8

Organic light-emitting devices were manufactured in the same manner as in Example 1, except that, in forming an n-type charge generation layer, corresponding compounds shown in Table 1 were used.

### Evaluation Example 1

The driving voltage, change in driving voltage, current efficiency, lifespan, and CIE color coordinate of the light-emitting devices manufactured according to Examples 1 to 42 and Comparative Examples 1 to 3 and 5 to 8 were measured by using Keithley SMU 236 and a luminance meter PR650, and results thereof are shown in Table 1. The lifespan (T₉₈) is a period of time that was taken until the luminance (@1200 nit) was reduced to 98 % of initial luminance (100 %) after a light-emitting device was driven. The change in driving voltage (voltage shift, ΔV) is a difference between the driving voltage measured after 100 hours of driving a light-emitting device and the initial driving voltage.

**[Table 1]**

| | n-type charge generation layer Compound | Driving voltage (V) | Efficiency (Cd/A) | Lifespan (T₉₈) | Luminance (nit) | ΔV (@100 hr) |
|---|---|---|---|---|---|---|
| Example 1 | Compound 1 | 9.7 | 20.5 | 350 | 1200 | 0.21 |
| Example 2 | Compound 2 | 10.0 | 19.5 | 348 | 1200 | 0.24 |
| Example 3 | Compound 3 | 10.2 | 19.6 | 340 | 1200 | 0.28 |
| Example 4 | Compound 4 | 10.3 | 19.5 | 347 | 1200 | 0.31 |
| Example 5 | Compound 5 | 10.1 | 20.1 | 350 | 1200 | 0.25 |
| Example 6 | Compound 6 | 10.1 | 19.2 | 348 | 1200 | 0.26 |
| Example 7 | Compound 7 | 9.9 | 19.5 | 335 | 1200 | 0.33 |
| Example 8 | Compound 8 | 9.6 | 19.8 | 335 | 1200 | 0.29 |
| Example 9 | Compound 9 | 9.8 | 20.2 | 340 | 1200 | 0.28 |
| Example 10 | Compound 10 | 9.4 | 20.6 | 336 | 1200 | 0.26 |
| Example 11 | Compound 11 | 9.5 | 19.5 | 350 | 1200 | 0.23 |
| Example 12 | Compound 12 | 9.6 | 19.3 | 352 | 1200 | 0.25 |
| Example 13 | Compound 13 | 9.6 | 18.9 | 337 | 1200 | 0.22 |
| Example 14 | Compound 14 | 9.7 | 19.2 | 364 | 1200 | 0.26 |
| Example 15 | Compound 15 | 9.6 | 20.3 | 366 | 1200 | 0.28 |
| Example 16 | Compound 16 | 9.5 | 20.5 | 365 | 1200 | 0.25 |
| Example 17 | Compound 17 | 9.6 | 22.1 | 363 | 1200 | 0.26 |
| Example 18 | Compound 18 | 9.6 | 20.5 | 370 | 1200 | 0.25 |
| Example 19 | Compound 19 | 9.5 | 21.5 | 358 | 1200 | 0.27 |
| Example 20 | Compound 20 | 9.5 | 22.0 | 348 | 1200 | 0.26 |
| Example 21 | Compound 21 | 10.1 | 20.8 | 320 | 1200 | 0.24 |
| Example 22 | Compound 22 | 9.8 | 20.2 | 342 | 1200 | 0.18 |
| Example 23 | Compound 23 | 9.8 | 19.8 | 335 | 1200 | 0.19 |
| Example 24 | Compound 24 | 9.9 | 20.3 | 360 | 1200 | 0.16 |
| Example 25 | Compound 25 | 9.7 | 20.4 | 335 | 1200 | 0.18 |
| Example 26 | Compound 26 | 9.6 | 20.1 | 337 | 1200 | 0.16 |
| Example 27 | Compound 27 | 9.7 | 19.5 | 340 | 1200 | 0.14 |
| Example 28 | Compound 28 | 9.8 | 19.8 | 342 | 1200 | 0.16 |
| Example 29 | Compound 29 | 9.6 | 20.2 | 340 | 1200 | 0.29 |
| Example 30 | Compound 30 | 9.7 | 19.8 | 338 | 1200 | 0.28 |
| Example 31 | Compound 31 | 9.8 | 20.2 | 335 | 1200 | 0.33 |
| Example 32 | Compound 32 | 9.7 | 19.8 | 345 | 1200 | 0.36 |
| Example 33 | Compound 33 | 9.6 | 20.2 | 330 | 1200 | 0.40 |
| Example 34 | Compound 34 | 9.9 | 19.9 | 342 | 1200 | 0.38 |
| Example 35 | Compound 35 | 9.7 | 21.0 | 328 | 1200 | 0.33 |
| Example 36 | Compound 36 | 9.5 | 23.2 | 388 | 1200 | 0.10 |
| Example 37 | Compound 37 | 9.6 | 23.1 | 385 | 1200 | 0.12 |
| Example 38 | Compound 38 | 9.6 | 22.5 | 377 | 1200 | 0.13 |
| Example 39 | Compound 39 | 9.5 | 21.5 | 368 | 1200 | 0.11 |
| Example 40 | Compound 40 | 9.6 | 21.6 | 375 | 1200 | 0.15 |
| Example 41 | Compound 41 | 9.5 | 22.5 | 380 | 1200 | 0.13 |
| Example 42 | Compound 42 | 9.5 | 23.0 | 369 | 1200 | 0.11 |
| Com parative Example 1 | Compound A | 10.0 | 18.5 | 280 | 1200 | 0.66 |
| Com parative Example 2 | Compound B | 10.1 | 18.3 | 250 | 1200 | 0.90 |
| Com parative Example 3 | Compound C | 10.1 | 18.0 | 230 | 1200 | 0.88 |
| Com parative Example 5 | Compound E | 10.3 | 18.3 | 205 | 1200 | 0.66 |
| Com parative Example 6 | Compound F | 10.0 | 18.5 | 265 | 1200 | 0.65 |
| Com parative Example 7 | Compound G | 10.1 | 18.5 | 224 | 1200 | 0.67 |
| Com parative Example 8 | Compound H | 9.9 | 19.2 | 320 | 1200 | 0.45 |

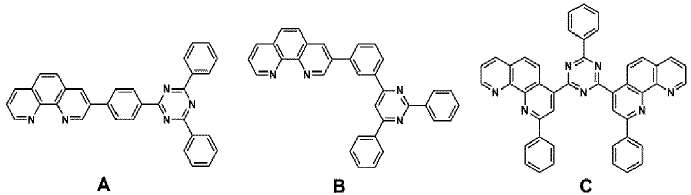

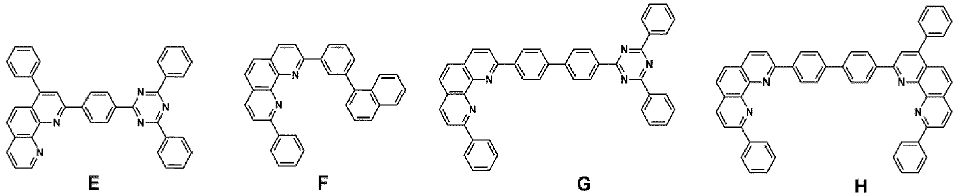

Referring to Table 1, it was confirmed that the organic light-emitting device according to an embodiment had low driving voltage, excellent efficiency, and long lifespan, and that the change in driving voltage was small.

### Examples 43 to 46 and Comparative Example 9

Organic light-emitting devices were manufactured in the same manner as in Example 1, except that, in forming an electron transport layer and an n-type charge generation layer, corresponding compounds shown in Table 2 were used.

### Evaluation Example 2

The driving voltage, change in driving voltage, current density, lifespan, and CIE color coordinate of the light-emitting devices manufactured according to Examples 43 to 46 and Comparative Example 9 were measured by using Keithley SMU 236 and a luminance meter PR650, and results thereof are shown in Table 2. The lifespan (T₉₇) is a period of time that was taken until the luminance (@1200 nit) was reduced to 97 % of initial luminance (100 %) after a light-emitting device was driven. The change in driving voltage (voltage shift, ΔV) is a difference between the driving voltage measured after 200 hours of driving a light-emitting device and the initial driving voltage.

**[Table 2]**

| | Electron transport layer | n-type charge generation layer compound | Driving voltage (V) | Efficiency (Cd/A) | Lifespan (T₉₇) | Luminance (nit) | ΔV (@200 hr) |
|---|---|---|---|---|---|---|---|
| Example 43 | ET39 | Compound 1 | 9.7 | 20.5 | 350 | 1200 | 0.21 |
| Example 44 | ET37 | Compound 1 | 10.2 | 22.8 | 180 | 1200 | 0.65 |
| Example 45 | ET39 | Compound 1 + ET39 | 9.4 | 24.1 | 350 | 1200 | 0.24 |
| Example 46 | ET37 | Compound 1 + ET37 | 9.4 | 25.3 | 340 | 1200 | 0.25 |
| Comparative Example 9 | ET39 | Compound J + Compound K | 9.6 | 23.5 | 48 | 1200 | 1.52 |

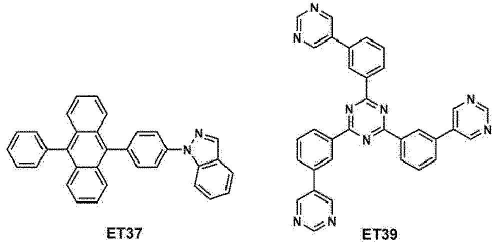

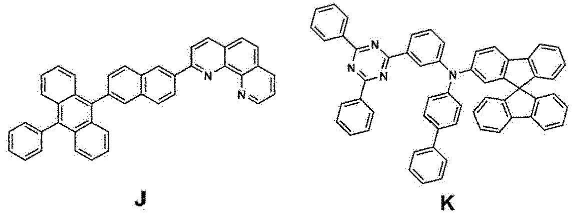

Referring to Table 2, it was confirmed that the organic light-emitting device according to an embodiment had low driving voltage, excellent efficiency, and long lifespan, and that the change in driving voltage was small.

In Comparative Example 9, a compound including an amine group having a single bond was used as an n-type charge generation layer material. Although the driving voltage of the organic light-emitting device of Comparative Example 9 was slightly reduced while the efficiency of the same organic light-emitting device was slightly increased, the amine group was easily susceptible to electron attack so that the n-type charge generation material may be easily deteriorated, the lifespan may be shortened, and the change in driving voltage (ΔV) may be rapidly increased.

### Examples 47 to 49 and Comparative Examples 10 to 12

Organic light-emitting devices were manufactured in the same manner as in Example 1, except that compounds shown in Table 3 were used as an emission unit material and an n-type charge generation layer material.

### Examples 50 and 51, Comparative Examples 13 and 14

Organic light-emitting devices were manufactured in the same manner as in Example 1, except that, instead of forming the cathode on the third emission unit, a cathode was formed after the third charge generation unit and a fourth emission unit were sequentially formed on the third emission unit. The third charge generation unit was formed in the same way as the second charge generation unit, and the fourth emission unit was formed in the same way as the second charge generation unit, wherein compounds shown in Table 3 were used as an emission unit material and an n-type charge generation layer material.

### Evaluation Example 3

The driving voltage, change in driving voltage, current efficiency, lifespan, and CIE color coordinate of the light-emitting devices manufactured according to Examples 47 to 51 and Comparative Examples 10 to 14 were measured by using Keithley SMU 236 and a luminance meter PR650, and results thereof are shown in Table 3. The lifespan (T₉₇) is a period of time that was taken until the luminance (@1200 nit) was reduced to 97 % of initial luminance (100 %) after a light-emitting device was driven. The change in driving voltage (voltage shift, ΔV) is a difference between the driving voltage measured after 200 hours of driving a light-emitting device and the initial driving voltage.

**[Table 3]**

| | First emission unit compound (Emitting color) | Second emission unit compound (Emitting color) | Third emission unit compound (Emitting color) | Fourth emission unit compound (Emitting color) | Electr on transport layer compound | n-type charge gener ation layer compound | Driving voltage (V) | Efficiency (Cd/A ) | Life span (T₉₇) | Luminance (nit) | ΔV (@200 hr) |
|---|---|---|---|---|---|---|---|---|---|---|---|
| Example 47 | H56+FD1 (blue) | H56+FD1 (blue) | H56+FD1 (blue) | - | ET38 | 36 | 9.7 | 20.5 | 350 | 120 0 | 0.21 |
| Exampl e 48 | H56+FD1 (blue) | H39+H52 +PD13 (green) | H56+FD1 (blue) | - | ET38 | 36 | 10.2 | 37.8 | 450 | 120 0 | 0.33 |
| Example 49 | H56+FD1 (blue) | H56+FD1 (blue) | H39+H52 +PD13 (green) | - | ET38 | 36 | 10.3 | 38.1 | 440 | 120 0 | 0.35 |
| Com parative Exa mpl e 10 | H56+FD1 (blue) | H56+FD1 (blue) | H56+FD1 (blue) | - | ET38 | A | 10.0 | 19.5 | 280 | 120 0 | 0.68 |
| Compara tive Exa mpl e 11 | H56+FD1 (blue) | H39+H52 +PD13 (green) | H56+FD1 (blue) | - | ET38 | A | 10.3 | 32.5 | 280 | 120 0 | 0.78 |
| Com para tive Exa mpl e 12 | H56+FD1 (blue) | H56+FD1 (blue) | H39+H52 +PD13 (green) | - | ET38 | A | 10.5 | 37.5 | 300 | 120 0 | 0.80 |
| Exa mpl e 50 | H56+FD1 (blue) | H56+FD1 (blue) | H39+H52 +PD13 (green) | H56+FD1 (blue) | ET38 | 36 | 12.3 | 53.4 | 650 | 120 0 | 0.40 |
| Exa mple 51 | H56+FD1 (blue) | H56+FD1 (blue) | H56+FD1 (blue) | H39+H52 +PD13 (green) | ET38 | 36 | 11.9 | 67.6 | 680 | 120 0 | 0.42 |
| Com para tive Exa mpl e 13 | H56+FD1 (blue) | H56+FD1 (blue) | H39+H52 +PD13 (green) | H56+FD1 (blue) | ET38 | A | 12.6 | 50.2 | 450 | 120 0 | 0.94 |
| Com para tive Exa mpl e 14 | H56+FD1 (blue) | H56+FD1 (blue) | H56+FD1 (blue) | H39+H52 +PD13 (green) | ET38 | A | 12.2 | 61.6 | 480 | 120 0 | 1.01 |

Referring to Table 3, it was confirmed that the organic light-emitting device according to an embodiment had low driving voltage, excellent efficiency, and long lifespan, and that the change in driving voltage was small.

The organic light-emitting device including the heterocyclic compound had low driving voltage, high emission efficiency, and improved lifespan characteristics.

It should be understood that embodiments described herein should be considered in a descriptive sense only and not for purposes of limitation. Descriptions of features or aspects within each embodiment should typically be considered as available for other similar features or aspects in other embodiments. While embodiments have been described with reference to the figures, it will be understood by those of ordinary skill in the art that various changes in form and details may be made therein without departing from the scope as defined by the following claims.

## Claims

1. An organic light-emitting device comprising:
a first electrode;
a second electrode facing the first electrode; and
an organic layer disposed between the first electrode and the second electrode, the organic layer comprising:
emission units; and
charge generation units disposed between two neighboring ones of the emission units, wherein
at least one of the charge generation units comprises a heterocyclic compound represented by Formula 1: 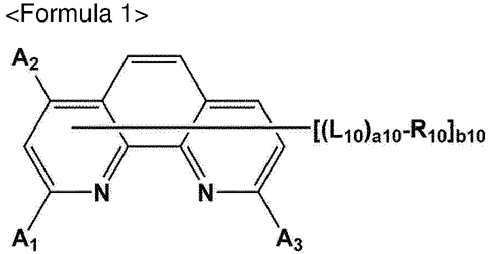 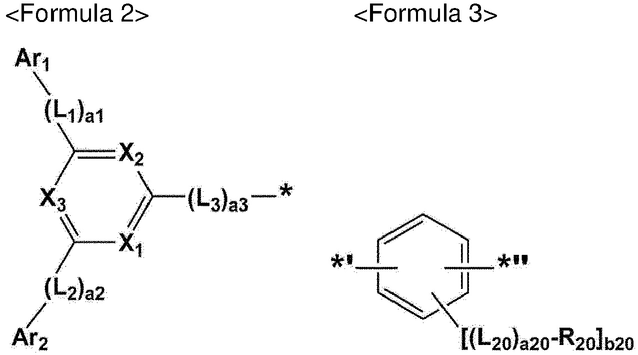
wherein, in Formulae 1 to 3,
A₁ to A₃ are each independently selected from a group represented by Formula 2, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group,-Si(Q₁)(Q₂)(Q₃), -N(Q₁)(Q₂), -B(Q₁)(Q₂), -P(Q₁)(Q₂), -C(=O)(Q₁), -S(=O)₂(Q₁), and-P(=O)(Q₁)(Q₂),
at least one selected from A₁ to A₃ is a group represented by Formula 2,
X₁ is N or C(R₃₁),
X2 is N or C(R₃₂),
X₃ is N or C(R₃₃),
at least one selected from X₁ to X₃ is N,
L₁, L₂, L₁₀, and L₂₀ are each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
a1, a2, a10, and a20 are each independently an integer from 0 to 5,
L₃ is a group represented by Formula 3,
a3 is an integer from 0 to 5,
Ar₁ and Ar₂ are each independently selected from deuterium, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₁)(Q₂), -B(Q₁)(Q₂),-C(=O)(Q₁), -S(=O)₂(Q₁), and -P(=O)(Q₁)(Q₂),
R₁₀, R₂₀, and R₃₁ to R₃₃ are each independently selected from hydrogen, deuterium, -F, -Cl, -Br, -I, a hydroxyl group, a cyano group, a nitro group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₁)(Q₂), -B(Q₁)(Q₂),-P(Q₁)(Q₂), -C(=O)(Q₁), -S(=O)₂(Q₁), and -P(=O)(Q₁)(Q₂),
two or more substituents selected from the group consisting of R₁₀, R₂₀, and R₃₁ to R₃₃ are optionally linked to each other to form a substituted or unsubstituted C₅-C₆₀ carbocyclic group or a substituted or unsubstituted C₁-C₆₀ heterocyclic group,
b10 is an integer from 1 to 5,
b20 is an integer from 1 to 4,
*, *', and *" each indicate a binding site to a neighboring atom, and
at least one substituent of the substituted C₅-C₆₀ carbocyclic group, the substituted C₁-C₆₀ heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is selected from:
deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group,
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl,-Br, -I, a hydroxyl group, a cyano group, a nitro group, an amidino group, a hydrazine group, a hydrazone group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₁₁)(Q₁₂)(Q₁₃), -N(Q₁₁)(Q₁₂), -B(Q₁₁)(Q₁₂), -P(Q₁₁)(Q₁₂),-C(=O)(Q₁₁), -S(=O)₂(Q₁₁), and -P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, a biphenyl group, and a terphenyl group,
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, a biphenyl group, and a terphenyl group, each substituted with at least one selected from deuterium, -F, -Cl, -Br, -I, a hydroxyl group, a cyano group, a nitro group, an amidino group, a hydrazine group, a hydrazone group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, a biphenyl group, a terphenyl group, -Si(Q₂₁)(Q₂₂)(Q₂₃), -N(Q₂₁)(Q₂₂), -B(Q₂₁)(Q₂₂),-P(Q₂₁)(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁),-S(=O)₂(Q₃₁), and -P(=O)(Q₃₁)(Q₃₂),
wherein Q₁ to Q₃, Q₁₁ to Q₁₃, Q₂₁ to Q₂₃, and Q₃₁ to Q₃₃ are each independently selected from hydrogen, deuterium, -F, -Cl, -Br, -I, a hydroxyl group, a cyano group, a nitro group, an amidino group, a hydrazine group, a hydrazone group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with a C₁-C₆₀ alkyl group, a C₁-C₆₀ heteroaryl group, a monovalent non-aromatic condensed polycyclic group, a monovalent non-aromatic condensed heteropolycyclic group, a biphenyl group, and a terphenyl group.

2. An organic light-emitting device according to claim 1, wherein
at least one of the charge generation units includes an n-type charge generation layer and a p-type charge generation layer, and
the n-type charge generation layer includes the heterocyclic compound.

3. An organic light-emitting device according to claim 2, wherein:
(i) the n-type charge generation layer includes an electron transport compound; and/or
(ii) the n-type charge generation layer includes an alkali metal or a lanthanide metal.

4. An organic light-emitting device according to any one of claims 1 to 3, wherein
each of the emission units includes an emission layer, and
the emission layer includes a host and a dopant.

5. An organic light-emitting device according to any one of claims 1 to 4, wherein:
(i) at least one of the emission units emits blue light having a maximum emission wavelength in a range of about 410 nm to about 490 nm; and/or
(ii) at least one of the emission units emits green light having a maximum emission wavelength in a range of about 490 nm to about 580 nm.

6. An organic light-emitting device according to claim 4 or claim 5, wherein
each of the emission units includes a hole transport region and an electron transport region,
the hole transport region includes at least one selected from a hole injection layer, a hole transport layer, a buffer layer, an emission auxiliary layer, and an electron blocking layer, and
the electron transport region includes at least one selected from a hole blocking layer, an electron transport layer, and an electron injection layer.

7. An organic light-emitting device according to any one of claims 1 to 6, wherein
at least one selected from A₁ to A₃ is a group represented by Formula 2, and
the remaining substituents are each independently selected from a substituted or unsubstituted C₁-C₆₀ alkyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group.

8. An organic light-emitting device according to any one of claims 1 to 7, wherein
at least one selected from A₁ to A₃ is a group represented by Formula 2, and
the remaining substituents are each independently selected from:
a methyl group, an ethyl group, a propyl group, an isobutyl group, a sec-butyl group, a ter-butyl group, a pentyl group, an isoamyl group, and a hexyl group;
a methyl group, an ethyl group, a propyl group, an isobutyl group, a sec-butyl group, a ter-butyl group, a pentyl group, an isoamyl group, and a hexyl group, each substituted with at least one selected from deuterium, -F, -Cl, -Br, -I, a hydroxyl group, a cyano group, a nitro group, an amino group, a C₁-C₆₀ alkyl group, a C₆-C₆₀ aryl group, and a C₁-C₆₀ heteroaryl group;
a phenyl group, a naphthyl group, a pyridyl group, a pyrimidyl group, a triazinyl group, a quinoline group, an isoquinoline group, a biphenyl group, and a terphenyl group; and
a phenyl group, a naphthyl group, a pyridyl group, a pyrimidyl group, a triazinyl group, a quinoline group, an isoquinoline group, a biphenyl group, and a terphenyl group, each substituted with at least one selected from deuterium, -F, -Cl, -Br, -I, a hydroxyl group, a cyano group, a nitro group, an amino group, a C₁-C₆₀ alkyl group, a C₆-C₆₀ aryl group, and a C₁-C₆₀ heteroaryl group.

9. An organic light-emitting device according to any one of claims 1 to 8, wherein
L₁, L₂, L₁₀, and L₂₀ are each independently a group represented by one of Formulae 3-1 to 3-99: 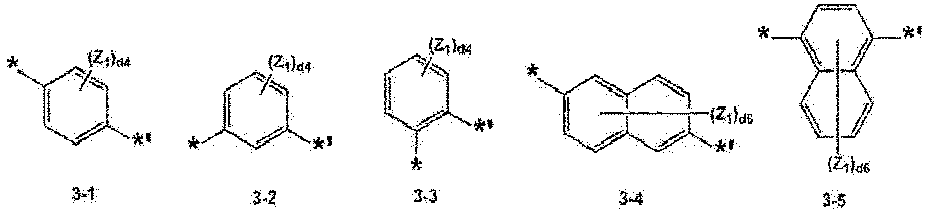 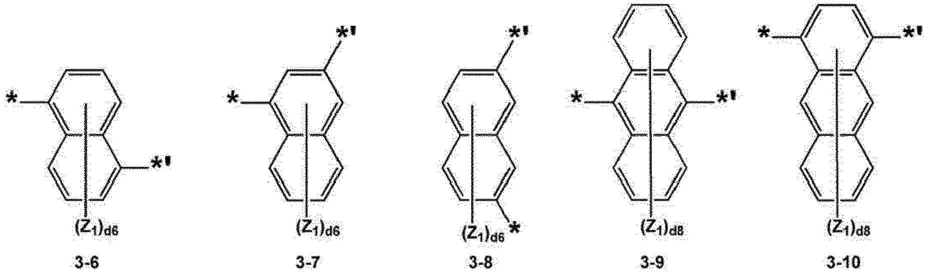 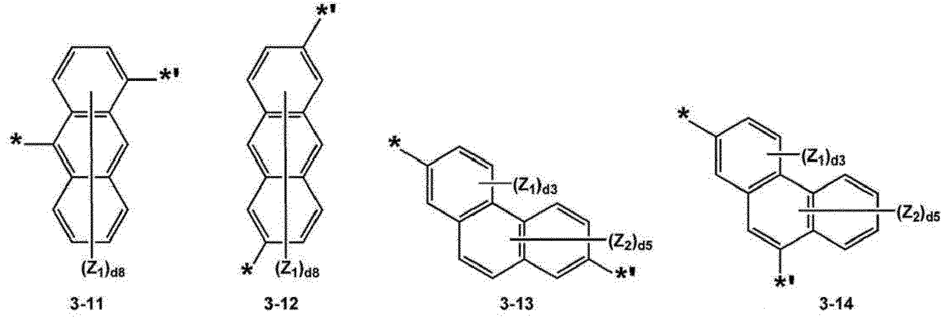 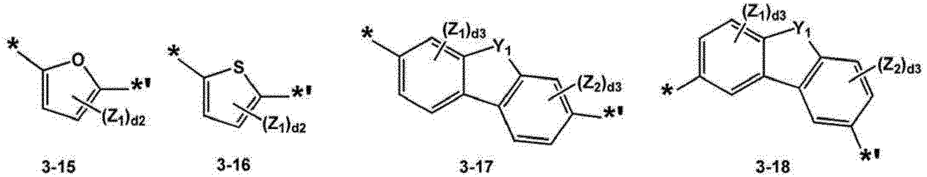 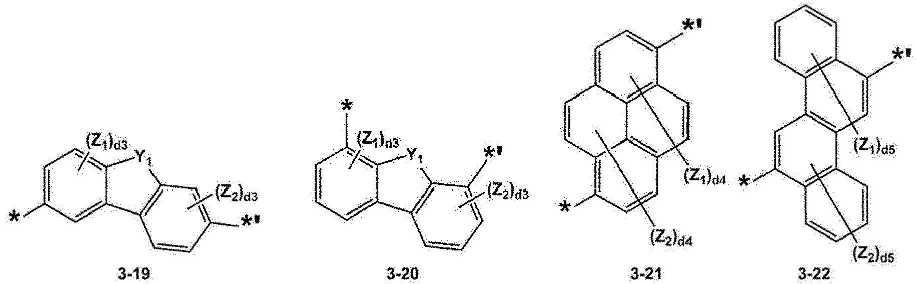 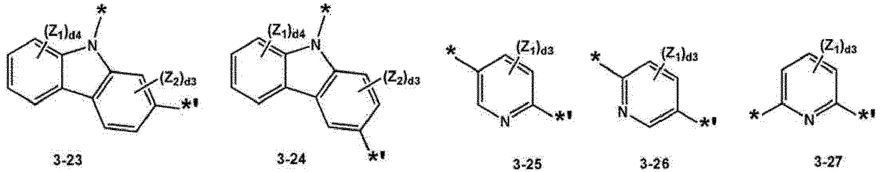 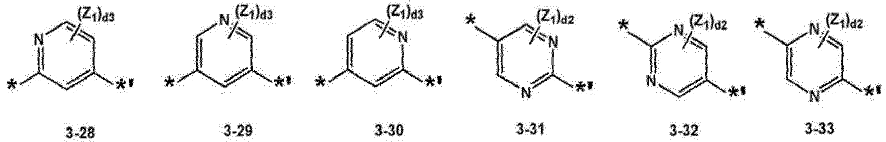 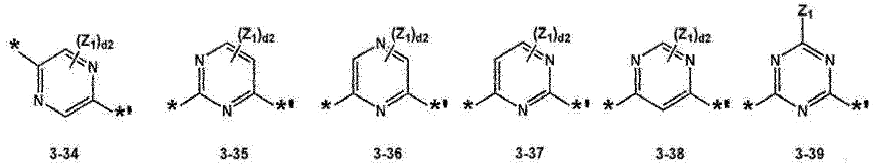 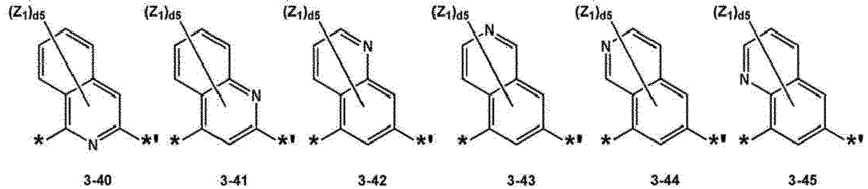 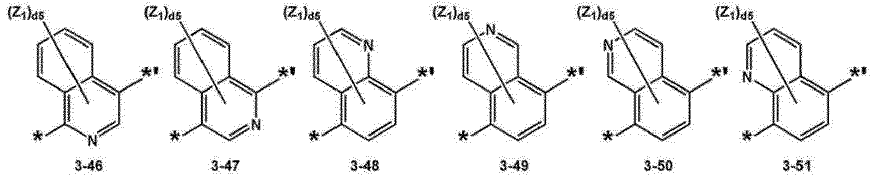 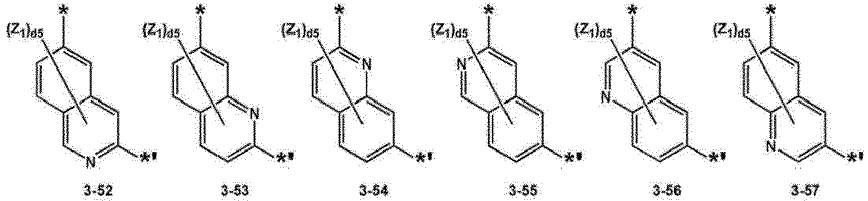 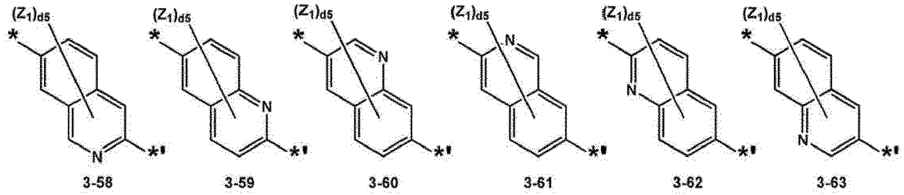 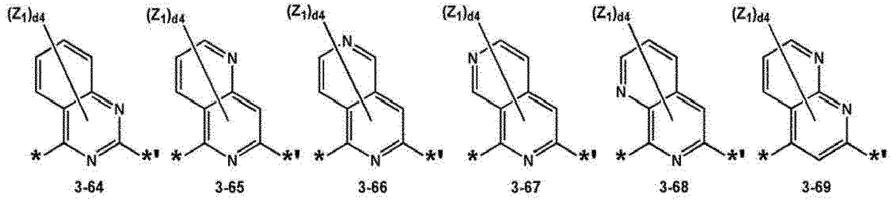 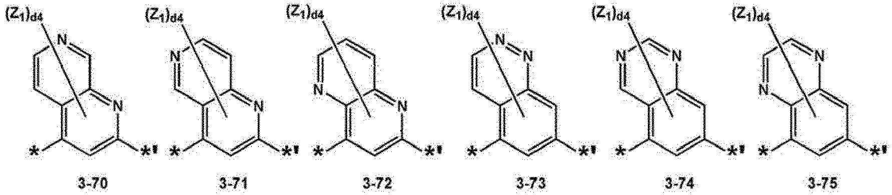 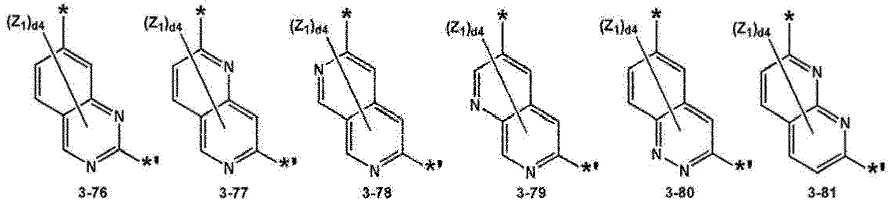 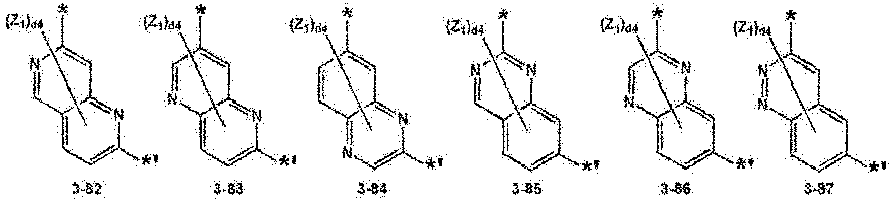 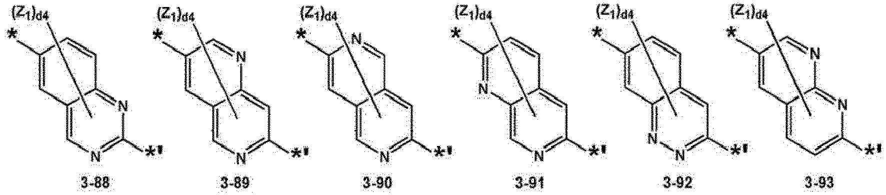 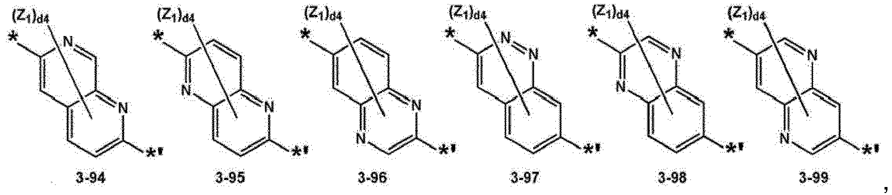 wherein, in Formulae 3-1 to 3-99,
Y₁ is O, S, C(Z₃)(Z₄), N(Z₅), or Si(Z₆)(Z₇),
wherein Z₁ to Z₇ are each independently selected from hydrogen, deuterium,-F, -Cl, -Br, -I, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a carbazolyl group, a benzocarbazolyl group, a dibenzocarbazolyl group, a thiadiazolyl group, an imidazopyridinyl group, an imidazopyrimidinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and-P(=O)(Q₃₁)(Q₃₂),
wherein Q₃₁ to Q₃₃ are each independently selected from:
a C₁-C₁₀ alkyl group, a C₁-C₁₀ alkoxy group, a phenyl group, a biphenyl group, a terphenyl group, a naphthyl group, a pyridinyl group, a pyrimidinyl group, a pyrazinyl group, a quinolinyl group, an isoquinolinyl group, a quinoxalinyl group, and a quinazolinyl group; and
a phenyl group, a biphenyl group, a terphenyl group, a naphthyl group, a pyridinyl group, a pyrimidinyl group, a pyrazinyl group, a quinolinyl group, an isoquinolinyl group, a quinoxalinyl group, and a quinazolinyl group, each substituted with at least one selected from a C₁-C₁₀ alkyl group, a C₁-C₁₀ alkoxy group, and a phenyl group,
d2 is an integer from 0 to 2,
d3 is an integer from 0 to 3,
d4 is an integer from 0 to 4,
d5 is an integer from 0 to 5,
d6 is an integer from 0 to 6,
d8 is an integer from 0 to 8, and
* and *' each indicate a binding site to a neighboring atom.

10. An organic light-emitting device according to any one of claims 1 to 9, wherein
L₃ is a group represented by one of Formulae 4-1 to 4-3: 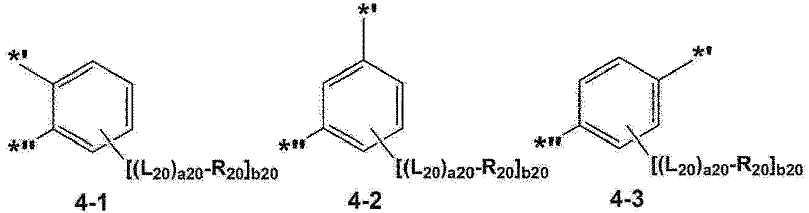 wherein, in Formulae 4-1 to 4-3,
L₂₀, a20, R₂₀, and b20 are the same as described in connection with Formula 3, and
*' and *" each indicate a binding site to a neighboring atom.

11. An organic light-emitting device according to any one of claims 1 to 9, wherein
Ar₁ and Ar₂ are each independently selected from:
deuterium; and a group represented by one of Formulae 5-1 to 5-26 and 6-1 to 6-55: 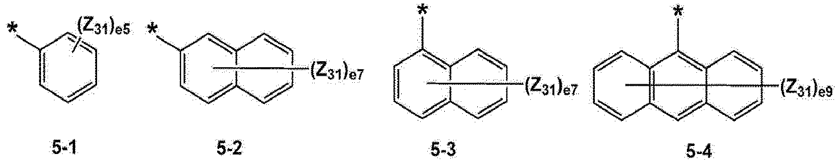 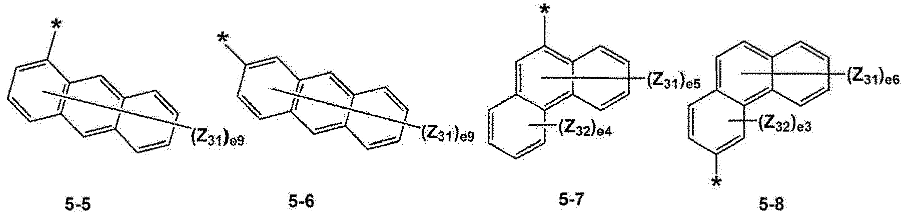 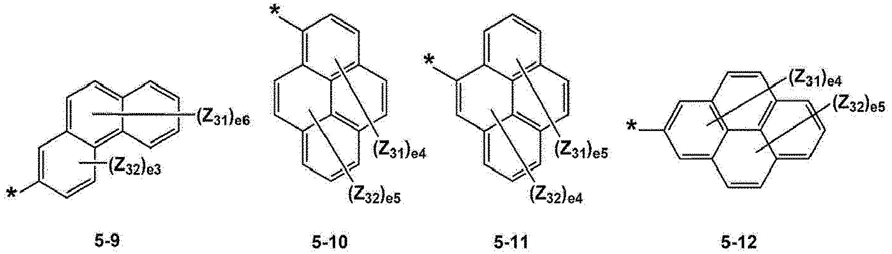 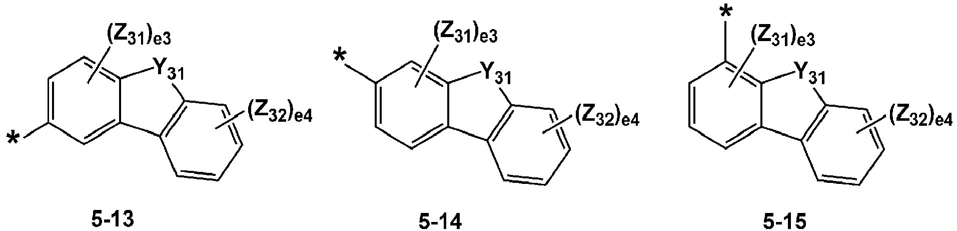 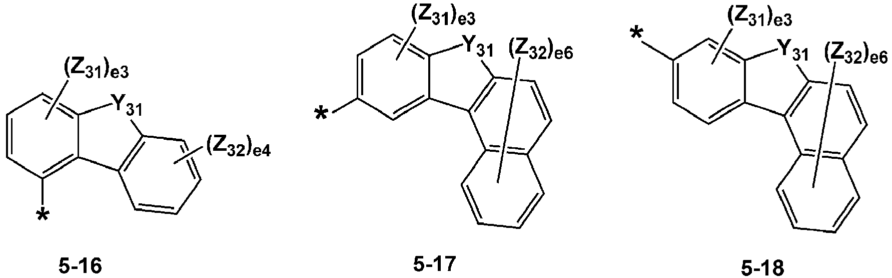 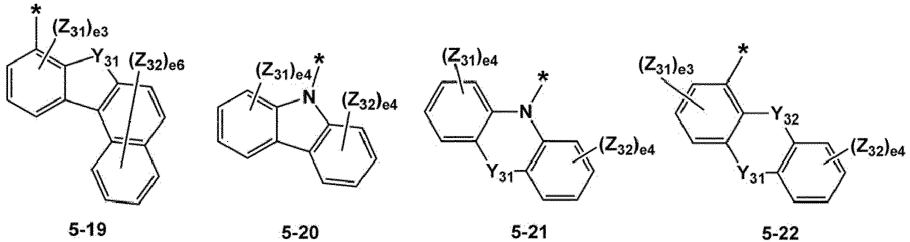 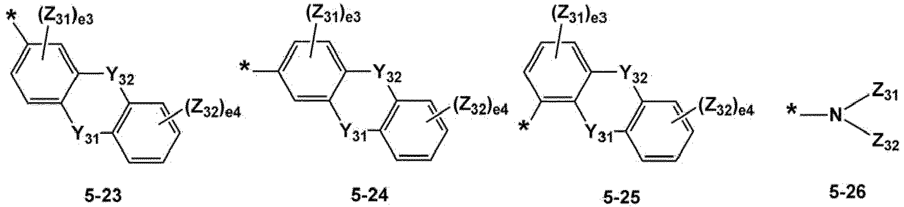 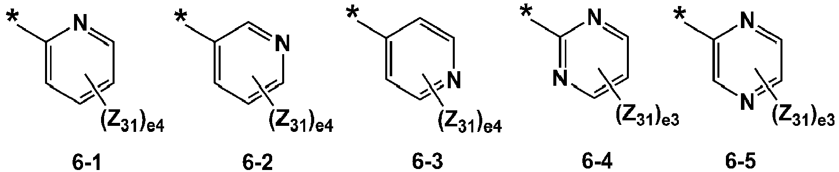 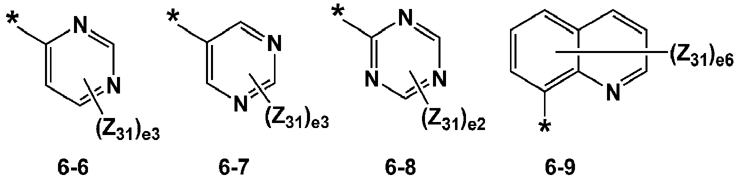 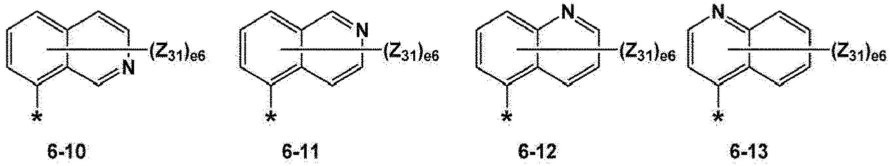 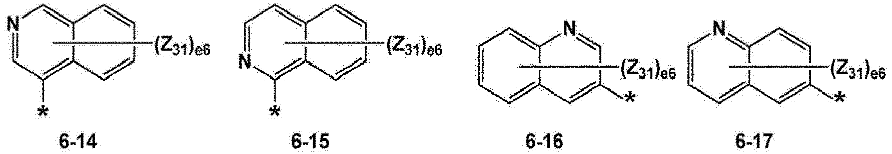 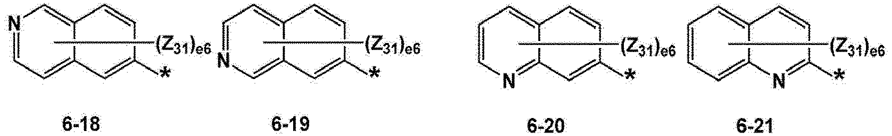 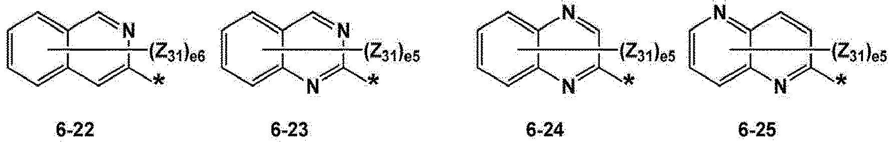 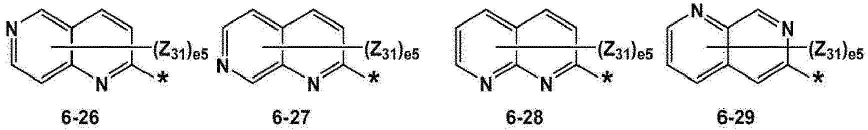 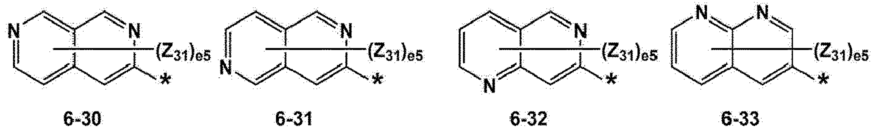 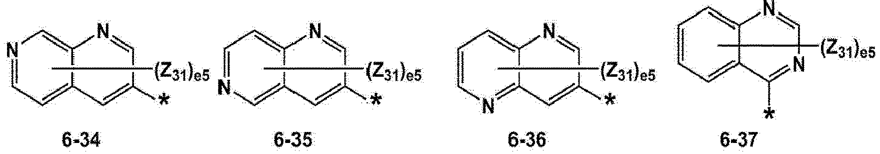 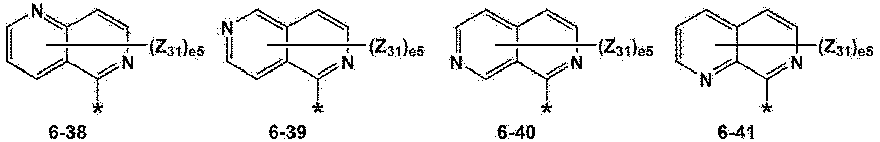 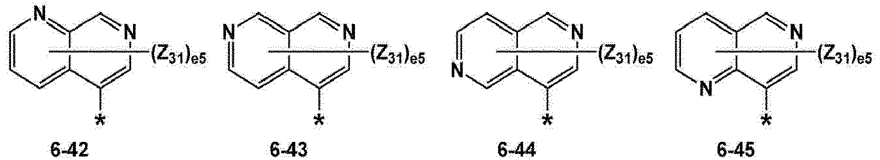 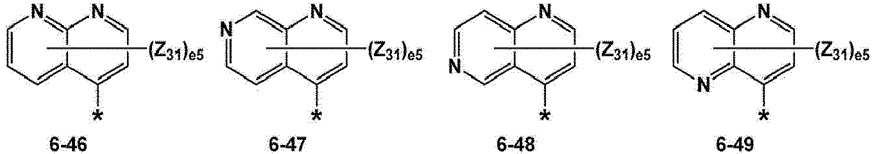 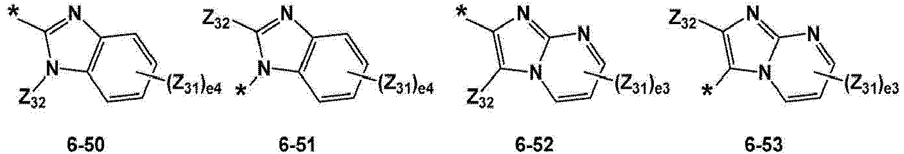 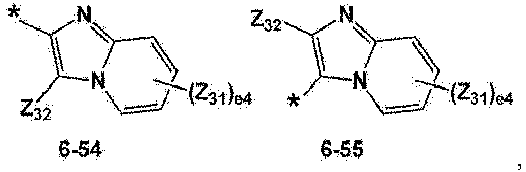 wherein, in Formulae 5-1 to 5-26 and 6-1 to 6-55,
Y₃₁ and Y₃₂ are each independently O, S, C(Z₃₃)(Z₃₄), N(Z₃₃), or Si(Z₃₃)(Z₃₄),
Z₃₁ to Z₃₄ are each independently selected from hydrogen, deuterium, -F, -Cl,-Br, -I, a hydroxyl group, a cyano group, a nitro group, an amidino group, a hydrazino group, a hydrazono group, a C₁-C₂₀ alkyl group, a C₂-C₂₀ alkenyl group, a C₂-C₂₀ alkynyl group, a C₁-C₂₀ alkoxy group, a phenyl group, a biphenyl group, a terphenyl group, a naphthyl group, a fluorenyl group, a spiro-bifluorenyl group, a phenanthrenyl group, an anthracenyl group, a triphenylenyl group, a pyridinyl group, a pyrimidinyl group, a carbazolyl group, and a triazinyl group,
e2 is 1 or 2,
e3 is an integer from 1 to 3,
e4 is an integer from 1 to 4,
e5 is an integer from 1 to 5,
e6 is an integer from 1 to 6,
e7 is an integer from 1 to 7,
e9 is an integer from 1 to 9, and
* indicates a binding site to a neighboring atom.

12. An organic light-emitting device according to any one of claims 1 to 11, wherein Ar₁ and Ar₂ are each independently selected from:
a phenyl group, a naphthyl group, a pyridyl group, a pyrimidyl group, a triazinyl group, a quinoline group, an isoquinoline group, a biphenyl group, and a terphenyl group; and
a phenyl group, a naphthyl group, a pyridyl group, a pyrimidyl group, a triazinyl group, a quinoline group, an isoquinoline group, a biphenyl group, and a terphenyl group, each substituted with at least one selected from deuterium, -F, -Cl, -Br, -I, a hydroxyl group, a cyano group, a nitro group, an amino group, a C₁-C₆₀ alkyl group, a C₆-C₆₀ aryl group, and a C₁-C₆₀ heteroaryl group.

13. An organic light-emitting device according to any one of claims 1 to 12, wherein R₁₀ and R₂₀ are each independently selected from:
a methyl group, an ethyl group, a propyl group, an isobutyl group, a sec-butyl group, a ter-butyl group, a pentyl group, an isoamyl group, and a hexyl group;
a methyl group, an ethyl group, a propyl group, an isobutyl group, a sec-butyl group, a ter-butyl group, a pentyl group, an isoamyl group, and a hexyl group, each substituted with at least one selected from deuterium, -F, -Cl, -Br, -I, a hydroxyl group, a cyano group, a nitro group, an amino group, a C₁-C₆₀ alkyl group, a C₆-C₆₀ aryl group, and a C₁-C₆₀ heteroaryl group;
a phenyl group, a naphthyl group, a pyridyl group, a pyrimidyl group, a triazinyl group, a quinoline group, an isoquinoline group, a biphenyl group, and a terphenyl group; and
a phenyl group, a naphthyl group, a pyridyl group, a pyrimidyl group, a triazinyl group, a quinoline group, an isoquinoline group, a biphenyl group, and a terphenyl group, each substituted with at least one selected from deuterium, -F, -Cl, -Br, -I, a hydroxyl group, a cyano group, a nitro group, an amino group, a C₁-C₆₀ alkyl group, a C₆-C₆₀ aryl group, and a C₁-C₆₀ heteroaryl group.

14. An organic light-emitting device according to any one of claims 1 to 13, wherein the heterocyclic compound is represented by one of Formulae 11-1 to 11-3: 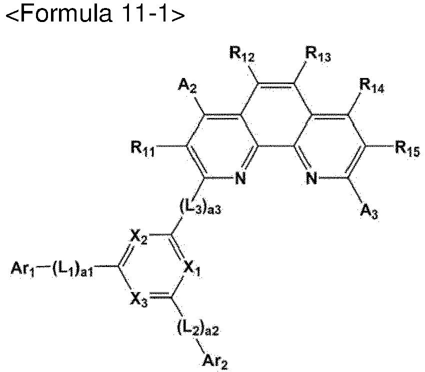 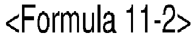 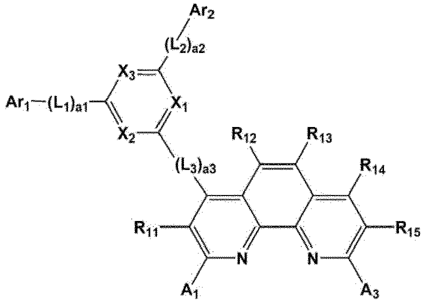 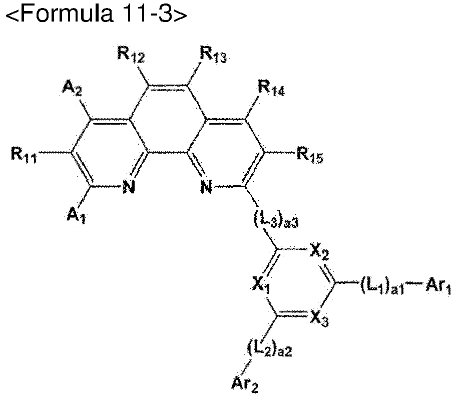 wherein, in Formulae 11-1 to 11-3,
A₁ to A₃ are the same as described in connection with Formula 1,
X₁ to X₃, L₁ to L₃, a1 to a3, Ar₁, and Ar₂ are the same as described in connection with Formula 2, and
R₁₁ to R₁₅ are the same as described in connection with R₁₀ in Formula 1.

15. An organic light-emitting device according to any one of claims 2 to 14, wherein:
(i) the electron transport compound is a metal-free compound including at least one π electron-deficient nitrogen-containing ring; and/or
(ii) the electron transport compound is selected from one of Compounds ET1 to ET39:

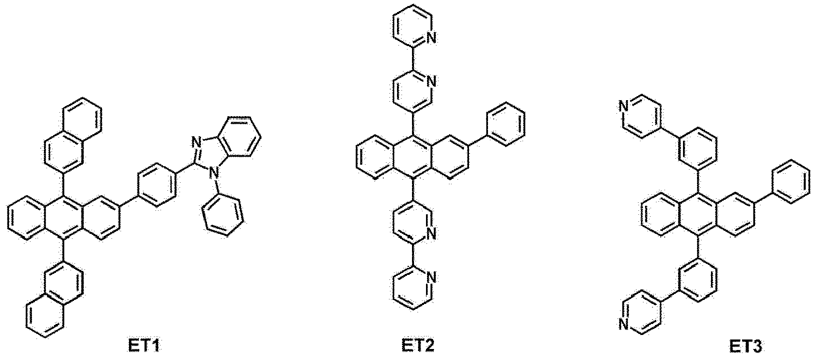

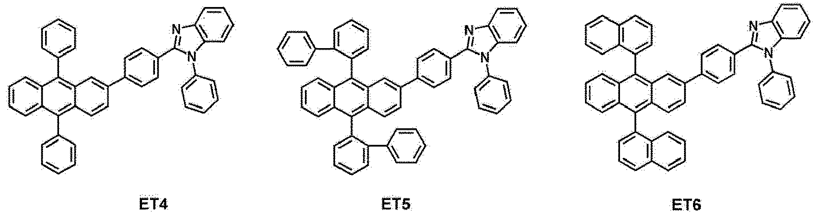

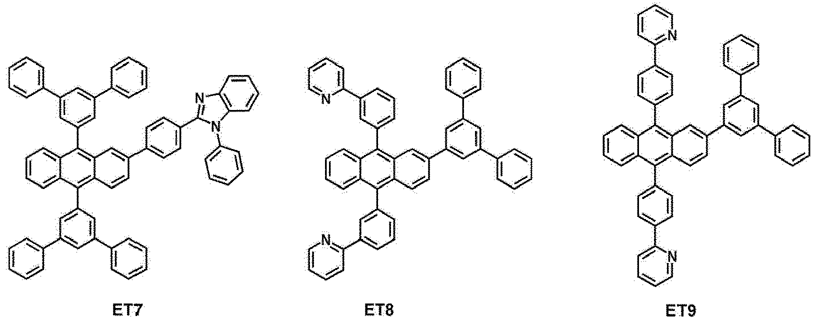

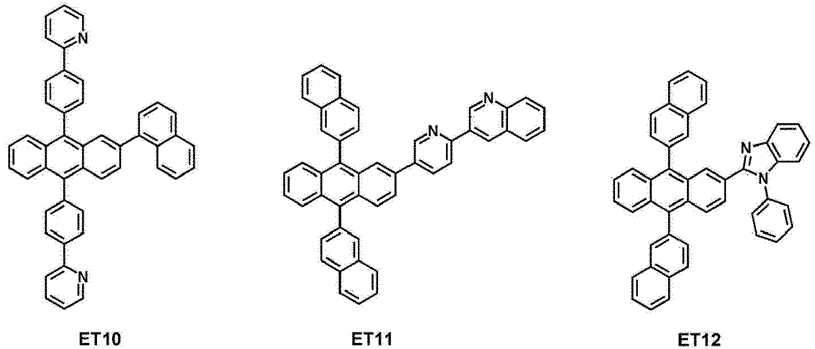

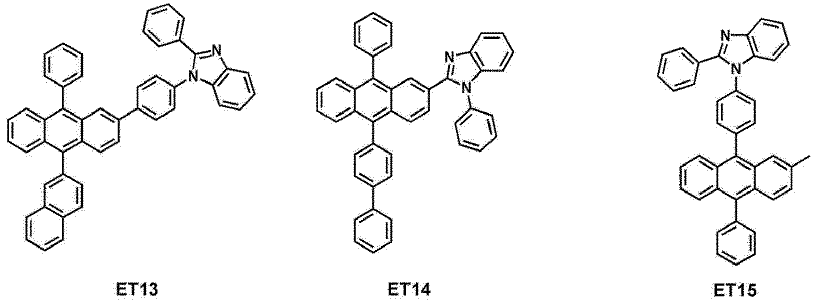

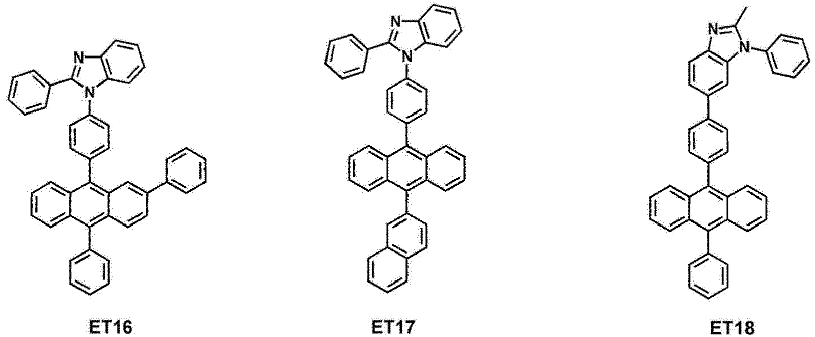

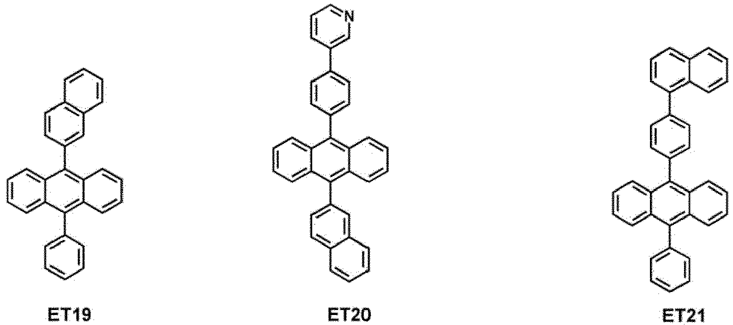

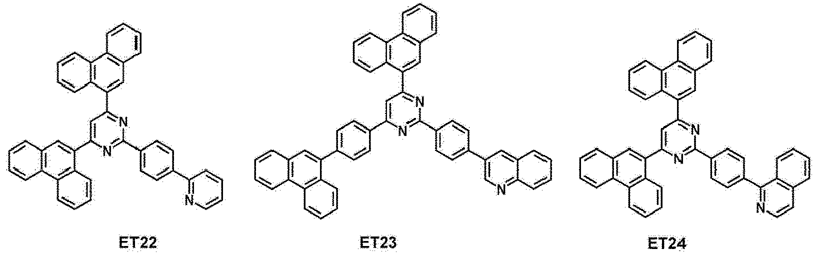

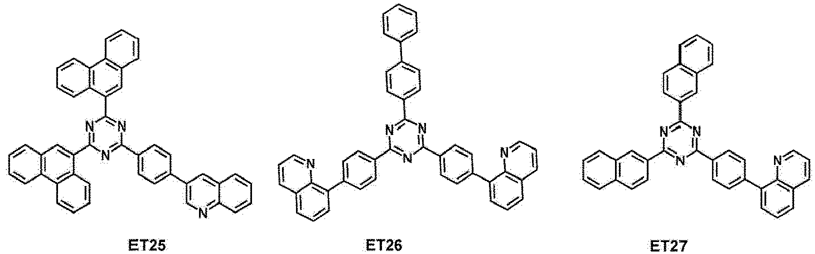

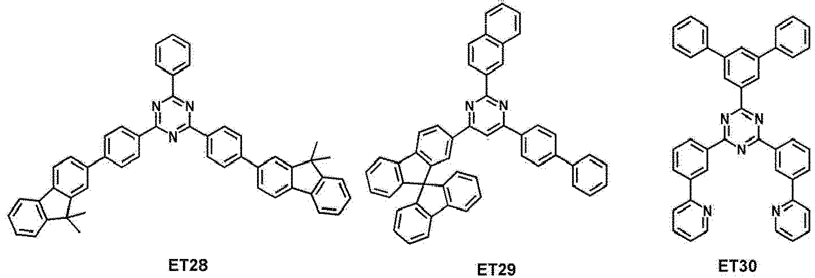

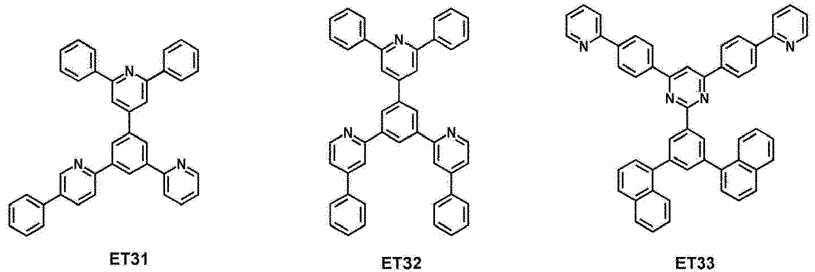

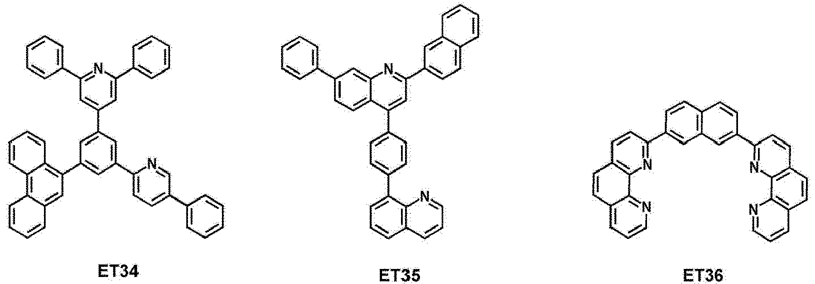

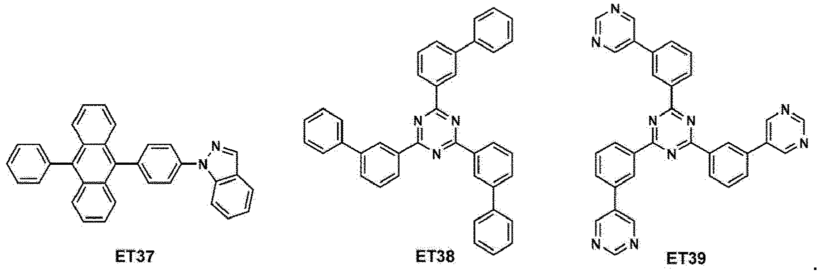

16. An organic light-emitting device according to any one of claims 1 to 15, wherein the heterocyclic compound is selected from one of Compounds 1 to 42:

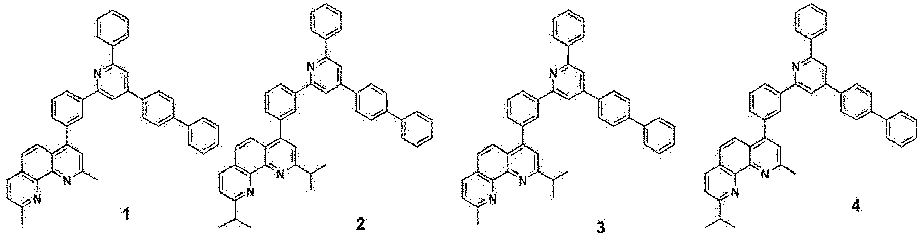

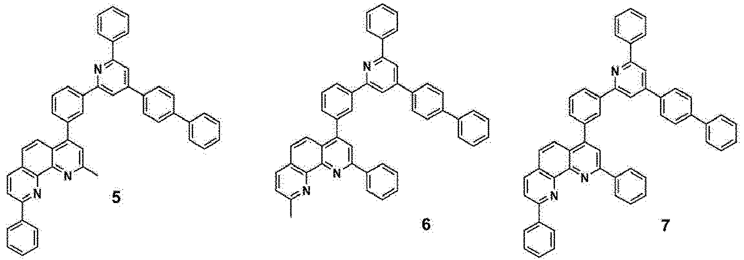

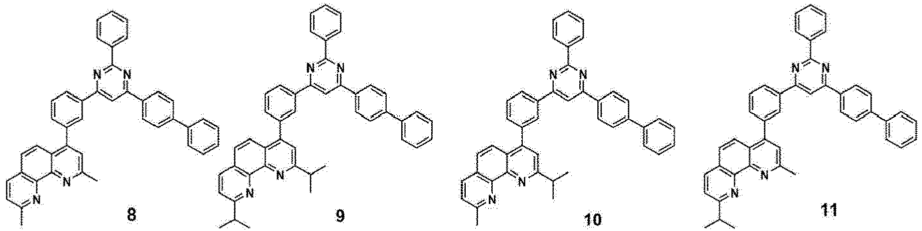

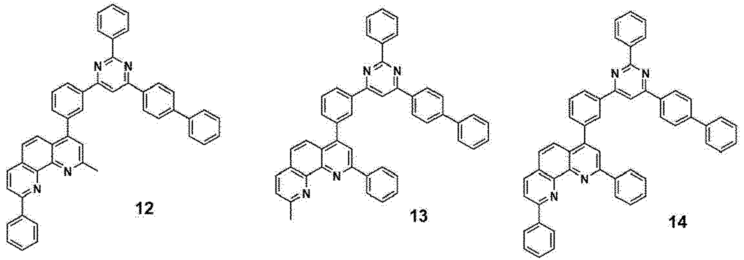

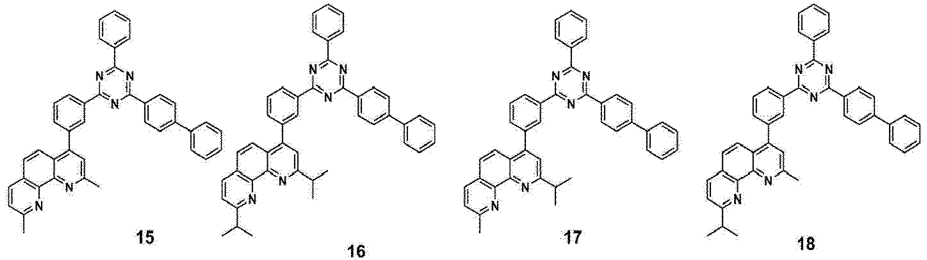

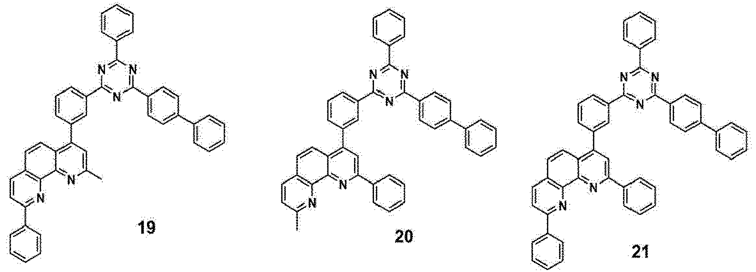

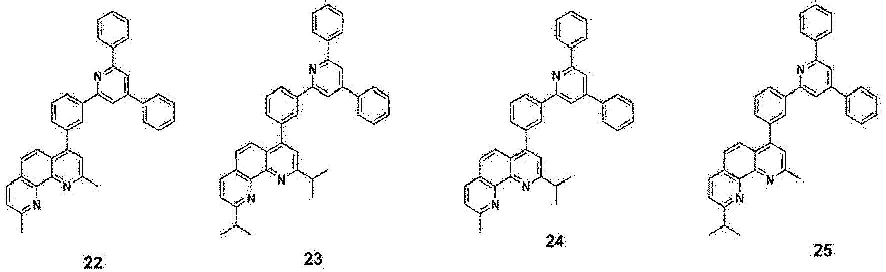

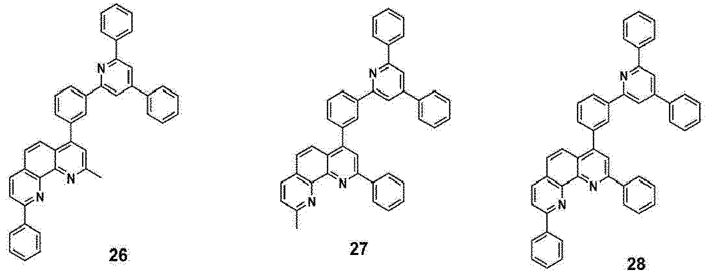

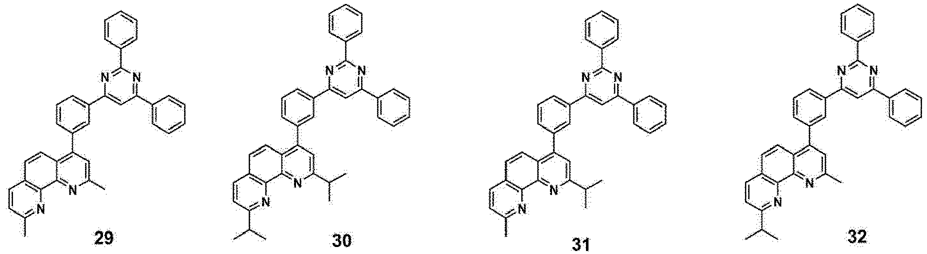

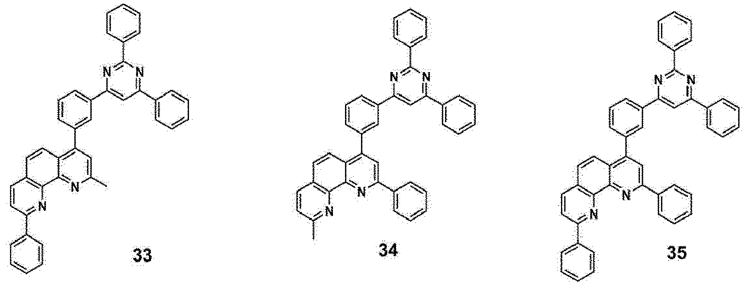

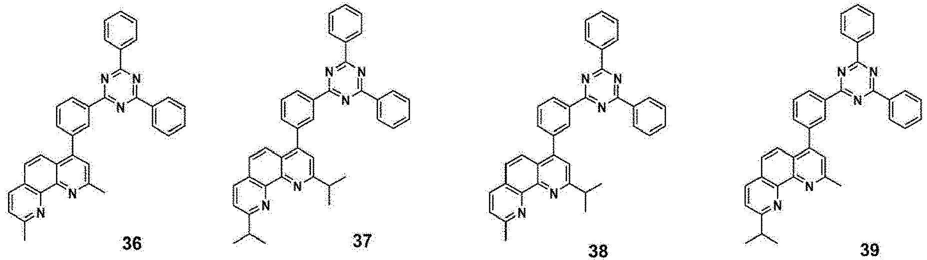

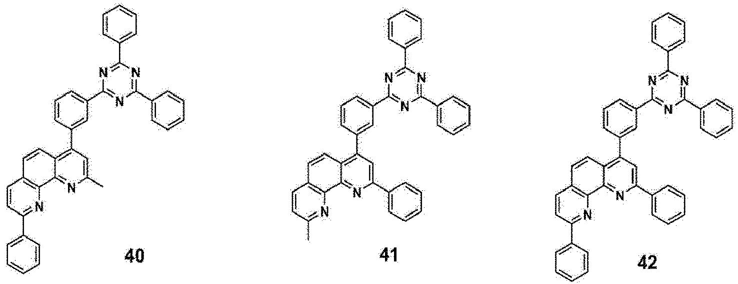

17. An apparatus comprising:
a substrate;
an organic light-emitting device according to any one of claims 1 to 16 disposed on the substrate; and
a color conversion layer disposed on at least one traveling direction of light emitted from the organic light-emitting device,
wherein the color conversion layer includes quantum dots.
